# EUROPEAN PATENT APPLICATION

(11) **EP 4 198 050 A1**
(43) Date of publication of application: **21.06.2023**
(21) Application number: 21856206.4
(22) Date of filing: 10.08.2021
(51) Int. Cl.: C07K 14/54, C07K 16/40, C07K 16/28, A61K 38/00, A61P 35/00

(54) **FUSION PROTEIN COMPRISING IL-12 AND ANTI-CD20 ANTIBODY AND USE THEREOF**

(30) Priority: 11.08.2020 KR 20200100229
(71) Applicant: Kanaph Therapeutics Inc., Seoul 04348 (KR)
(72) Inventor: LEE, Dahea, Seoul 03938 (KR); RYU, Soomin, Anyang-si Gyeonggi-do 14109 (KR); KIM, Donggeon, Seoul 05629 (KR); CHANG, Jihoon, Seoul 04211 (KR); LEE, Byoung Chul, Seoul 06289 (KR)
(74) Representative: HGF
(86) International application number: PCT/KR2021/010611
(87) International publication number: WO 2022/035200

(57) **Abstract**

The present invention provides a bispecific antibody comprising IL-12 or a variant thereof and an antigen binding site that specifically binds to CD20. The bispecific antibody exhibits an anticancer effect by IL-12. In particular, when the anti-CD20 antibody is implemented in one antibody, IL-12 is specifically localized to a tumor site by targeting CD20, which is specifically expressed in a tumor at a high level, thereby efficiently treating cancer. Therefore, the bispecific antibody can be utilized as a pharmaceutical composition for anticancer treatment, and thus has a high potential for industrial application.

## Description

### Technical Field

The present invention relates to a novel fusion protein comprising IL-12 or a variant thereof; and an anti-CD20 antibody, and a pharmaceutical composition comprising the same for treating cancer.

### Background Art

Interleukin-12 (IL-12) is a representative inflammatory cytokine and plays an essential role in effectively inducing a cellular immune response. IL-12 is a heterodimeric protein comprising 40 kDa (p40) and 35 kDa (p35) subunits linked by a disulfide bond and is produced by activated macrophages, monocytes, dendritic cells and activated B lymphocytes. IL-12 may enhance T-helper 1 cell immunity, increase cytotoxicity of cellular T-lymphocytes, and inhibit angiogenesis. In general, IL-12 activates IFN-γ production in T cells and NK cells.

Local expression of IL-12 renders tumor cells sensitive to T-cell mediated cytotoxicity, resulting in tumor growth inhibition and the establishment of humoral immunity. However, a disadvantage in the clinical application of IL-12 is that upon administration, cytokine-related toxicity may occur systemically. These clinical results show the limitations of IL-12 as a monotherapeutic agent for the treatment of cancer.

In addition, CD20 is a hydrophobic transmembrane protein with a molecular weight of approximately 35 kDa that is localized on immature B (pre-B) lymphocytes and mature B lymphocytes, and is expressed over 90% in B cell non-Hodgkin's lymphoma (NHL). However, it is not found in hematopoietic stem cells, normal plasma cells or other normal tissues. Rituximab, an antibody against the human CD20 antigen, has been used for the treatment of patients suffering from B cell non-Hodgkin's lymphoma, and is an antibody referred to as "C2B8" in US Patent No. 5,776,456. Despite the success of antibodies such as C2B8, there is a continuing need for anti-CD20 with improved properties.

### Detailed Description of Invention

### Technical Problem

Accordingly, the present inventors have studied to develop a novel fusion protein having an anticancer effect. As a result, the present inventors have found that a fusion protein comprising IL-12 and an anti-CD20 antibody has an increased anticancer activity while reducing systemic toxicity, thereby completing the present invention.

### Solution to Problem

In one aspect of the present invention, there is provided a fusion protein comprising a first monomer comprising IL-12 or a variant thereof; and a second monomer comprising an antigen binding site that specifically binds to CD20.

In another aspect of the present invention, there is provided a pharmaceutical composition for preventing or treating cancer, comprising the fusion protein as an active ingredient.

In another aspect of the present invention, there is provided a polynucleotide encoding the first monomer.

In another aspect of the present invention, there is provided a polynucleotide encoding the second monomer.

In another aspect of the present invention, there is provided a vector comprising the polynucleotide.

In another aspect of the present invention, there is provided a transformed cell into which the vector has been introduced.

In another aspect of the present invention, there is provided a method of producing a fusion protein, comprising: i) culturing the transformed cell; and ii) recovering a fusion protein comprising a first monomer and a second monomer.

In yet another aspect of the present invention, there is provided a method for treating or preventing cancer, comprising administering, to a subject, a fusion protein comprising a first monomer comprising IL-12 or a variant thereof; and a second monomer comprising an antigen binding site that specifically binds to CD20.

In yet another aspect of the present invention, there is provided a use of a fusion protein comprising a first monomer comprising IL-12 or a variant thereof; and a second monomer comprising an antigen binding site that specifically binds to CD20 for treatment of cancer.

In yet another aspect of the present invention, there is provided a use of a fusion protein comprising a first monomer comprising IL-12 or a variant thereof; and a second monomer comprising an antigen binding site that specifically binds to CD20 for manufacture of a medicament for treating cancer.

### Effects of Invention

A fusion protein comprising IL-12 and an antigen binding site that specifically binds to CD20 may exhibit an anticancer effect by IL-12. In addition, it was found that when the anti-CD20 antibody is implemented in one antibody, IL-12 is specifically accumulated in a tumor, thereby reducing systemic toxicity and exhibiting a tumor-specific anticancer effect. That is, IL-12 is specifically localized to a tumor site by targeting CD20, which is specifically expressed in a tumor at a high level, thereby efficiently treating cancer.

### Brief Description of Drawings

FIG. 1 illustrates a result obtained by identifying T2.01, T2.02, T2.03, T2.04, T2.05, T2.06 and T2.07 according to one embodiment with SDS-PAGE.
FIG. 2 illustrates a result obtained by identifying T2.08, T2.09, T2.10, T2.11, T2.12 and T2.13 according to one embodiment with SDS-PAGE.
FIG. 3 illustrates a result obtained by identifying T2.14, T2.15, T2.16, T2.17 and Rituximab according to one embodiment with SDS-PAGE.
FIG. 4 illustrates a result obtained by identifying T2.01m, T2.02m, T2.03m, T2.04m, T2.05m, T2.06m and T2.07m according to one embodiment with SDS-PAGE.
FIG. 5 illustrates a result obtained by identifying T2.08m, T2.09m, T2.10m, T2.11m, T2.12m and T2.13m according to one embodiment with SDS-PAGE.
FIG. 6 illustrates a result obtained by identifying T2.15m, T2.16m, T2.17m and 18B12 according to one embodiment with SDS-PAGE.
FIG. 7 illustrates a graph showing the binding affinities of T2.01 and T2.02 for recombinant human CD20 (rhCD20) through surface plasmon resonance analysis method.
FIG. 8 illustrates a graph showing the binding affinities of Rituximab, T2.01 and T2.02 for Raji cells, Ramos cells and Jurkat cells through flow cytometry.
FIG. 9 illustrates a graph showing the binding affinities of 18B12, T2.01m and T2.02m for A20 cells through flow cytometry.
FIG. 10 illustrates a graph showing the binding affinities of Rituximab, T2.01 and T2.02 for IL-12 receptor beta 1 through enzyme-linked immunosorbent assay.
FIG. 11 illustrates a diagram showing antibody-dependent cellular cytotoxicity of Rituximab, T2.01, T2.02 and T2.12 through ADCC reporter analysis.
FIG. 12 illustrates a graph showing a result obtained by identifying the IL-12 activity of T2.01m, T2.02m, T2.03m and 18B12 through absorbance in HEKblue reporter cells.
FIG. 13 illustrates a graph showing the IL-12 activity of T2.01, T2.02 and T2.17 through the signaling ability of human T cells.
FIG. 14 illustrates a graph showing a result obtained by measuring the ability of human T cells to secrete cytokine IFN-γ by Rituximab, T2.01, T2.02 and T2.03.
FIG. 15 illustrates a graph showing the ability of Rituximab, T2.01 and T2.02 to secrete IFN-γ upon mixed lymphocyte reaction through enzyme-linked immunosorbent assay.
FIG. 16 illustrates a graph showing a spider plot by measuring the change in tumor size for each mouse after treatment with T2.01m, T2.02m and chemotherapy for each concentration in a mouse model transplanted with A20 cells.
FIG. 17 illustrates a graph showing a result obtained by measuring the change in tumor size after treatment with T2.01m, T2.02m and chemotherapy in a mouse model transplanted with A20 cells.
FIG. 18 illustrates a graph showing a result obtained by measuring the weight of the mouse in order to identify the side effects after treatment with T2.01m, T2.02m and chemotherapy in a mouse model transplanted with A20 cells.
FIG. 19 illustrates a graph showing a result obtained by measuring the weight of tumor tissues harvested from mice on day 9 after treatment with T2.01m, T2.02m and chemotherapy in a mouse model transplanted with A20 cells.
FIG. 20 illustrates a graph showing the distribution of immune cells in the tumor after isolating immune cells from tumor tissues harvested on day 9 after treatment with T2.01m, T2.02m and chemotherapy in a mouse model transplanted with A20 cells through flow cytometry.
FIG. 21 illustrates a graph showing the change in A20 tumor size after construction of a tumor rechallenge model by A20 cells and 4T1 cells transplantation into mice in which complete response was achieved by T2.02m treatment in a mouse model transplanted with A20 cells.
FIG. 22 illustrates a graph showing the change in 4T1 tumor size after construction of a tumor rechallenge model by A20 cells and 4T1 cells transplantation into mice in which complete response was achieved by T2.02m treatment in a mouse model transplanted with A20 cells.
FIG. 23 illustrates a graph showing a result obtained by measuring the weight of the mouse in order to identify the side effects after construction of a tumor rechallenge model by A20 cells and 4T1 cells transplantation into mice in which complete response was achieved by T2.02m treatment in a mouse model transplanted with A20 cells.
FIG. 24 illustrates a graph showing a result obtained by measuring the change in tumor size after treatment with T2.02m or 18B12 in a mouse model transplanted with A20 cells.
FIG. 25 illustrates a graph showing a result obtained by measuring the change in tumor size after treatment with T2.02m or T2.13m in a mouse model transplanted with A20 cells.
FIG. 26 illustrates a graph showing a result obtained by measuring the change in tumor size after treatment with T2.02m or 18B12 and T2.13m in combination in a mouse model transplanted with A20 cells.
FIG. 27 illustrates a graph showing a result obtained by measuring the change in tumor size after treatment with T2.01m, T2.02m, or T2.03m in a mouse model transplanted with A20 cells.
FIG. 28 illustrates a graph showing a result obtained by measuring the weight of the mouse in order to identify the side effects after treatment with T2.01m, T2.02m, or T2.03m in a mouse model transplanted with A20 cells.
FIG. 29 illustrates a graph showing the binding affinities of T2.04, T2.05, T2.06, and T2.07 for recombinant human CD20 through the surface plasmon resonance analysis method.
FIG. 30 illustrates a graph showing the binding affinities of T2.08, T2.09, T2.10 and T2.11 for recombinant human CD20 through the surface plasmon resonance analysis method.
FIG. 31 illustrates a schematic diagram of an anti-CD20/IL-12 fusion protein (first from the left; in the figure, the left arm is an anti-CD20 monomer, and the right arm is a monomer comprising single-chain IL-12), an anti-CD20/IL-12 fusion protein having a dual variable domain (second), an anti-CD20/IL-12 bispecific antibody to which CD20-specific a single chain variable fragment (scFv) is bound (third and fourth), and an anti-CD20/IL-12 fusion protein in which IL-12 is bound to the C-terminus of Fc (fifth).
FIG. 32 illustrates a schematic diagram ofCD20-specific Fab/CD20-specific single chain variable fragment (scFv) and an IL-12 fusion protein (left), a fusion protein dimer comprising CD20-specific Fab and IL-12 (middle), and a fusion protein dimer comprisingCD20-specific a single chain variable fragment (scFv) and IL-12 (right).

### Mode for Carrying out the Invention

### Fusion protein comprising IL-12 and anti-CD20 antibody

In one aspect of the present invention, there is provided a fusion protein comprising IL-12 or a variant thereof, and an antigen binding site that specifically binds to CD20. Specifically, the fusion protein may comprise an immunoglobulin Fc region. Then, the IL-12 or the variant thereof may be a variant having low heparin binding ability.

In one embodiment, there is provided a fusion protein comprising a first monomer comprising IL-12 or a variant thereof; and a second monomer comprising an antigen binding site that specifically binds to CD20.

In another embodiment, there is provided a fusion protein dimer comprising IL-12 or a variant thereof, and an antigen binding site that specifically binds to CD20.

### IL-12 or variant thereof

As used herein, the term "IL-12" is a heterodimeric cytokine composed of the p35 and p40 subunits encoded by two separate genes, IL-12A and IL-12B, respectively. IL-12 is produced by antigen-presenting cells such as macrophages and binds to receptors on the cell surface of activated T cells and NK cells. IL-12 promotes the proliferation of T cells and NK cells, and enhances the cytotoxic effects of T cells, NK cells and macrophages. In addition, IL-12 induces the productions of IFN-γ, TNF-α and GM-CSF, and acts to induce activation of Th1 cells. On the other hand, IL-12 binds to the IL-12 receptor, a heterodimeric receptor formed by IL-12Rβ1 and IL-12Rβ2.

As used herein, the term "variant" of IL-12 comprises an amino acid sequence having a function identical to or similar to that of a wild-type IL-12 protein. Specifically, the amino acid sequence of the p35 and p40 subunits constituting IL-12 may have a substitution, an insertion or a deletion as compared to the wild-type.

The IL-12 or the variant thereof may comprise IL-12A (p35) or a variant thereof, and IL-12B (p40) or a variant thereof.

In addition, the IL-12 or the variant thereof may comprise a structure in which IL-12A or a variant thereof; and IL-12B or a variant thereof are bound by a peptide linker.

In one embodiment, the IL-12 or the variant thereof may be a fusion protein comprising the following structural formula (I) or (II):

N'-Y-[linker (1)]o-Z-C' (I)

N'-Z-[linker (1)]o-Y-C' (II)

in the structural formulas (I) and (II),
N' may be the N-terminus of the fusion protein,
C' may be the C-terminus of the fusion protein,
Y may be the IL-12A or the variant thereof,
Z may be the IL-12B or the variant thereof,
the linker (1) may be a peptide linker, and
o may be 0 or 1.

One embodiment of the IL-12 or the variant thereof may be a structure in which IL-12B or a variant thereof, a peptide linker, and IL-12A or a variant thereof are bound sequentially from the N-terminus. In addition, one embodiment of the IL-12 or the variant thereof may be a structure in which IL-12A or a variant thereof, a peptide linker, and IL-12B or a variant thereof are bound sequentially from the N-terminus.

As used herein, the term "IL-12A" is a 35 kDa subunit (p35) of IL-12, which binds to the 40 kDa subunit (p40) of IL-12 through a disulfide bond to generate an active cytokine.

The amino acid sequence of IL-12A may be one described in GenBank accession No. AAK84425 (for the amino acid sequence of mouse p35, see GenBank accession No. AAA39292). In addition, the IL-12A (p35) gene is a sequence encoding the IL-12A subunit, and may be a nucleotide sequence corresponding to a coding sequence (CDS) among the sequences described in GenBank accession No. AF404773 (for the mouse sequence, see GenBank accession No. M86672).

As used herein, the term "variant" of IL-12A comprises an amino acid sequence having a function identical to or similar to that of a wild-type IL-12A protein. Specifically, it means that the amino acid sequence of IL-12A has a substitution, an insertion or a deletion as compared to the wild-type. Specifically, the IL-12A variant may be a fragment of IL-12A, and may have an amino acid sequence in which the 1^{st} to 22^{nd} amino acids of SEQ ID NO: 138 are deleted. More specifically, the variant of IL-12A may have the amino acid sequence of SEQ ID NO: 125.

In addition, IL-12A used in one embodiment of the present invention may be the amino acid sequence of SEQ ID NO: 125 as a human IL-12A. In one embodiment, a mouse IL-12A may be the amino acid sequence of SEQ ID NO: 132.

As used herein, the term "IL-12B" refers to a 40 kDa subunit (p40) of IL-12, which binds to a 35 kDa subunit (p35) of IL-12 through a disulfide bond to form IL-12, and also binds to the subunit p19 of IL-23 to form IL-23. On the other hand, IL-12B is also called natural killer cell-stimulating factor 2.

The amino acid sequence of "IL-12B" may be one described in GenBank accession No. AAD56386 (for the amino acid sequence of mouse p40, see GenBank accession No. AAA39296). In addition, the IL-12B (p40) gene is a sequence encoding the IL-12B subunit, and may be a nucleotide sequence corresponding to a coding sequence (CDS) among the sequences described in GenBank accession No. AF180563 (for the mouse sequence, see GenBank accession No. M86671).

In addition, IL-12B used in one embodiment of the present invention may be a human IL-12B, and specifically, IL-12B may be the amino acid sequence of SEQ ID NO: 124. In one embodiment, a mouse IL-12B may have the amino acid sequence of SEQ ID NO: 131.

As used herein, the term "variant" of IL-12B comprises an amino acid sequence having a function identical to or similar to that of a wild-type IL-12B protein. Specifically, it means that the amino acid sequence of IL-12B has a substitution, an insertion or a deletion as compared to the wild-type.

Specifically, the IL-12B variant may be one in which the 1^{st} to 22^{nd} amino acids of SEQ ID NO: 137 are deleted. More specifically, the IL-12B variant may be one in which 1 to 8 amino acids are substituted in the sequence of SEQ ID NO: 124. In addition, the IL-12B variant may be one in which the 1^{st} to 22^{nd} amino acids of SEQ ID NO: 139 are deleted. More specifically, the IL-12B variant may be one in which at most 1 to 8 amino acids are substituted in the sequence of SEQ ID NO: 131.

The variant of the IL-12B (p40) may be one in which an amino acid involved in heparin binding of IL-12 is substituted.

In one embodiment, the variant of the IL-12B (p40) may be a form in which lysine (K) binding to heparin in IL-12B is substituted with another amino acid. Specifically, it may be a form in which the 258^{th}, 260^{th}, 263^{rd} and 264^{th} lysines of SEQ ID NO: 124 are substituted with other amino acids. Specifically, the variant of the IL-12B (p40) may comprise an amino acid sequence obtained by at least one substitution selected from the group consisting of K258A, K260A, K263A, and K264Ain the amino acid sequence of SEQ ID NO: 124. Specifically, the variant of the IL-12B (p40) may comprise an amino acid sequence obtained by a substitution of K258A and K263A in the amino acid sequence of SEQ ID NO: 124, or may further comprise a mutation of K260A and/or K264A in the amino acid sequence of SEQ ID NO: 124.

In one embodiment, the variant of the IL-12B (p40) may be a form in which arginine (R) or lysine (K) binding to heparin in IL-12B is substituted with another amino acid. Specifically, it may be a form in which the 254^{th} arginine and/or the 255^{th}, 256^{th} and 260^{th} lysines of SEQ ID NO: 131 are substituted with other amino acids. Specifically, the variant of the IL-12B (p40) may comprise an amino acid sequence obtained by at least one substitution selected from the group consisting of R254A, K255A, K256A and K260A in the amino acid sequence of SEQ ID NO: 131. Specifically, the variant of the IL-12B (p40) may comprise an amino acid sequence obtained by a substitution of R254A and K260A in the amino acid sequence of SEQ ID NO: 131, or may further comprise a mutation of K255A and/or K256A in the amino acid sequence of SEQ ID NO: 131.

In one embodiment, the variant of the IL-12B (p40) may comprise the amino acid sequence of SEQ ID NO: 127, SEQ ID NO: 129, SEQ ID NO: 134 or SEQ ID NO: 136.

In one embodiment, the human IL-12B variant may consist of the following structural formula A:

[Structural formula A] X₁-L-X₂

wherein, X₁ is the amino acid sequence of SEQ ID NO: 141,
X₂ is the amino acid sequence of SEQ ID NO: 142, and
L is an amino acid sequence consisting of V-A₁-A₂-Q-A₃-K^{∗}-A₄-A₅-A₆-A₇-K^{∗}-A₈.
A₁ is R or Q; A₂ is V, A or I; A₃ is G or R^{∗;} A₄ is S, N or K*; A₅ is K*, N or E; A₆ is R or K; A₇ is E, M or T; and As is K* or E.

At least one amino acid residue marked with "*" may be substituted with a non-polar amino acid residue selected from the group consisting of A, G, I, L, M, F, P and V, but is not limited thereto.

In one embodiment, the variant of the IL-12B (p40) may comprise a substitution of amino acids involved in heparin binding to reduce the cytokine-associated toxicity of IL-12 while maintaining the killing effect on cancer cells.

### CD20 and antigen binding site that specifically binds to CD20

As used herein, the term "CD20" is a hydrophobic transmembrane protein with a molecular weight of about 35 kDa that is expressed on the surface of pre-B lymphocytes and mature B lymphocytes. CD20 is highly expressed in 90% of B cell non-Hodgkin's lymphoma (NHL), but is not found in hematopoietic stem cells, pro-B cells, normal plasma cells or other normal tissues.

The CD20 may be used interchangeably with the 'CD20 antigen' and may comprise any variant, isoform and species homologue of human CD20 that is expressed either naturally by a cell or on a cell transfected with the CD20 gene. The antibody of the present invention can bind to CD20 and mediate death of cells that express CD20 (for example, tumor cells). The death of cells that express CD20 may occur by one or more of antibody-dependent cellular cytotoxicity (ADCC) and complement dependent cytotoxicity (CDC) mechanisms.

As used herein, the term "specifically binding" refers to binding that is measurably different from a non-specific interaction. Specifically binding may be determined by competing with a control molecule similar to a target that does not have binding activity.

As used herein, the term "antigen binding site (epitope)" refers to a determinant that interacts with a specific antigen binding site in a variable region of the antibody. The antigen binding site is a group of molecules, such as amino acids or sugar side chains, which usually have specific structural characteristics as well as specific charge characteristics. The antigen binding site that specifically binds to CD20 may be a generic term for molecules capable of antigen-antibody binding specifically to CD20.

In addition, the antibody or fragment thereof may be used in any form as long as it contains an antigen binding domain capable of specifically binding to CD20.

The antigen binding site may comprise other amino acids not directly involved in binding, or amino acids whose effects are blocked by amino acid residues of the antigen binding site.

Specifically, the antigen binding site may be an antibody that specifically binds to CD20 or a fragment thereof.

As used herein, the term "antibody" refers to a molecule containing an antigen binding site, and an immunologically active fragment of an immunoglobulin molecule containing an antigen binding site. The immunoglobulin molecule may be an immunoglobulin molecule of IgG, IgE, IgM, IgD, IgA, IgY or a subclass thereof. Antibodies comprise synthetic antibodies, monoclonal antibodies, single domain antibodies, single chain antibodies, recombinantly produced antibodies, multispecific antibodies (including bispecific antibodies), human antibodies, humanized antibodies, chimeric antibodies, intrabodies, scFv (including for example monospecific and bispecific, etc.), Fab fragment, F(ab') fragment, disulfide-linked Fv (sdFv), anti-idiotypic (anti-Id) antibodies, and an antigen binding fragment of the antibodies, but are not limited thereto.

As used herein, the term "antibody fragment" may be a portion of an antibody, such as F(ab')₂, F(ab)₂, Fab', Fab, Fv, scFv. Regardless of structure, an antibody fragment binds to the same antigen recognized by the intact antibody.

In one embodiment, the antigen binding site that specifically binds to CD20 may comprise a heavy chain variable region comprising HCDR1 of SEQ ID NO: 107, HCDR2 of SEQ ID NO: 108 and HCDR3 of SEQ ID NO: 109; and a light chain variable region comprising LCDR1 of SEQ ID NO: 110, LCDR2 of SEQ ID NO: 111 and LCDR3 of SEQ ID NO: 112. Then, in one embodiment, the antigen binding site that specifically binds to CD20 may have a heavy chain variable region of SEQ ID NO: 113 and a light chain variable region of SEQ ID NO: 114.

In addition, in one embodiment, the antigen binding site that specifically binds to CD20 may comprise a heavy chain variable region comprising HCDR1 of SEQ ID NO: 115, HCDR2 of SEQ ID NO: 116 and HCDR3 of SEQ ID NO: 117; and a light chain variable region comprising LCDR1 of SEQ ID NO: 118, LCDR2 of SEQ ID NO: 119 and LCDR3 of SEQ ID NO: 120. Then, in one embodiment, the antigen binding site that specifically binds to CD20 may have a heavy chain variable region of SEQ ID NO: 121 and a light chain variable region of SEQ ID NO: 122.

In one embodiment, the antigen binding site that specifically binds to CD20 may be scFv. Then, the scFv may comprise the above-described heavy chain CDRs and light chain CDRs. In one embodiment, the scFv that specifically binds to CD20 may have the amino acid sequence of SEQ ID NO: 88 or SEQ ID NO: 89.

In addition, the antigen binding site that specifically binds to CD20 may comprise a known anti-CD20 antibody or a fragment thereof. The anti-CD20 antibody or fragment thereof may refer to an antibody known to those skilled in the art without limitation.

The anti-CD20 antibody or fragment thereof may comprise at least one variable region selected from the group consisting of Ocrelizumab (RG-1594), Obinutuzumab (RG-7159), Ofatumumab (GSK-1841157), 2B8 (Biogen), Tositumomab (Bexxar), Ublituximab (EMAB-603), 7D8 of Epcortitamab (GEN-3013), Divozilimab (BCD-132), Odronextabmab (REGN-1979), Veltuzumab, MB-CART2019.1, Ocaratuzumab (LY-2469298), 3H7 of ADI-001, Pamotamab (THG-338), hA20 of DNL-1 and mAb-1.5.3.

In another example, as the antibody, an anti-CD20 antibody or a fragment thereof disclosed in International Patent Application Publication No. WO 2004-056312 A2, International Patent Application Publication No. WO 2005-044859 A2, US Patent No. US 8,529,902 B2, US Patent No. US 8,563,269 B2, US Patent No. US 9,694,071 B2, International Patent Application Publication No. WO 2019-155008 A1, International Patent Application Publication No. WO 2020-091634 A1, US Patent Application Publication No. US 2017-0174781 A1, International Patent Application Publication No. WO 2003-068821 A2, US Patent Application Publication No. US 2020-0109210 A1, US Patent No. US 8,153,125 B2, International Patent Application Publication No. WO 2020-072536 A1, US Patent Application Publication No. US 2021-0095027 A1, International Patent Application Publication No. WO 2003-068821 A2 or US Patent Application Publication No. US 2011-0129412 A1 may be used.

In one embodiment, the anti-CD20 antibody may comprise a variable region of Ocrelizumab. Specifically, the antibody may comprise a heavy chain variable region comprising HCDR1 of SEQ ID NO: 143, HCDR2 of SEQ ID NO: 144 and HCDR3 of SEQ ID NO: 145; and a light chain variable region comprising LCDR1 of SEQ ID NO: 146, LCDR2 of SEQ ID NO: 147 and LCDR3 of SEQ ID NO: 148. In addition, the anti-CD20 antibody may comprise a heavy chain variable region of SEQ ID NO: 240 and a light chain variable region of SEQ ID NO: 241.

In one embodiment, the anti-CD20 antibody may comprise a variable region of Afutuzumab or Obinutuzumab. Specifically, the antibody may comprise a heavy chain variable region comprising HCDR1 of SEQ ID NO: 149, HCDR2 of SEQ ID NO: 150 and HCDR3 of SEQ ID NO: 151; and a light chain variable region comprising LCDR1 of SEQ ID NO: 152, LCDR2 of SEQ ID NO: 153 and LCDR3 of SEQ ID NO: 154. In addition, the anti-CD20 antibody may comprise a heavy chain variable region of SEQ ID NO: 242 and a light chain variable region of SEQ ID NO: 243.

In one embodiment, the anti-CD20 antibody may comprise a variable region of Ofatumumab. Specifically, the antibody may comprise a heavy chain variable region comprising HCDR1 of SEQ ID NO: 155, HCDR2 of SEQ ID NO: 156 and HCDR3 of SEQ ID NO: 157; and a light chain variable region comprising LCDR1 of SEQ ID NO: 158, LCDR2 of SEQ ID NO: 159 and LCDR3 of SEQ ID NO: 160. In addition, the anti-CD20 antibody may comprise a heavy chain variable region of SEQ ID NO: 244 and a light chain variable region of SEQ ID NO: 245.

In one embodiment, the anti-CD20 antibody may comprise a variable region of 2B8. Specifically, the antibody may comprise a heavy chain variable region comprising HCDR1 of SEQ ID NO: 161, HCDR2 of SEQ ID NO: 162 and HCDR3 of SEQ ID NO: 163; and a light chain variable region comprising LCDR1 of SEQ ID NO: 164, LCDR2 of SEQ ID NO: 165 and LCDR3 of SEQ ID NO: 166. In addition, the anti-CD20 antibody may comprise a heavy chain variable region of SEQ ID NO: 246 and a light chain variable region of SEQ ID NO: 247.

In one embodiment, the anti-CD20 antibody may comprise a variable region of Tositumomab. Specifically, the antibody may comprise a heavy chain variable region comprising HCDR1 of SEQ ID NO: 167, HCDR2 of SEQ ID NO: 168 and HCDR3 of SEQ ID NO: 169; and a light chain variable region comprising LCDR1 of SEQ ID NO: 170, LCDR2 of SEQ ID NO: 171 and LCDR3 of SEQ ID NO: 172. In addition, the anti-CD20 antibody may comprise a heavy chain variable region of SEQ ID NO: 248 and a light chain variable region of SEQ ID NO: 249.

In one embodiment, the anti-CD20 antibody may comprise a variable region of Ublituximab. Specifically, the antibody may comprise a heavy chain variable region comprising HCDR1 of SEQ ID NO: 173, HCDR2 of SEQ ID NO: 174 and HCDR3 of SEQ ID NO: 175; and a light chain variable region comprising LCDR1 of SEQ ID NO: 176, LCDR2 of SEQ ID NO: 177 and LCDR3 of SEQ ID NO: 178. In addition, the anti-CD20 antibody may comprise a heavy chain variable region of SEQ ID NO: 250 and a light chain variable region of SEQ ID NO: 251.

In one embodiment, the anti-CD20 antibody may comprise a variable region of Epcortitamab. Specifically, the antibody may comprise a heavy chain variable region comprising HCDR1 of SEQ ID NO: 179, HCDR2 of SEQ ID NO: 180 and HCDR3 of SEQ ID NO: 181; and a light chain variable region comprising LCDR1 of SEQ ID NO: 182, LCDR2 of SEQ ID NO: 183 and LCDR3 of SEQ ID NO: 184. In addition, the anti-CD20 antibody may comprise a heavy chain variable region of SEQ ID NO: 252 and a light chain variable region of SEQ ID NO: 253.

In one embodiment, the anti-CD20 antibody may comprise a variable region of Divozilimab. Specifically, the antibody may comprise a heavy chain variable region comprising HCDR1 of SEQ ID NO: 185, HCDR2 of SEQ ID NO: 186 and HCDR3 of SEQ ID NO: 187; and a light chain variable region comprising LCDR1 of SEQ ID NO: 188, LCDR2 of SEQ ID NO: 189 and LCDR3 of SEQ ID NO: 190. In addition, the anti-CD20 antibody may comprise a heavy chain variable region of SEQ ID NO: 254 and a light chain variable region of SEQ ID NO: 255.

In one embodiment, the anti-CD20 antibody may comprise a variable region of Odronextabmab. Specifically, the antibody may comprise a heavy chain variable region comprising HCDR1 of SEQ ID NO: 191, HCDR2 of SEQ ID NO: 192 and HCDR3 of SEQ ID NO: 193; and a light chain variable region comprising LCDR1 of SEQ ID NO: 194, LCDR2 of SEQ ID NO: 195 and LCDR3 of SEQ ID NO: 196. In addition, the anti-CD20 antibody may comprise a heavy chain variable region of SEQ ID NO: 256 and a light chain variable region of SEQ ID NO: 257.

In one embodiment, the anti-CD20 antibody may comprise a variable region of Veltuzumab. Specifically, the antibody may comprise a heavy chain variable region comprising HCDR1 of SEQ ID NO: 197, HCDR2 of SEQ ID NO: 198 and HCDR3 of SEQ ID NO: 199 or SEQ ID NO: 200; and a light chain variable region comprising LCDR1 of SEQ ID NO: 201, LCDR2 of SEQ ID NO: 202 and LCDR3 of SEQ ID NO: 203. In addition, the anti-CD20 antibody may comprise a heavy chain variable region of SEQ ID NO: 258 and a light chain variable region of SEQ ID NO: 259.

In one embodiment, the anti-CD20 antibody may comprise a variable region of MB-CART2019.1. Specifically, the antibody may comprise a heavy chain variable region comprising HCDR1 of SEQ ID NO: 204, HCDR2 of SEQ ID NO: 205 and HCDR3 of SEQ ID NO: 206; and a light chain variable region comprising LCDR1 of SEQ ID NO: 207, LCDR2 of SEQ ID NO: 208 and LCDR3 of SEQ ID NO: 209. In addition, the anti-CD20 antibody may comprise a heavy chain variable region of SEQ ID NO: 260 and a light chain variable region of SEQ ID NO: 261.

In one embodiment, the anti-CD20 antibody may comprise a variable region of Ocaratuzumab. Specifically, the antibody may comprise a heavy chain variable region comprising HCDR1 of SEQ ID NO: 210, HCDR2 of SEQ ID NO: 211 and HCDR3 of SEQ ID NO: 212; and a light chain variable region comprising LCDR1 of SEQ ID NO: 213, LCDR2 of SEQ ID NO: 214 and LCDR3 of SEQ ID NO: 215. In addition, the anti-CD20 antibody may comprise a heavy chain variable region of SEQ ID NO: 262 and a light chain variable region of SEQ ID NO: 263.

In one embodiment, the anti-CD20 antibody may comprise a variable region of ADI-001. Specifically, the antibody may comprise a heavy chain variable region comprising HCDR1 of SEQ ID NO: 216, HCDR2 of SEQ ID NO: 217 and HCDR3 of SEQ ID NO: 218; and a light chain variable region comprising LCDR1 of SEQ ID NO: 219, LCDR2 of SEQ ID NO: 220 and LCDR3 of SEQ ID NO: 221. In addition, the anti-CD20 antibody may comprise a heavy chain variable region of SEQ ID NO: 264 and a light chain variable region of SEQ ID NO: 265.

In one embodiment, the anti-CD20 antibody may comprise a variable region of Pamotamab. Specifically, the antibody may comprise a heavy chain variable region comprising HCDR1 of SEQ ID NO: 222, HCDR2 of SEQ ID NO: 223 and HCDR3 of SEQ ID NO: 224; and a light chain variable region comprising LCDR1 of SEQ ID NO: 225, LCDR2 of SEQ ID NO: 226 and LCDR3 of SEQ ID NO: 227. In addition, the anti-CD20 antibody may comprise a heavy chain variable region of SEQ ID NO: 266 and a light chain variable region of SEQ ID NO: 267.

In one embodiment, the anti-CD20 antibody may comprise a variable region of DNL-1. Specifically, the antibody may comprise a heavy chain variable region comprising HCDR1 of SEQ ID NO: 228, HCDR2 of SEQ ID NO: 229 and HCDR3 of SEQ ID NO: 230; and a light chain variable region comprising LCDR1 of SEQ ID NO: 231, LCDR2 of SEQ ID NO: 232 and LCDR3 of SEQ ID NO: 233. In addition, the anti-CD20 antibody may comprise a heavy chain variable region of SEQ ID NO: 268 and a light chain variable region of SEQ ID NO: 269.

In one embodiment, the anti-CD20 antibody may comprise a variable region of mAb-1.5.3. Specifically, the antibody may comprise a heavy chain variable region comprising HCDR1 of SEQ ID NO: 234, HCDR2 of SEQ ID NO: 235 and HCDR3 of SEQ ID NO: 236; and a light chain variable region comprising LCDR1 of SEQ ID NO: 237, LCDR2 of SEQ ID NO: 238 and LCDR3 of SEQ ID NO: 239. In addition, the anti-CD20 antibody may comprise a heavy chain variable region of SEQ ID NO: 270 and a light chain variable region of SEQ ID NO: 271.

The anti-CD20 antibody specifically binds to CD20 present on cancer cells, and any antibody may be used as long as it may induce the death of cancer cells.

### Structure of first monomer

The first monomer comprises IL-12 or a variant thereof.

The first monomer may further comprise an antigen binding site that specifically binds to CD20.

In one embodiment, the first monomer may comprise IL-12 or a variant thereof; and an Fc region fragment or a variant thereof.

In one embodiment, the first monomer may comprise IL-12 or a variant thereof, an Fc region fragment or a variant thereof, and an antigen binding site that specifically binds to CD20.

The first monomer may comprise the following structural formula (III) or (IV):

N'-X-[linker (2)]p-Fc region fragment or variant thereof-[linker (3)]q-(T)r-C' (III)

N'-(T)r-[linker (2)]q-Fc region fragment or variant thereof-[linker (3)]p-X-C' (IV)

in the structural formulas (III) and (IV),
N' may be the N-terminus of the fusion protein,
C' may be the C-terminus of the fusion protein,
X may be the structural formula (I) or (II),
T may be the antigen binding site that specifically binds to CD20,
the linkers (2) and (3) may be peptide linkers, and
p, q and r may be each independently 0 or 1.

In one embodiment, when r is 0, the first monomer may be in a form of a fusion protein in which IL-12 or a variant thereof and an Fc region fragment or a variant thereof are linked by a peptide linker.

Then, the first monomer may comprise the amino acid sequence of SEQ ID NO: 3, SEQ ID NO: 4, SEQ ID NO: 5, SEQ ID NO: 13, SEQ ID NO: 14, SEQ ID NO: 15, SEQ ID NO: 24, SEQ ID NO: 25, SEQ ID NO: 26, SEQ ID NO: 34, SEQ ID NO: 35 or SEQ ID NO: 36.

Then, in the structural formula (III), when r is 1, the first monomer may be in a form in which a monomer of an anti-CD20 antibody is bound to IL-12 or a variant thereof.

Then, in the structural formula (III), when r is 1, the first monomer may comprise the amino acid sequence of SEQ ID NO: 272, SEQ ID NO: 273, SEQ ID NO: 276, SEQ ID NO: 277, SEQ ID NO: 278, SEQ ID NO: 279, SEQ ID NO: 282 or SEQ ID NO: 283.

Then, in the structural formula (IV), when r is 1, the first monomer may comprise the amino acid sequence of SEQ ID NO: 274, SEQ ID NO: 275, SEQ ID NO: 280 or SEQ ID NO: 281.

In one embodiment, when r is 1, the structural formula (III) may comprise the following structural formulas (III') and (III"):

N'-X-[linker (2)]p-Fc region fragment or variant thereof-[linker (3)]q-(T')-C' (III')

N'-(T")-C' (III")

in the structural formulas (III') and (III"),
T' is a heavy chain region of an antibody that specifically binds to CD20, comprising a variable region and a CH1 region, or a light chain region of the antibody;
T" is a light chain region of an antibody that specifically binds to CD20, or a heavy chain region of the antibody, comprising a variable region and a CH1 region;
wherein, T' and T" bind to each other to form a variable region of the antibody, wherein the variable region specifically binds to CD20;
the linkers (2) to (3) are peptide linkers,
p and q are each independently 0 or 1, and
N', X and C' are as defined above.

In one embodiment, when r is 1, the structural formula (IV) may comprise the following structural formulas (IV') and (IV"):

N'-(T')-[linker (2)]q-Fc region fragment or variant thereof-[linker (3)]p-X-C' (IV');

and

N'-(T")-C' (IV")

in the structural formulas (IV') and (IV"),
T' is a heavy chain region of an antibody that specifically binds to CD20, comprising a variable region and a CH1 region, or a light chain region of the antibody;
T" is a light chain region of an antibody that specifically binds to CD20, or a heavy chain region of the antibody, comprising a variable region and a CH1 region;
wherein, T' and T" bind to each other to form a variable region of the antibody, wherein the variable region specifically binds to CD20;
the linkers (2) to (3) are peptide linker,
p and q are each independently 0 or 1, and
N', X and C' are as defined above.

Then, the first monomer may comprise the amino acid sequence of SEQ ID NO: 10 and SEQ ID NO: 2; SEQ ID NO: 11 and SEQ ID NO: 2; SEQ ID NO: 31 and SEQ ID NO: 23; or SEQ ID NO: 32 and SEQ ID NO: 23; SEQ ID NO: 274 and SEQ ID NO: 2; SEQ ID NO: 275 and SEQ ID NO: 2; SEQ ID NO: 280 and SEQ ID NO: 23; or SEQ ID NO: 281 and SEQ ID NO: 23.

### Structure of second monomer

The second monomer may further comprise an antigen binding site that specifically binds to CD20.

In one embodiment, the second monomer may comprise a first CD20 binding site; and a second CD20 binding site.

The antigen binding site that specifically binds to CD20 may be Fab, scFv, Fv, or a fragment thereof. Then, the first CD20 binding site may be Fab, and the second CD20 binding site may be Fv or scFv.

The second CD20 binding site may be bound to the N-terminus or C-terminus of the second monomer. Specifically, the second CD20 binding site may be additionally bound to the C-terminus of the heavy chain, the C-terminus of the light chain, or the N-terminus of the variable region.

The second monomer may comprise the following structural formula (V):

N'-(R)s-[linker (4)]t-Q-[linker (5)]u-Fc region fragment or variant thereof-[linker (6)]v-(W)a-C' (V)

in the structural formula (V),
N' may be the N-terminus of the fusion protein,
C' may be the C-terminus of the fusion protein,
R and Q may be an antigen binding site that specifically binds to CD20,
W may be scFv that specifically binds to CD20; or the IL-12 of structural formula (I) or (II) or the variant thereof,
the linkers (4), (5) and (6) may be each peptide linkers, and
s, t, u, v and a may be each independently 0 or 1.

In one embodiment, the structural formula (V) may comprise at least one amino acid sequence selected from the group consisting of SEQ ID NO: 1, SEQ ID NO: 2, SEQ ID NO: 6, SEQ ID NO: 7, SEQ ID NO: 8, SEQ ID NO: 9, SEQ ID NO: 12, SEQ ID NO: 21, SEQ ID NO: 22, SEQ ID NO: 23, SEQ ID NO: 27, SEQ ID NO: 28, SEQ ID NO: 29, SEQ ID NO: 30, SEQ ID NO: 33, SEQ ID NO: 42, SEQ ID NO: 274, SEQ ID NO: 275, SEQ ID NO: 280 and SEQ ID NO: 281.

In one embodiment, the structural formula (V) may comprise the following structural formulas (V') and (V"):

N'-(R')s-[linker (4)]t-Q'-[linker (5)]u-Fc region fragment or variant thereof-[linker (6)]p-(W)a-C (V)

N'-(R")s-[linker (4)]t-Q"-[linker (7)]x-(W)b-C' (V")

in the structural formulas (V') and (V"),
R' may be a heavy chain region of an antibody that specifically binds to CD20, comprising a variable region and a CH1 region, or a light chain region of the antibody;
R" may be a light chain region of an antibody that specifically binds to CD20, or a heavy chain region of the antibody, comprising a variable region and a CH1 region;
wherein R' and R" may bind to each other to form a variable region of the antibody, wherein the variable region specifically binds to CD20;
Q' may be a heavy chain region of an antibody that specifically binds to CD20, comprising a variable region and a CH1 region, or a light chain region of the antibody;
Q" may be a light chain region of an antibody that specifically binds to CD20, or a heavy chain region of the antibody, comprising a variable region and a CH1 region;
wherein Q' and Q" may bind to each other to form a variable region of the antibody, wherein the variable region specifically binds to CD20,
W may be scFv that specifically binds to CD20; or the IL-12 of structural formula (I) or (II) or the variant thereof,
the linkers (4), (5), (6) and (7) may be each peptide linkers, and
s, t, u, x, a and b may be each independently 0 or 1.

In one embodiment, the structural formula (V') may be SEQ ID NO: 1, SEQ ID NO: 6, SEQ ID NO: 8, SEQ ID NO: 12, SEQ ID NO: 21, SEQ ID NO: 22, SEQ ID NO: 27, SEQ ID NO: 29, SEQ ID NO: 33, SEQ ID NO: 42, SEQ ID NO: 274, SEQ ID NO: 275, SEQ ID NO: 280 or SEQ ID NO: 281.

In one embodiment, the structural formula (V") may be SEQ ID NO: 2, SEQ ID NO: 7, SEQ ID NO: 9, SEQ ID NO: 23, SEQ ID NO: 28 or SEQ ID NO: 30.

In one embodiment, the second monomer may comprise a heavy chain consisting of the amino acid sequence of SEQ ID NO: 113 and a light chain consisting of the amino acid sequence of SEQ ID NO: 114; or a heavy chain consisting of the amino acid sequence of SEQ ID NO: 121 and a light chain consisting of the amino acid sequence of SEQ ID NO: 122.

In addition, when W is scFv, W may have the amino acid sequence of SEQ ID NO: 88 or SEQ ID NO: 89.

### Peptide linker

As used herein, the term "peptide linker" refers to a peptide used to provide a physicochemical distance or to connect between domains in a fusion protein. The linker may comprise a hinge region of an immunoglobulin.

The peptide linkers (1) to (7) refer to peptide linkers composed of amino acids. Specifically, the peptide linker (2) or (5) may consist of 5 to 80 consecutive amino acids, 7 to 70 consecutive amino acids, or 10 to 60 consecutive amino acids, or 12 to 50 amino acids. The peptide linker may comprise (G₄S)n (wherein, n is an integer from 1 to 10). Then, in (G₄S)n, n may be each 1, 2, 3, 4, 5, 6, 7, 8, 9 or 10.

The peptide linker (2) or (5) may consist of 5 to 80 consecutive amino acids, 7 to 70 consecutive amino acids, or 10 to 60 consecutive amino acids, or 12 to 50 amino acids. In one embodiment, the peptide linker (2) may consist of 30 amino acids. In addition, the peptide linker (2) may comprise at least one cysteine. Specifically, it may comprise one, two or three cysteines. In addition, the peptide linker (2) may be derived from a hinge of an immunoglobulin, and may further comprise (G₄S)n (wherein, n is an integer from 1 to 10). Specifically, the peptide linker (2) or (5) may comprise a hinge region consisting of an amino acid sequence of any one of SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101 and SEQ ID NO: 102.

In addition, the peptide linkers (3), (4), (6) and (7) may consist of 1 to 30 consecutive amino acids, 5 to 20 consecutive amino acids, or 10 to 15 consecutive amino acids. The peptide linker may comprise (G₄S)n (wherein, n is an integer from 1 to 10). Then, in (G₄S)n, n may be each 1, 2, 3, 4, 5, 6, 7, 8, 9 or 10.

In one embodiment, the peptide linker (1) may be a peptide linker consisting of the amino acid sequence of SEQ ID NO: 91, SEQ ID NO: 92 or SEQ ID NO: 93.

In addition, specifically, the peptide linker (2), (3) or (5) may comprise SEQ ID NO: 91 or SEQ ID NO: 92.

In one embodiment of the present invention, the peptide linker (2) or (5) may be a peptide linker comprising the amino acid sequence of SEQ ID NO: 100 or SEQ ID NO: 102.

### Fc region or fragment thereof

Then, the above-described immunoglobulin fragment may be an Fc region of an immunoglobulin. The Fc region of an immunoglobulin may be a wild-type Fc domain as well as an Fc domain variant. Then, the Fc region may be an Fc region of IgG, IgA, IgE, IgD or IgM. Specifically, it may be derived from IgG1 or IgG2a.

As used herein, the term "Fc domain variant" may refer to a form which is different from the wild-type Fc domain in terms of glycosylation pattern, has a high glycosylation as compared with the wild-type Fc domain, has a low glycosylation as compared with the wild-type Fc domain, or has a deglycosylated form. In addition, an aglycosylated Fc domain is comprised therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD or IgM. In addition, the Fc domain variant may be in a form in which some amino acids of the Fc domain are substituted with other amino acids.

The "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y) and valine (V).

In addition, the Fc region may comprise a knob structure or a hole structure.

As used herein, the term "knob-into-hole" is a design strategy for manufacturing an antibody that specifically binds to different regions, such as a bispecific antibody, a multispecific antibody, or a heterodimeric antibody. In general, this technique involves introducing a knob at the interface of a first polypeptide (for example, a first CH3 domain of a first antibody heavy chain) and a corresponding hole at the interface of a second polypeptide (for example, a second CH3 domain of a second antibody heavy chain), so that the knob may be positioned within the hole to promote heterodimer formation and hinder homodimer formation.

A 'knob' is constructed by replacing small amino acid side chains from the interface of a first polypeptide (for example, a first CH3 domain of a first antibody heavy chain) with larger side chains (for example, arginine, phenylalanine, tyrosine or tryptophan). A complementary 'hole' of the same or similar size in the knob is produced by replacing large amino acid side chains from the interface of a second polypeptide (for example, a second CH3 domain of a second antibody heavy chain) with smaller side chains (for example, alanine, serine, valine, or threonine). The knob and hole may be produced by altering a nucleic acid encoding a polypeptide, for example, by site-specific mutagenesis or by peptide synthesis.

In one embodiment, the knob structure of the IgG1 may be SEQ ID NO: 19 or 103, and the hole structure may be SEQ ID NO: 20 or 104. The knob structure or the hole structure may be a form in which the 146^{th}, 148^{th} and 187^{th} amino acids of the amino acid sequence of SEQ ID NO: 284 are substituted with other amino acids. Specifically, in one embodiment of the present invention, the knob structure or the hole structure may be a form in which the amino acid sequence of SEQ ID NO: 284 is substituted with T146W, T146S, L148A and Y187V. Specifically, the knob structure may be a form in which it is substituted with T146W, and the hole structure may be a form in which it is substituted with T146S, L148A and Y187V.

On the other hand, in one embodiment of the present invention, the knob structure of the IgG2a may be SEQ ID NO: 40 or 105, and the hole structure may be SEQ ID NO: 41 or 106. The knob structure or the hole structure may be a form in which the 152^{nd}, 154^{th} and 193^{rd} amino acids of the amino acid sequence of SEQ ID NO: 285 are substituted with other amino acids. Specifically, in one embodiment of the present invention, the knob structure or the hole structure may be a form in which the amino acid sequence of SEQ ID NO: 285 is substituted with T152W or T152S, M154A and Y193V. Specifically, the knob structure may be a form in which it is substituted with T152W, and the hole structure may be a form in which it is substituted with T152S, M154A and Y193V.

In one embodiment, the Fc domain variant may comprise a DANG mutation or an NG mutation. Then, a "DANG mutation" refers to the D265A/N297G mutation for removing an effector function in human IgG1 or mouse IgG2a.

The effector functions mediated by the Fc region in an IgG molecule comprise C1q binding, complement-dependent cytotoxicity, Fc receptor binding, antibody dependent cell mediated cytotoxicity (ADCC), phagocytosis, downregulation of cell surface receptors (for example, B cell receptor, BCR) and the like. In general, these effector functions require the binding of the Fc region with a binding domain (for example, an antibody variable domain).

The effector function may be changed by the substitution of the amino acid sequence of the non-mutated Fc region, and the Fc region in which the effector function is changed may be designed for example, by modifying C1q binding and/or FcR binding, thereby changing CDC activity and/or ADCC activity. That is, the 'DANG mutation' means that the effector function mediated by the Fc region is removed from the IgG molecule so that an unwanted effector function does not occur during antibody production.

In one embodiment, the DANG mutation may be a form in which the amino acid in human IgG1 of SEQ ID NO: 104 is substituted with D45A/N77G. In addition, it may be a form in which the amino acid in mouse IgG2a of SEQ ID NO: 106 is substituted with D51A/N83G.

### Structure of fusion protein

The fusion protein may be an antibody. Specifically, the antibody may be a heterodimeric antibody including a knob-into-hole structure.

The fusion protein may comprise structural formula (III) and structural formula (V); or structural formula (IV) and structural formula (V).

More specifically, one embodiment of the fusion protein may comprise a first monomer of (i) or (ii) below; and a second monomer of (iii), (iv), (v) or (vi) below:

### Examples of a first monomer:

(i) when r is 0, structural formula (III)
(ii) when r is 1, structural formula (IV)

### Examples of a second monomer:

(iii) when s, a and b are 0, structural formula (V') and structural formula (V")
(iv) when s is 1 and a and b are 0, structural formula (V') and structural formula (V")
(v) when s and b are 0 and a is 1, structural formula (V') and structural formula (V")
(vi) when b is 1 and s and a are 0, structural formula (V') and structural formula (V").

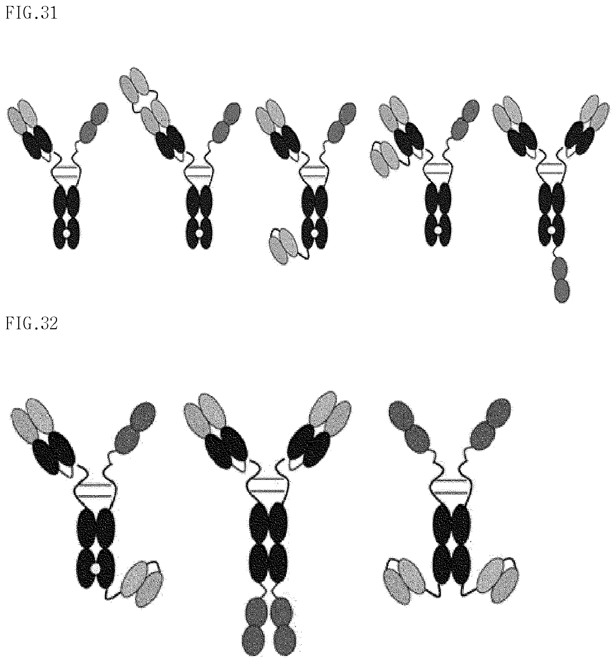

In one embodiment, the fusion protein may comprise a first monomer comprising (i) above; and a second monomer comprising (iii) above (first from the left in FIG. 31). Then, the fusion protein may comprise at least one amino acid sequence selected from the group consisting of SEQ ID NO: 1, SEQ ID NO: 2, SEQ ID NO: 3, SEQ ID NO: 4, SEQ ID NO: 5, SEQ ID NO: 12, SEQ ID NO: 13, SEQ ID NO: 14, SEQ ID NO: 22, SEQ ID NO: 23, SEQ ID NO: 24, SEQ ID NO: 25, SEQ ID NO: 26, SEQ ID NO: 33, SEQ ID NO: 34 and SEQ ID NO: 35. Specifically, the fusion protein may comprise SEQ ID NO: 1, SEQ ID NO: 2 and SEQ ID NO: 3; SEQ ID NO: 1, SEQ ID NO: 2 and SEQ ID NO: 4; SEQ ID NO: 1, SEQ ID NO: 2 and SEQ ID NO: 5; SEQ ID NO: 2, SEQ ID NO: 12 and SEQ ID NO: 13; SEQ ID NO: 2, SEQ ID NO: 12 and SEQ ID NO: 14; SEQ ID NO: 22, SEQ ID NO: 23 and SEQ ID NO: 24; SEQ ID NO: 22, SEQ ID NO: 23 and SEQ ID NO: 25; SEQ ID NO: 22, SEQ ID NO: 23 and SEQ ID NO: 26; SEQ ID NO: 23, SEQ ID NO: 33 and SEQ ID NO: 34; or SEQ ID NO: 23, SEQ ID NO: 33 and SEQ ID NO: 35.

In one embodiment, the fusion protein may comprise a first monomer comprising (i) above; and a second monomer comprising (iv) above (second from the left in FIG. 31). Then, the fusion protein may comprise at least one amino acid sequence selected from the group consisting of SEQ ID NO: 3, SEQ ID NO: 5, SEQ ID NO: 6, SEQ ID NO: 7, SEQ ID NO: 24, SEQ ID NO: 26, SEQ ID NO: 27 and SEQ ID NO: 28. Specifically, the fusion protein may comprise SEQ ID NO: 3, SEQ ID NO: 6 and SEQ ID NO: 7; SEQ ID NO: 5, SEQ ID NO: 6 and SEQ ID NO: 7; SEQ ID NO: 24, SEQ ID NO: 27 and SEQ ID NO: 28; or SEQ ID NO: 26, SEQ ID NO: 27 and SEQ ID NO: 28.

In one embodiment, the fusion protein may comprise a first monomer comprising (i) above; and a second monomer comprising (v) above (third from the left in FIG. 31). Then, the fusion protein may comprise at least one amino acid sequence selected from the group consisting of SEQ ID NO: 2, SEQ ID NO: 3, SEQ ID NO: 5, SEQ ID NO: 8, SEQ ID NO: 23, SEQ ID NO: 24, SEQ ID NO: 26 and SEQ ID NO: 29. Specifically, the fusion protein may comprise SEQ ID NO: 2, SEQ ID NO: 3 and SEQ ID NO: 8; SEQ ID NO: 2, SEQ ID NO: 5 and SEQ ID NO: 8; SEQ ID NO: 23, SEQ ID NO: 24 and SEQ ID NO: 29; or SEQ ID NO: 23, SEQ ID NO: 26 and SEQ ID NO: 29.

In one embodiment, the fusion protein may comprise a first monomer comprising (i) above; and a second monomer comprising (vi) above (fourth from the left in FIG. 31). Then, the fusion protein may comprise at least one amino acid sequence selected from the group consisting of SEQ ID NO: 1, SEQ ID NO: 3, SEQ ID NO: 5, SEQ ID NO: 9, SEQ ID NO: 22, SEQ ID NO: 24, SEQ ID NO: 26 and SEQ ID NO: 30. Specifically, the fusion protein may comprise SEQ ID NO: 1, SEQ ID NO: 3 and SEQ ID NO: 9; SEQ ID NO: 1, SEQ ID NO: 5 and SEQ ID NO: 9; SEQ ID NO: 22, SEQ ID NO: 24 and SEQ ID NO: 30; or SEQ ID NO: 22, SEQ ID NO: 26 and SEQ ID NO: 30.

In one embodiment, the fusion protein may comprise a first monomer comprising (ii) above; and a second monomer comprising (iii) above (fifth from the left in FIG. 31). Then, the fusion protein may comprise at least one amino acid sequence selected from the group consisting of SEQ ID NO: 1, SEQ ID NO: 2, SEQ ID NO: 10, SEQ ID NO: 11, SEQ ID NO: 22, SEQ ID NO: 23, SEQ ID NO: 31 and SEQ ID NO: 32. Specifically, the fusion protein may comprise SEQ ID NO: 1, SEQ ID NO: 2 and SEQ ID NO: 10; SEQ ID NO: 1, SEQ ID NO: 2 and SEQ ID NO: 11; SEQ ID NO: 22, SEQ ID NO: 23 and SEQ ID NO: 31; or SEQ ID NO: 22, SEQ ID NO: 23 and SEQ ID NO: 32.

In addition, in one embodiment, a fusion protein dimer comprising IL-12 or a variant thereof and an antigen binding site that specifically binds to CD20 may be a fusion protein dimer comprising the monomer of the structural formula (III) and the monomer of the structural formula (V) (left in FIG. 32). Then, in the structural formula (III), r is 1, and in the structural formula (V), s, a and t are 0. Then, the structural formula (V) may comprise (V') and (V"). Then, in the (V') and (V"), s, a, t and b are 0. In addition, the Fc region of the dimer has a knob-into-hole structure, through which different fusion proteins may be bound to each other.

Specifically, the fusion protein may comprise at least one amino acid sequence selected from the group consisting of SEQ ID NO: 1, SEQ ID NO: 2, SEQ ID NO: 22, SEQ ID NO: 23, SEQ ID NO: 272, SEQ ID NO: 273, SEQ ID NO: 278 and SEQ ID NO: 279. Specifically, the fusion protein may comprise SEQ ID NO: 1, SEQ ID NO: 2 and SEQ ID NO: 272; SEQ ID NO: 1, SEQ ID NO: 2 and SEQ ID NO: 273; SEQ ID NO: 22, SEQ ID NO: 23 and SEQ ID NO: 278; or SEQ ID NO: 22, SEQ ID NO: 23 and SEQ ID NO: 279.

In addition, a fusion protein dimer comprising IL-12 or a variant thereof and an antigen binding site that specifically binds to CD20 may be a fusion protein dimer including a structure of the structural (V) (middle in FIG. 32). Then, the fusion protein dimer may comprise (V') and (V"). Then, in the structural formula (V), s and t are 0, and a is 1. In addition, in the (V') and (V"), s, t and b are 0, and a is 1. In addition, the fusion protein dimer may be a fusion protein dimer including a structure of the structural formula (IV). Then, in the structural formula (IV), r is 1.

Specifically, the fusion protein may comprise at least one amino acid sequence selected from the group consisting of SEQ ID NO: 2, SEQ ID NO: 23, SEQ ID NO: 274, SEQ ID NO: 275, SEQ ID NO: 280 and SEQ ID NO: 281. Specifically, the fusion protein may comprise SEQ ID NO: 2 and SEQ ID NO: 274; SEQ ID NO: 2 and SEQ ID NO: 275; SEQ ID NO: 23 and SEQ ID NO: 280; or SEQ ID NO: 23 and SEQ ID NO: 281.

In one embodiment, a fusion protein dimer comprising IL-12 or a variant thereof and an antigen binding site that specifically binds to CD20 may be a fusion protein dimer including a structure of the structural formula (III) (right in FIG. 32). Then, in the structural formula (III), r is 1.

Specifically, the fusion protein may comprise SEQ ID NO: 276, SEQ ID NO: 277, SEQ ID NO: 282 or SEQ ID NO: 283.

The multispecific fusion protein herein may be in a chemically modified form. In one embodiment, the fusion protein may be chemically modified by glycosylation, acetylation, PEGylation, phosphorylation, amidation, derivatization with known protecting/blocking groups, proteolytic cleavage and/or binding to cellular ligands or other proteins. Many of these chemical modifications may be performed by known techniques.

### Polynucleotide encoding fusion protein

In another aspect of the present invention, there is provided a polynucleotide encoding the first monomer or a polynucleotide encoding the second monomer.

In one embodiment, a polypeptide of the first monomer may comprise an amino acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 3, SEQ ID NO: 4, SEQ ID NO: 5, SEQ ID NO: 10, SEQ ID NO: 11, SEQ ID NO: 13, SEQ ID NO: 14, SEQ ID NO: 15, SEQ ID NO: 24, SEQ ID NO: 25, SEQ ID NO: 26, SEQ ID NO: 31, SEQ ID NO: 32, SEQ ID NO: 34, SEQ ID NO: 35, SEQ ID NO: 36, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, or SEQ ID NO: 283.

In one embodiment, a polynucleotide encoding the first monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 45, SEQ ID NO: 46, SEQ ID NO: 47, SEQ ID NO: 52, SEQ ID NO: 53, SEQ ID NO: 55, SEQ ID NO: 56, SEQ ID NO: 57, SEQ ID NO: 66, SEQ ID NO: 67, SEQ ID NO: 68, SEQ ID NO: 73, SEQ ID NO: 74, SEQ ID NO: 76, SEQ ID NO: 77, SEQ ID NO: 78.

In one embodiment, a polypeptide of the second monomer may comprise an amino acid sequence having about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 1, SEQ ID NO: 2, SEQ ID NO: 6, SEQ ID NO: 7, SEQ ID NO: 8, SEQ ID NO: 9, SEQ ID NO: 12, SEQ ID NO: 21, SEQ ID NO: 22, SEQ ID NO: 23, SEQ ID NO: 27, SEQ ID NO: 28, SEQ ID NO: 29, SEQ ID NO: 30, SEQ ID NO: 33, SEQ ID NO: 42, SEQ ID NO: 274, SEQ ID NO: 275, SEQ ID NO: 280 or SEQ ID NO: 281.

In one embodiment, a polynucleotide encoding the second monomer may have at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to SEQ ID NO: 43, SEQ ID NO: 44, SEQ ID NO: 48, SEQ ID NO: 49, SEQ ID NO: 50, SEQ ID NO: 51, SEQ ID NO: 54, SEQ ID NO: 63, SEQ ID NO: 64, SEQ ID NO: 65, SEQ ID NO: 69, SEQ ID NO: 70, SEQ ID NO: 71, SEQ ID NO: 72, SEQ ID NO: 75, SEQ ID NO: 84.

The polynucleotide may further comprise a nucleic acid encoding a signal sequence or a leader sequence. As used herein, the term "signal sequence" refers to a signal peptide that directs the secretion of a target protein. The signal peptide is translated and then cleaved in a host cell. Specifically, the signal sequence is an amino acid sequence that initiates transportation of a protein across the endoplasmic reticulum (ER) membrane.

Signal sequences are well known in the art for their characteristics. Such signal sequences typically contain 16 to 30 amino acid residues and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during transportation of an immature polypeptide through the membrane lipid bilayer.

After initiation, signal sequences are cleaved in the lumen of ER by cellular enzymes, commonly known as signal peptidases. Then, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, a wild-type signal sequence may be used, or a signal sequence that has been substituted with a codon having high expression frequency in a host cell may be used.

### Vector loaded with polynucleotide

In another aspect of the present invention, there is provided a vector comprising the polynucleotide.

The vector may be introduced into a host cell to be recombined with and inserted into the genome of the host cell. Alternatively, the vector is understood as nucleic acid means containing a polynucleotide sequence which is autonomously replicable as an episome. The vectors comprise linear nucleic acids, plasmids, phagemids, cosmids, RNA vectors, viral vectors, and analogs thereof. Examples of the viral vector comprise, but are not limited to, retroviruses, adenoviruses, and adeno-associated viruses.

Specifically, the vector may comprise plasmid DNA, phage DNA, and the like; and commercially developed plasmids (pUC18, pBAD, pIDTSAMRT-AMP, and the like), *E. coli-*derived plasmids (pYG601BR322, pBR325, pUC118, pUC119, and the like), *Bacillus subtilis-*derived plasmids (pUB110, pTP5, and the like), yeast-derived plasmids (YEp13, YEp24, YCp50, and the like), phage DNA (Charon4A, Charon21A, EMBL3, EMBL4, λgt10, λgt11, λZAP, and the like), animal viral vectors (retroviruses, adenoviruses, vaccinia viruses, and the like), insect viral vectors (baculoviruses and the like). Since the vector exhibits different expression levels and modifications of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. Expression vectors may contain a human cytomegalovirus (CMV) promoter for promoting continuous transcription of a target gene in mammalian cells, and a bovine growth hormone polyadenylation signal sequence for increasing the stability level of RNA after transcription.

### Transformed cell expressing fusion protein

In another aspect of the present invention, there is provided a transformed cell into which the vector has been introduced.

Host cells for the transformed cell may comprise, but are not limited to, prokaryotic cells, eukaryotic cells, and cells of mammalian, plant, insect, fungal, or celluar origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK293 cells, HEK293T cells, or the like may be used. However, the mammalian cells are not limited thereto, and any cells which are known to those skilled in the art to be usable as mammalian host cells may be used.

In addition, for the introduction of an expression vector into the host cell, CaCl₂ precipitation, Hanahan method whose efficiency has been increased by using a reducing agent such as dimethyl sulfoxide (DMSO) in CaCl₂ precipitation, electroporation, calcium phosphate precipitation, protoplast fusion, agitation using silicon carbide fiber, agrobacteria-mediated transformation, transformation using PEG, dextran sulfate-, lipofectamine-, or dry/inhibitionmediated transformation, or the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those skilled in the art, glycosylation-related genes possessed by host cells.

### Method of producing a fusion protein

In another aspect of the present invention, there is provided a method of producing a fusion protein, comprising i) culturing the transformed cell; and ii) recovering a fusion protein comprising a first monomer and a second monomer.

A method of culturing the transformed cells may be carried out using methods well known in the art. Specifically, the culture may be carried out in a batch process, or carried out continuously in a fed batch or repeated fed batch process.

### Use of fusion protein

In another aspect of the present invention, there is provided a pharmaceutical composition for preventing or treating cancer, comprising the fusion protein as an active ingredient.

Then, the cancer may be any one selected from the group consisting of gastric cancer, liver cancer, lung cancer, colorectal cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer and lymphoma.

A preferred dose of the pharmaceutical composition varies depending on the patient's condition and body weight, severity of disease, form of drug, route and duration of administration and may be appropriately selected by those skilled in the art. In the pharmaceutical composition for treating or preventing tumor of the present invention, the active ingredient may be contained in any amount (effective amount) depending on application, dosage form, blending purpose, and the like, as long as the active ingredient may exhibit an activity of treating tumor or, in particular, a therapeutic effect on cancer. A conventional effective amount thereof will be determined within a range of 0.001% to 20.0% by weight, based on the total weight of the composition. Then, the term "effective amount" refers to an amount of an active ingredient capable of inducing an effect of improving or treating the condition of a disease, and in particular, inducing an effect of improving or treating the condition of cancer. Such an effective amount may be experimentally determined within the scope of common knowledge of those skilled in the art.

As used herein, the term "treatment" may be used to mean both therapeutic and prophylactic treatment. Then, prevention may be used to mean that a pathological condition or disease of a subject is alleviated or mitigated. In one embodiment, the term "treatment" comprises both application or any form of administration for treating a disease in a mammal, including a human. In addition, the term comprises inhibiting or slowing down the progression of a disease; and comprises meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Therefore, "enhanced efficacy" (for example, improvement in efficacy) may be due to enhanced pharmacokinetic parameters and enhanced efficacy, which may be measured by comparing parameters such as clearance rate and treatment or improvement of tumor in test animals or human subjects.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause side effects. A level of the effective amount may be determined depending on factors including the patient's health condition, type and severity of disease, activity of drug, the patient's sensitivity to drug, mode of administration, time of administration, route of administration and excretion rate, duration of treatment, formulation or simultaneously used drugs, and other factors well known in the medical field. In one embodiment, the therapeutically effective amount refers to an amount of drug effective to treat cancer.

Then, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffering agent, dispersing agent) may also be contained in the pharmaceutical composition.

Specifically, by including a pharmaceutically acceptable carrier in addition to the active ingredient, the pharmaceutical composition may be prepared into a parenteral formulation depending on route of administration using conventional methods known in the art. Then, the term "pharmaceutically acceptable" means that the carrier does not have more toxicity than the subject to be applied (prescribed) may adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may be preferably used. Formulation of pharmaceutical compositions is known in the art, and reference may specifically be made to Remington's Pharmaceutical Sciences (19th ed., 1995) and the like. This document is considered part of the present specification.

A preferred dose of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dose may be administered once a day or may be divided into several times a day. Such a dose should not be construed as limiting the scope of the present invention in any aspect.

Subjects to which the pharmaceutical composition may be applied (prescribed) are mammals and humans, with humans being particularly preferred. In addition to the active ingredient, the pharmaceutical composition of the present application may further contain any compound or natural extract, which is known to have a therapeutic effect on tumors.

In another aspect of the present invention, there is provided a use of a fusion protein comprising a first monomer comprising IL-12 or a variant thereof; and a second monomer comprising an antigen binding site that specifically binds to CD20, for treatment of cancer.

In another aspect of the present invention, there is provided a use of a fusion protein comprising a first monomer comprising IL-12 or a variant thereof; and a second monomer comprising an antigen binding site that specifically binds to CD20, for manufacture of a medicament for treating cancer.

In yet another aspect of the present invention, there is provided a method for treating or preventing cancer, comprising administering, to a subject, a fusion protein comprising a first monomer comprising IL-12 or a variant thereof; and a second monomer comprising an antigen binding site that specifically binds to CD20.

Then, the subject may be a subject suffering from cancer. In addition, the subject may be a mammal, preferably a human.

Route of administration, dose, and frequency of administration of the fusion protein or fusion protein dimer may vary depending on the patient's condition and the presence or absence of side effects, and thus the fusion protein or fusion protein dimer may be administered to a subject in various ways and amounts. The optimal administration method, dose, and frequency of administration may be selected in an appropriate range by those skilled in the art. In addition, the fusion protein or fusion protein dimer may be administered in combination with other drugs or physiologically active substances whose therapeutic effect is known with respect to a disease to be treated, or may be formulated in the form of combination preparations with other drugs.

Hereinafter, the present invention will be described in more detail by way of the following examples. However, the following examples are only for illustrating the present invention, and the scope of the present invention is not limited thereto.

### Preparation Example 1. Overview of anti-CD20/IL-12 IgG1 human version fusion protein

**[Table 1]**

| Code | Format | Description | Corresponding sequence |
|---|---|---|---|
| T2.01 | K&H | anti-hu CD20/hu IL-12, hu IgG1 | seq1, seq2, seq3 |
| T2.02 | K&H | anti-hu CD20/hu IL-12 mut1, hu IgG1 | seq1, seq2, seq4 |
| T2.03 | K&H | anti-hu CD20/hu IL-12 mut2, hu IgG1 | seq1, seq2, seq5 |
| T2.04 | K&H | anti-hu CD20/hu IL-12, hu IgG1 (2+1) | seq3, seq6, seq7 |
| T2.05 | K&H | anti-hu CD20/hu IL-12 mut2, hu IgG1 (2+1) | seq5, seq6, seq7 |
| T2.06 | K&H | anti-hu CD20/hu IL-12, hu IgG1 (2+1, HC scFv) | seq2, seq3, seq8 |
| T2.07 | K&H | anti-hu CD20/hu IL-12 mut2, hu IgG1 (2+1, HC scFv) | seq2, seq5, seq8 |
| T2.08 | K&H | anti-hu CD20/hu IL-12, hu IgG1 (2+1, LC scFv) | seq1, seq3, seq9 |
| T2.09 | K&H | anti-hu CD20/hu IL-12 mut2, hu IgG1 (2+1, LC scFv) | seq1, seq5, seq9 |
| T2.10 | K&H | anti-hu CD20/anti-hu CD20-hu IL-12, hu IgG1 (2+1) | seq1, seq2, seq10 |
| T2.11 | K&H | anti-hu CD20/anti-hu CD20-hu IL-12 mut2, hu IgG1 (2+1) | seq1, seq2, seq11 |
| T2.12 | K&H | anti-hu CD20/hu IL-12 mut1, hu IgG1 DANG | seq2, seq12, seq14 |
| T2.13 | K&H | null/hu IL-12, hu IgG1 DANG | seq13, seq19 |
| T2.14 | K&H | null/hu IL-12 mut1, hu IgG1 DANG | seq14, seq19 |
| T2.15 | Fc-fusion | hu IL-12 mut2, huIgG1 | seq 15 |
| T2.16 | K&H | anti-hu FAP/hu IL-12 mut1, hu IgG1 | seq4, seq16, seq17 |
| T2.17 | K&H | anti-hu FAP/null, hu IgG1 DANG | seq17, seq18, seq20 |
| Rituxim ab | Mab | anti-hu CD20 (Rituximab) | seq2, seq21 |
| T2.18 | K&H | anti-hu CD20/hu IL-12-anti-hu CD20 scFv, hu IgG1 (2+1) | seq1, seq2, seq272 |
| T2.19 | K&H | anti-hu CD20/hu IL-12 mut2-anti-hu CD20 scFv, hu IgG1 (2+1) | seq1, seq2, seq273 |
| T2.20 | Fc-fusion | anti-hu CD20-hu IL-12, hu IgG1 | seq2, seq274 |
| T2.21 | Fc-fusion | anti-hu CD20-hu IL-12 mut2, hu IgG1 | seq2, seq275 |
| T2.22 | Fc-fusion | hu IL-12-anti-hu CD20 scFv, hu IgG1 | Seq276 |
| T2.23 | Fc-fusion | hu IL-12 mut2-anti-hu CD20 scFv, hu IgG1 | Seq277 |

[seq1] is composed of a human anti-CD20 heavy chain variable region sequence and a human IgG1 Fc in which a knob structure is formed by a T366W mutation.

[seq2] is composed of a human anti-CD20 light chain sequence.

[seq3] is composed of a human IL-12 p40 (beta) region sequence, and a linker GGGGSGGGGSGGGGS, a human interleukin 12 p35 (alpha) region sequence, a linker GGGGSGGGGS, and a human IgG1 Fc in which a hole structure is formed by T366S, L368A and Y407V mutations.

[seq4] is composed of a sequence in which lysine (K) at the 280^{th} and 285^{th} amino acids involved in heparin binding in a human IL-12 p40 (beta) region is mutated to alanine (A), a linker GGGGSGGGGSGGGGS, a human IL-12 p35 (alpha) region sequence, a linker GGGGSGGGGS, and a human IgG1 Fc hole.

[seq5] is composed of a sequence in which lysine (K) at the 280^{th}, 282^{nd}, 285^{th} and 286^{th} amino acids involved in heparin binding in a human IL-12 p40 (beta) region is mutated to alanine (A), a linker GGGGSGGGGSGGGGS, a human IL-12 p35 (alpha) region sequence, a linker GGGGSGGGGS, and a human IgG1 Fc hole.

[seq6] is composed of a human anti-CD20 heavy chain variable region sequence, a linker GGGGSGGGGSGGGGS, a human anti-CD20 heavy chain variable region sequence, and a human IgG1 Fc knob.

[seq7] is composed of a human anti-CD20 light chain variable region sequence, a linker GGGGSGGGGSGGGGS, and a human anti-CD20 light chain sequence.

[seq8] is composed of a human anti-CD20 heavy chain variable region sequence, a human IgG1 Fc knob, a linker GGGGSGGGGSGGGGS, a human anti-CD20 heavy chain variable region sequence, a linker GGGGSGGGGSGGGGSGGGGS, and a human anti-CD20 light chain variable region sequence.

[seq9] is composed of a human anti-CD20 light chain sequence, a linker GGGGSGGGGSGGGGS, a human anti-CD20 heavy chain variable region sequence, a linker GGGGSGGGGSGGGGSGGGGS, and a human anti-CD20 light chain variable region sequence.

[seq10] is composed of a human anti-CD20 heavy chain sequence, a human IgG1 Fc hole, a linker GGGGSGGGGSGGGGS, a human IL-12 p40 (beta) region sequence, a linker GGGGSGGGGSGGGGS, and a human IL-12 p35 (alpha) region sequence.

[seq11] is composed of a human anti-CD20 heavy chain sequence, a human IgG1 Fc hole, a linker GGGGSGGGGSGGGGS, a human IL-12 p40 (beta) region sequence containing mutations (four) of amino acids involved in heparin binding, a linker GGGGSGGGGSGGGGS, and a human IL-12 p35 (alpha) region sequence.

[seq12] is composed of a heavy chain sequence of a human anti-CD20 antibody and a human IgG1 Fc knob DANG.

[seq13] is composed of a human IL-12 p40 (beta) region sequence, a linker GGGGSGGGGSGGGGS, a human IL-12 p35 (alpha) region sequence, a linker GGGGSGGGGS, and a human IgG1 Fc hole DANG.

[seq14] is composed of a human IL-12 p40 (beta) region sequence containing mutations (two) of amino acids involved in heparin binding, a linker GGGGSGGGGSGGGGS, a human IL-12 p35 (alpha) region sequence, a linker GGGGSGGGGS, and a human IgG1 Fc hole DANG.

[seq15] is composed of a human IL-12 p40 (beta) region sequence containing mutations (four) of amino acids involved in heparin binding, a linker GGGGSGGGGSGGGGS, a human IL-12 p35 (alpha) region sequence, and a linker GGGGSGGGGS, and a human IgG1 Fc.

[seq16] is composed of a human anti-FAP heavy chain variable region sequence and a human IgG1 Fc knob.

[seq17] is composed of a human anti-FAP light chain sequence.

[seq 18] is composed of a human anti-FAP heavy chain variable region sequence and a human IgG1 Fc knob DANG.

[seq19] is composed of only a human IgG1 Fc knob DANG.

[seq20] is composed of only a human IgG1 Fc hole DANG.

[seq21] is a heavy chain sequence of the human anti-CD20 antibody Rituximab.

[seq272] is composed of a human IL-12 p40 (beta) region sequence, a linker GGGGSGGGGSGGGGS, a human IL-12 p35 (alpha) region sequence, a linker GGGGSGGGGS and a human IgG1 Fc hole, a linker GGGGSGGGGSGGGGS, a human anti-CD20 heavy chain variable region sequence, a linker GGGGSGGGGSGGGGSGGGGS, and a human anti-CD20 light chain variable region sequence.

[seq273] is composed of a human IL-12 p40 (beta) region sequence containing mutations (four) of amino acids involved in heparin binding, a linker GGGGSGGGGSGGGGS, a human IL-12 p35 (alpha) region sequence, a linker GGGGSGGGGS and a human IgG1 Fc hole, a linker GGGGSGGGGSGGGGS, a human anti-CD20 heavy chain variable region sequence, a linker GGGGSGGGGSGGGGSGGGGS, and a human anti-CD20 light chain variable region sequence.

[seq274] is composed of a human anti-CD20 heavy chain sequence, a human IgG1 Fc, a linker GGGGSGGGGSGGGGS, a human IL-12 p40 (beta) region sequence, a linker GGGGSGGGGSGGGGS, and a human IL-12 p35 (alpha) region sequence.

[seq275] is composed of a human anti-CD20 heavy chain sequence, a human IgG1 Fc, a linker GGGGSGGGGSGGGGS, a human IL-12 p40 (beta) region sequence containing mutations (four) of amino acids involved in heparin binding, a linker GGGGSGGGGSGGGGS, and a human IL-12 p35 (alpha) region sequence.

[seq276] is composed of a human IL-12 p40 (beta) region sequence, a linker GGGGSGGGGSGGGGS, a human IL-12 p35 (alpha) region sequence, a linker GGGGSGGGGS and a human IgG1 Fc, a linker GGGGSGGGGSGGGGS, a human anti-CD20 heavy chain variable region sequence, a linker GGGGSGGGGSGGGGSGGGGS, and a human anti-CD20 light chain variable region sequence.

[seq277] is composed of a human IL-12 p40 (beta) region sequence containing mutations (four) of amino acids involved in heparin binding, a linker GGGGSGGGGSGGGGS, a human IL-12 p35 (alpha) region sequence, a linker GGGGSGGGGS and a human IgG1 Fc, a linker GGGGSGGGGSGGGGS, a human anti-CD20 heavy chain variable region sequence, a linker GGGGSGGGGSGGGGSGGGGS, and a human anti-CD20 light chain variable region sequence.

T2.01 (seq1, seq2, seq3) is a bispecific antibody in which a human anti-CD20 sequence targeting protein CD20 and a human IL-12 sequence form a knob-into-hole structure.

T2.02 (seq1, seq2, seq4) is a bispecific antibody in which a human anti-CD20 sequence targeting protein CD20 and a human IL-12 sequence containing mutations (two) of amino acids involved in heparin binding form a knob-into-hole structure.

T2.03 (seq1, seq2, seq5) is a bispecific antibody in which a human anti-CD20 sequence targeting protein CD20 and a human IL-12 sequence containing mutations (four) of amino acids involved in heparin binding form a knob-into-hole structure.

T2.04 (seq3, seq6, seq7) is a bispecific antibody in which a human anti-CD20 sequence in a dual variable domain immunoglobulin (DVD-Ig) form and a human IL-12 sequence form a knob-into-hole structure.

T2.05 (seq5, seq6, seq7) is a bispecific antibody in which a human anti-CD20 sequence in a dual variable domain immunoglobulin (DVD-Ig) form and a human IL-12 sequence containing mutations (four) of amino acids involved in heparin binding form a knob-into-hole structure.

T2.06 (seq2, seq3, seq8) is a bispecific antibody in which a human anti-CD20 sequence in which a single chain variable fragment (scFv) of a human anti-CD20 sequence targeting protein CD20 is linked to the C-terminus of the heavy chain and a human IL-12 sequence form a knob-into-hole structure.

T2.07 (seq2, seq5, seq8) is a bispecific antibody in which a human anti-CD20 sequence in which a single chain variable fragment (scFv) of a human anti-CD20 sequence targeting protein CD20 is linked to the C-terminus of the heavy chain and a human IL-12 sequence containing mutations (four) of amino acids involved in heparin binding form a knob-into-hole structure.

T2.08 (seq1, seq3, seq9) is a bispecific antibody in which a human anti-CD20 sequence in which a single chain variable fragment (scFv) of a human anti-CD20 sequence targeting protein CD20 is linked to the C-terminus of the light chain and a human IL-12 sequence form a knob-into-hole structure.

T2.09 (seq1, seq5, seq9) is a bispecific antibody in which a human anti-CD20 sequence in which a single chain variable fragment (scFv) of a human anti-CD20 sequence targeting protein CD20 is linked to the C-terminus of the light chain and a human IL-12 sequence containing mutations (four) of amino acids involved in heparin binding form a knob-into-hole structure.

T2.10 (seq1, seq2, seq10) is an antibody in which a human anti-CD20 sequence targeting protein CD20 and a human anti-CD20 sequence in which a human IL-12 sequence is linked to the C-terminus of the heavy chain form a knob-into-hole structure.

T2.11 (seq1, seq2, seq11) is an antibody in which a human anti-CD20 sequence targeting protein CD20 and a human anti-CD20 sequence in which a human IL-12 sequence containing mutations (four) of amino acids involved in heparin binding is linked to the C-terminus of the heavy chain form a knob-into-hole structure.

T2.12 (seq2, seq12, seq14) is a bispecific antibody in which a human anti-CD20 sequence targeting protein CD20 and a human IL-12 sequence containing mutations (two) of amino acids involved in heparin binding form a knob-into-hole structure, and an effector function is removed.

T2.13 (seq13, seq19) is an antibody in which an effector function of a knob-into-hole structure having only a human IL-12 sequence is removed.

T2.14 (seq14, seq19) is an antibody in which an effector function of a knob-into-hole structure having only a human IL-12 sequence containing mutations (two) of amino acids involved in heparin binding is removed.

T2.15 (seq15) is an Fc-fusion protein having only a human IL-12 sequence containing mutations (four) of amino acids involved in heparin binding.

T2.16 (seq4, seq16, seq17) is a bispecific antibody in which a human anti-FAP sequence targeting protein FAP and a human IL-12 sequence containing mutations (two) of amino acids involved in heparin binding form a knob-into-hole structure.

T2.17 (seq17, seq18, seq20) is an antibody in which an effector function of a knob-into-hole structure having only a human anti-FAP sequence targeting protein FAP is removed. Rituximab (seq2, seq21) is a human anti-CD20 (Rituximab) antibody targeting protein CD20.

T2.18 (seq1, seq2, seq272) is a bispecific antibody in which a human anti-CD20 sequence and a human IL-12 sequence in which a single chain variable fragment (scFv) of a human anti-CD20 sequence is linked to the C-terminus form a knob-into-hole structure.

T2.19 (seq1, seq2, seq273) is a bispecific antibody in which a human anti-CD20 heavy chain sequence and a human IL-12 sequence containing mutations (four) of amino acids involved in heparin binding in which a single chain variable fragment (scFv) of a human anti-CD20 sequence is linked to the C-terminus form a knob-into-hole structure.

T2.20 (seq2, seq274) is an Fc-fusion protein dimer containing a human anti-CD20 sequence in which a human IL-12 sequence is linked to the C-terminus of the heavy chain.

T2.21 (seq2, seq275) is an Fc-fusion protein dimer containing a human anti-CD20 sequence in which a human IL-12 sequence containing mutations (four) of amino acids involved in heparin binding is linked to the C-terminus of the heavy chain.

T2.22 (seq276) is an Fc-fusion protein dimer containing a human IL-12 sequence in which a single chain variable fragment (scFv) of a human anti-CD20 sequence is linked to the C-terminus.

T2.23 (seq277) is an Fc-fusion protein dimer composed of a human IL-12 sequence containing mutations (four) of amino acids involved in heparin binding in which a single chain variable fragment (scFv) of a human anti-CD20 sequence is linked to the C-terminus.
[seq1] anti-hu CD20 HC hu IgG1 Fc knob 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLWCLVKGFYPSDIAVEWESNGQPEN
NYKTTPPVLDSDGSFFLYSKLTVDKSRWQQGNVFSCSVMHEALHNHYTQKSLSLSPG
K
[seq2] anti-hu CD20 LC 
QIVLSQSPAILSASPGEKVTMTCRASSSVSYIHWFQQKPGSSPKPWIYATSNLA
SGVPVRFSGSGSGTSYSLTISRVEAEDAATYYCQQWTSNPPTFGGGTKLEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC
[seq3] hu scIL-12-hu IgG1 Fc hole 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGKSKREKKDRVFTDKTSATVICRKN
ASISVRAQDRYYSSSWSEWASVPCSGGGGSGGGGSGGGGSRNLPVATPDPGMFPCLH
HSQNLLRAVSNMLQKARQTLEFYPCTSEEIDHEDITKDKTSTVEACLPLELTKNESCL
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHEA
LHNHYTQKSLSLSPGK
[seq4] hu scIL-12 mut1-hu IgG1 Fc hole 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGASKREAKDRVFTDKTSATVICRKN
ASISVRAQDRYYSSSWSEWASVPCSGGGGSGGGGSGGGGSRNLPVATPDPGMFPCLH
 
HSQNLLRAVSNMLQKARQTLEFYPCTSEEIDHEDITKDKTSTVEACLPLELTKNESCL
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHEA
LHNHYTQKSLSLSPGK
[seq5] hu scIL-12 mut2-hu IgG1 Fc hole 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGASAREAADRVFTDKTSATVICRKN
ASISVRAQDRYYSSSWSEWASVPCSGGGGSGGGGSGGGGSRNLPVATPDPGMFPCLH
HSQNLLRAVSNMLQKARQTLEFYPCTSEEIDHEDITKDKTSTVEACLPLELTKNESCL
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLSCAVKGFYP
SDIAVEWESNGQPENNYKTTPPVLDSDGSFFLVSKLTVDKSRWQQGNVFSCSVMHEA
LHNHYTQKSLSLSPGK
[seq6] (anti-hu CD20 VH)2-hu IgG1 Fc knob 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAGGGGSGGGGSGGGGSQVQLQQPGAELVKPGASVKMSCKAS
GYTFTSYNMHWVKQTPGRGLEWIGAIYPGNGDTSYNQKFKGKATLTADKSSSTAYM
QLSSLTSEDSAVYYCARSTYYGGDWYFNVWGAGTTVTVSAASTKGPSVFPLAPSSKS
TSGGTAALGCLVKDYFPEPVTVSWNSGALTSGVHTFPAVLQSSGLYSLSSVVTVPSSS
LGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSRDELTKNQV
SLWCLVKGFYPSDIAVEWESNGQPENNYKTTPPVLDSDGSFFLYSKLTVDKSRWQQG
NVFSCSVMHEALHNHYTQKSLSLSPGK
[seq7] (anti-hu CD20 VL)2 
QIVLSQSPAILSASPGEKVTMTCRASSSVSYIHWFQQKPGSSPKPWIYATSNLA
SGVPVRFSGSGSGTSYSLTISRVEAEDAATYYCQQWTSNPPTFGGGTKLEIKGGGGSG
 
GGGSGGGGSQIVLSQSPAILSASPGEKVTMTCRASSSVSYIHWFQQKPGSSPKPWIYAT
SNLASGVPVRFSGSGSGTSYSLTISRVEAEDAATYYCQQWTSNPPTFGGGTKLEIKRT
VAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
[seq8] anti-hu CD20 HC hu IgG1 Fc knob-anti-hu CD20 scFv 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLWCLVKGFYPSDIAVEWESNGQPEN
NYKTTPPVLDSDGSFFLYSKLTVDKSRWQQGNVFSCSVMHEALHNHYTQKSLSLSPG
KGGGGSGGGGSGGGGSQVQLQQPGAELVKPGASVKMSCKASGYTFTSYNMHWVK
QTPGRGLEWIGAIYPGNGDTSYNQKFKGKATLTADKSSSTAYMQLSSLTSEDSAVYYC
ARSTYYGGDWYFNVWGAGTTVTVSAGGGGSGGGGSGGGGSGGGGSQIVLSQSPAI
LSASPGEKVTMTCRASSSVSYIHWFQQKPGSSPKPWIYATSNLASGVPVRFSGSGSGT
SYSLTISRVEAEDAATYYCQQWTSNPPTFGGGTKLEIK
[seq9] anti-hu CD20 LC-anti-hu CD20 scFv 
QIVLSQSPAILSASPGEKVTMTCRASSSVSYIHWFQQKPGSSPKPWIYATSNLA
SGVPVRFSGSGSGTSYSLTISRVEAEDAATYYCQQWTSNPPTFGGGTKLEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGECGGGGSGGGGSGGG
GSQVQLQQPGAELVKPGASVKMSCKASGYTFTSYNMHWVKQTPGRGLEWIGAIYP
GNGDTSYNQKFKGKATLTADKSSSTAYMQLSSLTSEDSAVYYCARSTYYGGDWYFN
VWGAGTTVTVSAGGGGSGGGGSGGGGSGGGGSQIVLSQSPAILSASPGEKVTMTCR
ASSSVSYIHWFQQKPGSSPKPWIYATSNLASGVPVRFSGSGSGTSYSLTISRVEAEDAA
TYYCQQWTSNPPTFGGGTKLEIK
[seq10] anti-hu CD20 HC hu IgG1 Fc hole-hu scIL-12 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLSCAVKGFYPSDIAVEWESNGQPEN
NYKTTPPVLDSDGSFFLVSKLTVDKSRWQQGNVFSCSVMHEALHNHYTQKSLSLSPG
KGGGGSGGGGSGGGGSIWELKKDVYVVELDWYPDAPGEMVVLTCDTPEEDGITWT
 
LDQSSEVLGSGKTLTIQVKEFGDAGQYTCHKGGEVLSHSLLLLHKKEDGIWSTDILK
DQKEPKNKTFLRCEAKNYSGRFTCWWLTTISTDLTFSVKSSRGSSDPQGVTCGAATLS
AERVRGDNKEYEYSVECQEDSACPAAEESLPIEVMVDAVHKLKYENYTSSFFIRDIIK
PDPPKNLQLKPLKNSRQVEVSWEYPDTWSTPHSYFSLTFCVQVQGKSKREKKDRVFT
DKTSATVICRKNASISVRAQDRYYSSSWSEWASVPCSGGGGSGGGGSGGGGSRNLPV
ATPDPGMFPCLHHSQNLLRAVSNMLQKARQTLEFYPCTSEEIDHEDITKDKTSTVEAC
LPLELTKNESCLNSRETSFITNGSCLASRKTSFMMALCLSSIYEDLKMYQVEFKTMNA
KLLMDPKRQIFLDQNMLAVIDELMQALNFNSETVPQKSSLEEPDFYKTKIKLCILLHA
FRIRAVTIDRVMSYLNAS
[seq11] anti-hu CD20 HC hu IgG1 Fc hole-hu scIL-12 mut2 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLSCAVKGFYPSDIAVEWESNGQPEN
NYKTTPPVLDSDGSFFLVSKLTVDKSRWQQGNVFSCSVMHEALHNHYTQKSLSLSPG
KGGGGSGGGGSGGGGSIWELKKDVYVVELDWYPDAPGEMVVLTCDTPEEDGITWT
LDQSSEVLGSGKTLTIQVKEFGDAGQYTCHKGGEVLSHSLLLLHKKEDGIWSTDILK
DQKEPKNKTFLRCEAKNYSGRFTCWWLTTISTDLTFSVKSSRGSSDPQGVTCGAATLS
AERVRGDNKEYEYSVECQEDSACPAAEESLPIEVMVDAVHKLKYENYTSSFFIRDIIK
PDPPKNLQLKPLKNSRQVEVSWEYPDTWSTPHSYFSLTFCVQVQGASAREAADRVFT
DKTSATVICRKNASISVRAQDRYYSSSWSEWASVPCSGGGGSGGGGSGGGGSRNLPV
ATPDPGMFPCLHHSQNLLRAVSNMLQKARQTLEFYPCTSEEIDHEDITKDKTSTVEAC
LPLELTKNESCLNSRETSFITNGSCLASRKTSFMMALCLSSIYEDLKMYQVEFKTMNA
KLLMDPKRQIFLDQNMLAVIDELMQALNFNSETVPQKSSLEEPDFYKTKIKLCILLHA
FRIRAVTIDRVMSYLNAS
[seq12] anti-hu CD20 HC hu IgG1 Fc knob DANG 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVAVSHEDPEVKFN
WYVDGVEVHNAKTKPREEQYGSTYRVVSVLTVLHQDWLNGKEYKCKVSNKALPAP
IEKTISKAKGQPREPQVYTLPPSRDELTKNQVSLWCLVKGFYPSDIAVEWESNGQPEN
NYKTTPPVLDSDGSFFLYSKLTVDKSRWQQGNVFSCSVMHEALHNHYTQKSLSLSPG
K
[seq13] hu scIL-12-hu IgG1 Fc hole DANG 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGKSKREKKDRVFTDKTSATVICRKN
ASISVRAQDRYYSSSWSEWASVPCSGGGGSGGGGSGGGGSRNLPVATPDPGMFPCLH
HSQNLLRAVSNMLQKARQTLEFYPCTSEEIDHEDITKDKTSTVEACLPLELTKNESCL
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVAVSHEDPEVKFNWYVDGVEVHNAKTKPREEQYGSTYRVVSVLTVLHQDWLNGK
EYKCKVSNKALPAPIEKTISKAKGQPREPQVYTLPPSRDELTKNQVSLSCAVKGFYPS
DIAVEWESNGQPENNYKTTPPVLDSDGSFFLVSKLTVDKSRWQQGNVFSCSVMHEAL
HNHYTQKSLSLSPGK
[seq14] hu scIL-12 mut1-hu IgG1 Fc hole DANG 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGASKREAKDRVFTDKTSATVICRKN
ASISVRAQDRYYSSSWSEWASVPCSGGGGSGGGGSGGGGSRNLPVATPDPGMFPCLH
HSQNLLRAVSNMLQKARQTLEFYPCTSEEIDHEDITKDKTSTVEACLPLELTKNESCL
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVAVSHEDPEVKFNWYVDGVEVHNAKTKPREEQYGSTYRVVSVLTVLHQDWLNGK
EYKCKVSNKALPAPIEKTISKAKGQPREPQVYTLPPSRDELTKNQVSLSCAVKGFYPS
DIAVEWESNGQPENNYKTTPPVLDSDGSFFLVSKLTVDKSRWQQGNVFSCSVMHEAL
HNHYTQKSLSLSPGK
[seq15] hu scIL-12 mut2-hu IgG1 Fc 
IWELKKDVYVVELDWYPDAPGEMVVLTCDTPEEDGITWTLDQSSEVLGSGK
TLTIQVKEFGDAGQYTCHKGGEVLSHSLLLLHKKEDGIWSTDILKDQKEPKNKTFLR
CEAKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLKPL
KNSRQVEVSWEYPDTWSTPHSYFSLTFCVQVQGASAREAADRVFTDKTSATVICRKN
ASISVRAQDRYYSSSWSEWASVPCSGGGGSGGGGSGGGGSRNLPVATPDPGMFPCLH
HSQNLLRAVSNMLQKARQTLEFYPCTSEEIDHEDITKDKTSTVEACLPLELTKNESCL
 
NSRETSFITNGSCLASRKTSFMMALCLSSIYEDLKMYQVEFKTMNAKLLMDPKRQIF
LDQNMLAVIDELMQALNFNSETVPQKSSLEEPDFYKTKIKLCILLHAFRIRAVTIDRVM
SYLNASGGGGSGGGGSDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEA
LHNHYTQKSLSLSPGK
[seq16] anti-hu FAP HC IgG1 Fc knob 
QVQLVQSGAEVKKPGASVKVSCKTSRYTFTEYTIHWVRQAPGQRLEWIGGI
NPNNGIPNYNQKFKGRVTITVDTSASTAYMELSSLRSEDTAVYYCARRRIAYGYDEGH
AMDYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALP
APIEKTISKAKGQPREPQVYTLPPSRDELTKNQVSLWCLVKGFYPSDIAVEWESNGQP
ENNYKTTPPVLDSDGSFFLYSKLTVDKSRWQQGNVFSCSVMHEALHNHYTQKSLSLS
PGK
[seq17] anti-hu FAP LC 
DIVMTQSPDSLAVSLGERATINCKSSQSLLYSRNQKNYLAWYQQKPGQPPKL
LIFWASTRESGVPDRFSGSGFGTDFTLTISSLQAEDVAVYYCQQYFSYPLTFGQGTKVE
IKRTVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
[seq18] anti-hu FAP HC hu IgG1 Fc knob DANG 
QVQLVQSGAEVKKPGASVKVSCKTSRYTFTEYTIHWVRQAPGQRLEWIGGI
NPNNGIPNYNQKFKGRVTITVDTSASTAYMELSSLRSEDTAVYYCARRRIAYGYDEGH
AMDYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVAVSHEDPEVKFN
WYVDGVEVHNAKTKPREEQYGSTYRVVSVLTVLHQDWLNGKEYKCKVSNKALPAP
IEKTISKAKGQPREPQVYTLPPSRDELTKNQVSLWCLVKGFYPSDIAVEWESNGQPEN
NYKTTPPVLDSDGSFFLYSKLTVDKSRWQQGNVFSCSVMHEALHNHYTQKSLSLSPG
K
[seq19] hu IgG1 Fc knob DANG 
DKTHTCPPCPAPELLGGPSVFLFPPKPKDTLMISRTPEVTCVVVAVSHEDPEVK
FNWYVDGVEVHNAKTKPREEQYGSTYRVVSVLTVLHQDWLNGKEYKCKVSNKALP
APIEKTISKAKGQPREPQVYTLPPSRDELTKNQVSLWCLVKGFYPSDIAVEWESNGQP
ENNYKTTPPVLDSDGSFFLYSKLTVDKSRWQQGNVFSCSVMHEALHNHYTQKSLSLS
PGK
[seq20] hu IgG1 Fc hole DANG 
DKTHTCPPCPAPELLGGPSVFLFPPKPKDTLMISRTPEVTCVVVAVSHEDPEVK
FNWYVDGVEVHNAKTKPREEQYGSTYRVVSVLTVLHQDWLNGKEYKCKVSNKALP
APIEKTISKAKGQPREPQVYTLPPSRDELTKNQVSLSCAVKGFYPSDIAVEWESNGQPE
NNYKTTPPVLDSDGSFFLVSKLTVDKSRWQQGNVFSCSVMHEALHNHYTQKSLSLSP
GK
[seq21] anti-hu CD20 (Rituximab) HC 
QVQLQQPGAELVKPGASVKMSCKASGYTFTSYNMHWVKQTPGRGLEWIGA
IYPGNGDTSYNQKFKGKATLTADKSSSTAYMQLSSLTSEDSAVYYCARSTYYGGDWY
FNVWGAGTTVTVSAASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPK
SCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK
[seq272] hu scIL-12-hu IgG1 Fc hole-anti-hu CD20 scFv 
IWELKKDVYVVELDWYPDAPGEMVVLTCDTPEEDGITWTLDQSSEVLGSGKTLTIQVKEFGDAGQYTC
HKGGEVLSHSLLLLHKKEDGIWSTDILKDQKEPKNKTFLRCEAKNYSGRFTCWWLTTISTDLTFSVKSSRGSSDP
QGVTCGAATLSAERVRGDNKEYEYSVECQEDSACPAAEESLPIEVMVDAVHKLKYENYTSSFFIRDIIKPDPPKN
LQLKPLKNSRQVEVSWEYPDTWSTPHSYFSLTFCVQVQGKSKREKKDRVFTDKTSATVICRKNASISVRAQDRYY
SSSWSEWASVPCSGGGGSGGGGSGGGGSRNLPVATPDPGMFPCLHHSQNLLRAVSNMLQKARQTLEFYPCTSEEI
DHEDITKDKTSTVEACLPLELTKNESCLNSRETSFITNGSCLASRKTSFMMALCLSSIYEDLKMYQVEFKTMNAK
LLMDPKRQIFLDQNMLAVIDELMQALNFNSETVPQKSSLEEPDFYKTKIKLCILLHAFRIRAVTIDRVMSYLNAS
GGGGSGGGGSDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPSRDELTK
NQVSLSCAVKGFYPSDIAVEWESNGQPENNYKTTPPVLDSDGSFFLVSKLTVDKSRWQQGNVFSCSVMHEALHNH
YTQKSLSLSPGGGGGSGGGGSGGGGSQVQLQQPGAELVKPGASVKMSCKASGYTFTSYNMHWVKQTPGRGLEWIG
AIYPGNGDTSYNQKFKGKATLTADKSSSTAYMQLSSLTSEDSAVYYCARSTYYGGDWYFNVWGAGTTVTVSAGGG
GSGGGGSGGGGSGGGGSQIVLSQSPAILSASPGEKVTMTCRASSSVSYIHWFQQKPGSSPKPWIYATSNLASGVP
VRFSGSGSGTSYSLTISRVEAEDAATYYCQQWTSNPPTFGGGTKLEIK
[seq274] anti-hu CD20 HC hu IgG1 Fc-hu scIL-12 
QVQLQQPGAELVKPGASVKMSCKASGYTFTSYNMHWVKQTPGRGLEWIGAIYPGNGDTSYNQKFKGKA
TLTADKSSSTAYMQLSSLTSEDSAVYYCARSTYYGGDWYFNVWGAGTTVTVSAASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYTQK
SLSLSPGGGGGSGGGGSGGGGSIWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAKNYSGRFTCWWLTTIST
DLTFSVKSSRGSSDPQGVTCGAATLSAERVRGDNKEYEYSVECQEDSACPAAEESLPIEVMVDAVHKLKYENYTS
SFFIRDIIKPDPPKNLQLKPLKNSRQVEVSWEYPDTWSTPHSYFSLTFCVQVQGKSKREKKDRVFTDKTSATVIC
RKNASISVRAQDRYYSSSWSEWASVPCSGGGGSGGGGSGGGGSRNLPVATPDPGMFPCLHHSQNLLRAVSNMLQK
ARQTLEFYPCTSEEIDHEDITKDKTSTVEACLPLELTKNESCLNSRETSFITNGSCLASRKTSFMMALCLSSIYE
DLKMYQVEFKTMNAKLLMDPKRQIFLDQNMLAVIDELMQALNFNSETVPQKSSLEEPDFYKTKIKLCILLHAFRI
RAVTIDRVMSYLNAS
[seq275] anti-hu CD20 HC hu IgG1 Fc-hu scIL-12 mut2 
QVQLQQPGAELVKPGASVKMSCKASGYTFTSYNMHWVKQTPGRGLEWIGAIYPGNGDTSYNQKFKGKA
TLTADKSSSTAYMQLSSLTSEDSAVYYCARSTYYGGDWYFNVWGAGTTVTVSAASTKGPSVFPLAPSSKSTSGGT
AALGCLVKDYFPEPVTVSWNSGALTSGVHTFPAVLQSSGLYSLSSVVTVPSSSLGTQTYICNVNHKPSNTKVDKK
VEPKSCDKTHTCPPCPAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGNVFSCSVMHEALHNHYTQK
SLSLSPGGGGGSGGGGSGGGGSIWELKKDVYVVELDWYPDAPGEMVVLTCDTPEEDGITWTLDQSSEVLGSGKTL
TIQVKEFGDAGQYTCHKGGEVLSHSLLLLHKKEDGIWSTDILKDQKEPKNKTFLRCEAKNYSGRFTCWWLTTIST
DLTFSVKSSRGSSDPQGVTCGAATLSAERVRGDNKEYEYSVECQEDSACPAAEESLPIEVMVDAVHKLKYENYTS
SFFIRDIIKPDPPKNLQLKPLKNSRQVEVSWEYPDTWSTPHSYFSLTFCVQVQGASAREAADRVFTDKTSATVIC
RKNASISVRAQDRYYSSSWSEWASVPCSGGGGSGGGGSGGGGSRNLPVATPDPGMFPCLHHSQNLLRAVSNMLQK
ARQTLEFYPCTSEEIDHEDITKDKTSTVEACLPLELTKNESCLNSRETSFITNGSCLASRKTSFMMALCLSSIYE
DLKMYQVEFKTMNAKLLMDPKRQIFLDQNMLAVIDELMQALNFNSETVPQKSSLEEPDFYKTKIKLCILLHAFRI
RAVTIDRVMSYLNAS
[seq276] hu scIL-12-hu IgG1 Fc-anti-hu CD20 scFv 
IWELKKDVYVVELDWYPDAPGEMVVLTCDTPEEDGITWTLDQSSEVLGSGKTLTIQVKEFGDAGQYTC
HKGGEVLSHSLLLLHKKEDGIWSTDILKDQKEPKNKTFLRCEAKNYSGRFTCWWLTTISTDLTFSVKSSRGSSDP
QGVTCGAATLSAERVRGDNKEYEYSVECQEDSACPAAEESLPIEVMVDAVHKLKYENYTSSFFIRDIIKPDPPKN
LQLKPLKNSRQVEVSWEYPDTWSTPHSYFSLTFCVQVQGKSKREKKDRVFTDKTSATVICRKNASISVRAQDRYY
SSSWSEWASVPCSGGGGSGGGGSGGGGSRNLPVATPDPGMFPCLHHSQNLLRAVSNMLQKARQTLEFYPCTSEEI
DHEDITKDKTSTVEACLPLELTKNESCLNSRETSFITNGSCLASRKTSFMMALCLSSIYEDLKMYQVEFKTMNAK
LLMDPKRQIFLDQNMLAVIDELMQALNFNSETVPQKSSLEEPDFYKTKIKLCILLHAFRIRAVTIDRVMSYLNAS
GGGGSGGGGSDKTHTCPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLTVDKSRWQQGNVFSCSVMHEALHNH
YTQKSLSLSPGGGGGSGGGGSGGGGSQVQLQQPGAELVKPGASVKMSCKASGYTFTSYNMHWVKQTPGRGLEWIG
AIYPGNGDTSYNQKFKGKATLTADKSSSTAYMQLSSLTSEDSAVYYCARSTYYGGDWYFNVWGAGTTVTVSAGGG
GSGGGGSGGGGSGGGGSQIVLSQSPAILSASPGEKVTMTCRASSSVSYIHWFQQKPGSSPKPWIYATSNLASGVP
VRFSGSGSGTSYSLTISRVEAEDAATYYCQQWTSNPPTFGGGTKLEIK
[seq277] hu scIL-12 mut2-hu IgG1 Fc-anti-hu CD20 scFv

IWELKKDVYVVELDWYPDAPGEMVVLTCDTPEEDGITWTLDQSSEVLGSGKTLTIQVKEFGDAGQYTCHKGGEVL
SHSLLLLHKKEDGIWSTDILKDQKEPKNKTFLRCEAKNYSGRFTCWWLTTISTDLTFSVKSSRGSSDPQGVTCGA
ATLSAERVRGDNKEYEYSVECQEDSACPAAEESLPIEVMVDAVHKLKYENYTSSFFIRDIIKPDPPKNLQLKPLK
NSRQVEVSWEYPDTWSTPHSYFSLTFCVQVQGASAREAADRVFTDKTSATVICRKNASISVRAQDRYYSSSWSEW
ASVPCSGGGGSGGGGSGGGGSRNLPVATPDPGMFPCLHHSQNLLRAVSNMLQKARQTLEFYPCTSEEIDHEDITK
DKTSTVEACLPLELTKNESCLNSRETSFITNGSCLASRKTSFMMALCLSSIYEDLKMYQVEFKTMNAKLLMDPKR
QIFLDQNMLAVIDELMQALNFNSETVPQKSSLEEPDFYKTKIKLCILLHAFRIRAVTIDRVMSYLNASGGGGSGG
GGSDKTHTCPPCPAPELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLPPSRDELTKNQVSLTC
LVKGFYPSDIAVEWESNGQPENNYKTTPPVLDSDGSFFLYSKLTVDKSRWQQGNVFSCSVMHEALHNHYTQKSLS
LSPGGGGGSGGGGSGGGGSQVQLQQPGAELVKPGASVKMSCKASGYTFTSYNMHWVKQTPGRGLEWIGAIYPGNG
DTSYNQKFKGKATLTADKSSSTAYMQLSSLTSEDSAVYYCARSTYYGGDWYFNVWGAGTTVTVSAGGGGSGGGGS
GGGGSGGGGSQIVLSQSPAILSASPGEKVTMTCRASSSVSYIHWFQQKPGSSPKPWIYATSNLASGVPVRFSGSG
SGTSYSLTISRVEAEDAATYYCQQWTSNPPTFGGGTKLEIK

### Preparation Example 2. Overview of anti-CD20/IL-12 IgG2a, mouse version fusion protein

**[Table 2]**

| PROTEIN | Format | Description | Corresponding sequence |
|---|---|---|---|
| T2.01m | K&H | anti-mu CD20/mu IL-12, mu IgG2a | seq22, seq23, seq24 |
| T2.02m | K&H | anti-mu CD20/mu IL-12 mut1, mu IgG2a | seq22, seq23, seq25 |
| T2.03m | K&H | anti-mu CD20/mu IL-12 mut2, mu IgG2a | seq22, seq23, seq26 |
| T2.04m | K&H | anti-mu CD20/mu IL-12, mu IgG2a (2+1) | seq24, seq27, seq28 |
| T2.05m | K&H | anti-mu CD20/mu IL-12 mut2, mu IgG2a (2+1) | seq26, seq27, seq28 |
| T2.06m | K&H | anti-mu CD20/mu IL-12, mu IgG2a (2+1, HC scFv) | seq23, seq24, seq29 |
| T2.07m | K&H | anti-mu CD20/mu II,-12 mut2, mu IgG2a (2+1, HC scFv) | seq23, seq26, seq29 |
| T2.08m | K&H | anti-mu CD20/mu IL-12, mu IgG2a (2+1, LC scFv) | seq22, seq24, seq30 |
| T2.09m | K&H | anti-mu CD20/mu IL-12 mut2, mu IgG2a (2+1, LC scFv) | seq22, seq26, seq30 |
| T2.10m | K&H | anti-mu CD20/anti-mu CD20-mu IL-12, mu IgG2a (2+1) | seq22, seq23, seq31 |
| T2.11m | K&H | anti-mu CD20/anti-mu CD20-mu IL-12 mut2, mu IgG2a (2+1) | seq22, seq23, seq32 |
| T2.12m | K&H | anti-mu CD20/mu IL-12 mut1, mu IgG2a DANG | seq23, seq33, seq35 |
| T2.13m | K&H | null/mu IL-12, mu IgG2a DANG | seq34, seq40 |
| T2.14m | K&H | null/mu IL-12 mut1, mu IgG2a DANG | seq35, seq40 |
| T2.15m | Fc-fusion | mu IL-12 mut2, mu IgG2a | seq36 |
| T2.16m | K&H | anti-mu FAP/mu II,-12 mut1, mu IgG2a | seq25, seq37, seq38 |
| T2.17m | K&H | anti-mu FAP/null, mu IgG2a DANG | seq38, seq39, seq41 |
| 18B12 | Mab | anti-mu CD20 (18B12) | seq23, seq42 |
| T2.18m | K&H | anti-mu CD20/mu IL-12-anti-mu CD20 scFv, mu IgG2a (2+1) | seq22, seq23, seq278 |
| T2.19m | K&H | anti-mu CD20/mu IL-12 mut2-anti-mu CD20 scFv, mu IgG2a (2+1) | seq22, seq23, seq279 |
| T1.20m | Fc-fusion | anti-mu CD20-mu IL-12, mu IgG2a | seq23, seq280 |
| T1.21m | Fc-fusion | anti-mu CD20-mu IL-12 mut2, mu IgG2a | seq23, seq281 |
| T 1.22m | Fc-fusion | mu IL-12-anti-mu CD20 scFv, mu IgG2a | seq282 |
| T1.23m | Fc-fusion | mu IL-12 mut2-anti-mu CD20 scFv, mu IgG2a | seq283 |

[seq22] is composed of a mouse anti-CD20 antibody heavy chain sequence and a mouse IgG2a Fc in which a knob structure is formed by a T321W mutation.

[seq23] is composed of a light chain sequence of a mouse anti-CD20 antibody 18B12.

[seq24] is composed of a mouse IL-12 p40 (beta) region sequence, a linker GGGGSGGGGSGGGGS, a mouse IL-12 p35 (alpha) region sequence, a linker GGGGSGGGGS, and a mouse IgG2a Fc in which a hole structure is formed by T321S, M323A, Y362V mutations.

[seq25] is composed of a sequence in which lysine (K) at the 277^{th} and 282^{nd} amino acids involved in heparin binding in a mouse IL-12 p40 (beta) region is mutated to alanine (A), a linker GGGGSGGGGSGGGGS, a mouse IL-12 p35 (alpha) region sequence, a linker GGGGSGGGGS, and a mouse IgG2a Fc hole.

[seq26] is composed of a sequence in which arginine (R) at the 276^{th} amino acid and lysine (K) at the 277^{th}, 278^{th} and 282^{nd} amino acids involved in heparin binding in a mouse IL-12 p40 (beta) region is mutated to alanine (A), a linker GGGGSGGGGSGGGGS, a mouse IL-12 p35 (alpha) region sequence, a linker GGGGSGGGGS and a mouse IgG2a Fc hole.

[seq27] is composed of a mouse anti-CD20 heavy chain variable region sequence, a linker GGGGSGGGGSGGGGS, a mouse anti-CD20 heavy chain variable region sequence, and a mouse IgG2a Fc knob.

[seq28] is composed of a mouse anti-CD20 light chain variable region sequence, a linker GGGGSGGGGSGGGGS, and a mouse anti-CD20 light chain sequence.

[seq29] is composed of a mouse anti-CD20 heavy chain variable region sequence, a mouse IgG2a Fc knob, a linker GGGGSGGGGSGGGGS, a mouse anti-CD20 heavy chain variable region sequence, a linker GGGGSGGGGSGGGGSGGGGS, and a mouse anti-CD20 light chain variable region sequence.

[seq30] is composed of a mouse anti-CD20 light chain sequence, a linker GGGGSGGGGSGGGGS, a mouse anti-CD20 heavy chain variable region sequence, a linker GGGGSGGGGSGGGGSGGGGS, and a mouse anti-CD20 light chain variable region sequence.

[seq31] is composed of a mouse anti-CD20 heavy chain variable region sequence, a mouse IgG2a Fc hole, a linker GGGGSGGGGSGGGGS, a mouse IL-12 p40 (beta) region sequence, a linker GGGGSGGGGSGGGGS, and a mouse IL-12 p35 (alpha) region sequence.

[seq32] is composed of a mouse anti-CD20 heavy chain variable region sequence, a mouse IgG2a Fc hole, a linker GGGGSGGGGSGGGGS, a mouse IL-12 p40 (beta) region sequence containing mutations (four) of amino acids involved in heparin binding, a linker GGGGSGGGGSGGGGS, and a mouse IL-12 p35 (alpha) region sequence.

[seq33] is composed of a heavy chain variable region sequence of a mouse anti-CD20 antibody and a mouse IgG2a Fc knob DANG.

[seq34] is composed of a mouse IL-12 p40 (beta) region sequence, a linker GGGGSGGGGSGGGGS, a mouse IL-12 p35 (alpha) region sequence, a linker GGGGSGGGGS, and a mouse IgG2a Fc hole DANG.

[seq35] is composed of a mouse IL-12 p40 (beta) region sequence containing mutations (two) of amino acids involved in heparin binding, a linker GGGGSGGGGSGGGGS, a mouse IL-12 p35 (alpha) region sequence, a linker GGGGSGGGGS, and a mouse IgG2a Fc hole DANG.

[seq36] is composed of a mouse IL-12 p40 (beta) region sequence containing mutations (four) of amino acids involved in heparin binding, a linker GGGGSGGGGSGGGGS, a mouse IL-12 p35 (alpha) region sequence, a linker GGGGSGGGGS, and a mouse IgG2a Fc.

[seq37] is composed of a mouse anti-FAP heavy chain variable region sequence and a mouse IgG2a Fc knob.

[seq38] is composed of a mouse anti-FAP light chain sequence.

[seq39] is composed of a mouse anti-FAP heavy chain variable region sequence and a mouse IgG2a Fc knob DANG.

[seq40] is composed of only a mouse IgG2a Fc knob DANG.

[seq41] is composed of only a mouse IgG2a Fc hole DANG.

[seq42] is a heavy chain sequence of a mouse anti-CD20 antibody 18B12.

[seq278] is composed of a mouse IL-12 p40 (beta) region sequence, a linker GGGGSGGGGSGGGGS, a mouse IL-12 p35 (alpha) region sequence, a linker GGGGSGGGGS, a mouse IgG2a Fc hole, a linker GGGGSGGGGSGGGGS, a mouse anti-CD20 heavy chain variable region sequence, a linker GGGGSGGGGSGGGGSGGGGS, and a mouse anti-CD20 light chain variable region sequence.

[seq279] is composed of a mouse IL-12 p40 (beta) region sequence containing mutations (four) of amino acids involved in heparin binding, a linker GGGGSGGGGSGGGGS, a mouse IL-12 p35 (alpha) region sequence, a linker GGGGSGGGGS and a mouse IgG2a Fc hole, a linker GGGGSGGGGSGGGGS, a mouse anti-CD20 heavy chain variable region sequence, a linker GGGGSGGGGSGGGGSGGGGS, and a mouse anti-CD20 light chain variable region sequence.

[seq280] is composed of a mouse anti-CD20 heavy chain sequence, a mouse IgG2a Fc, a linker GGGGSGGGGSGGGGS, a mouse IL-12 p40 (beta) region sequence, a linker GGGGSGGGGSGGGGS, and a mouse IL-12 p35 (alpha) region sequence.

[seq281] is composed of a mouse anti-CD20 heavy chain sequence, a mouse IgG2a Fc, a linker GGGGSGGGGSGGGGS, a mouse IL-12 p40 (beta) region sequence containing mutations (four) of amino acids involved in heparin binding, a linker GGGGSGGGGSGGGGS, and a mouse IL-12 p35 (alpha) region sequence.

[seq282] is composed of a mouse IL-12 p40 (beta) region sequence, a linker GGGGSGGGGSGGGGS, a mouse IL-12 p35 (alpha) region sequence, a linker GGGGSGGGGS and a mouse IgG2a Fc, a linker GGGGSGGGGSGGGGS, a mouse anti-CD20 heavy chain variable region sequence, a linker GGGGSGGGGSGGGGSGGGGS, and a mouse anti-CD20 light chain variable region sequence.

[seq283] is composed of a mouse IL-12 p40 (beta) region sequence containing mutations (four) of amino acids involved in heparin binding, a linker GGGGSGGGGSGGGGS, a mouse IL-12 p35 (alpha) region sequence, a linker GGGGSGGGGS and a mouse IgG2a Fc, a linker GGGGSGGGGSGGGGS, a mouse anti-CD20 heavy chain variable region sequence, a linker GGGGSGGGGSGGGGSGGGGS, and a mouse anti-CD20 light chain variable region sequence. T2.01m (seq22, seq23, seq24) is a bispecific antibody in which a mouse anti-CD20 sequence targeting protein CD20 and a mouse IL-12 sequence form a knob-into-hole structure.

T2.02m (seq22, seq23, seq25) is a bispecific antibody in which a mouse anti-CD20 sequence targeting protein CD20 and a mouse IL-12 sequence containing mutations (two) of amino acids involved in heparin binding form a knob-into-hole structure.

T2.03m (seq22, seq23, seq26) is a bispecific antibody in which a mouse anti-CD20 sequence targeting protein CD20 and a mouse IL-12 sequence containing mutations (four) of amino acids involved in heparin binding form a knob-into-hole structure.

T2.04m (seq24, seq27, seq28) is a bispecific antibody in which a mouse anti-CD20 sequence in a dual variable domain immunoglobulin (DVD-Ig) form and a mouse IL-12 sequence form a knob-into-hole structure.

T2.05m (seq26, seq27, seq28) is a bispecific antibody in which a mouse anti-CD20 sequence in a dual variable domain immunoglobulin (DVD-Ig) form and a mouse IL-12 sequence containing mutations (four) of amino acids involved in heparin binding form a knob-into-hole structure.

T2.06m (seq23, seq24, seq29) is a bispecific antibody in which a mouse anti-CD20 sequence in which a single chain variable fragment (scFv) of a mouse anti-CD20 sequence targeting protein CD20 is linked to the C-terminus of the heavy chain and a mouse IL-12 sequence form a knob-into-hole structure.

T2.07m (seq23, seq26, seq29) is a bispecific antibody in which a mouse anti-CD20 sequence in which a single chain variable fragment (scFv) of a mouse anti-CD20 sequence targeting protein CD20 is linked to the C-terminus of the heavy chain and a mouse IL-12 sequence containing mutations (four) of amino acids involved in heparin binding form a knob-into-hole structure.

T2.08m (seq22, seq24, seq30) is a bispecific antibody in which a mouse anti-CD20 sequence in which a single chain variable fragment (scFv) of a mouse anti-CD20 sequence targeting protein CD20 is linked to the C-terminus of the light chain and a mouse IL-12 sequence form a knob-into-hole structure.

T2.09m (seq22, seq26, seq30) is a bispecific antibody in which a mouse anti-CD20 sequence in which a single chain variable fragment (scFv) of a mouse anti-CD20 sequence targeting protein CD20 is linked to the C-terminus of the light chain and a mouse IL-12 sequence containing mutations (four) of amino acids involved in heparin binding form a knob-into-hole structure.

T2.10m (seq22, seq23, seq31) is an antibody in which a mouse anti-CD20 sequence targeting protein CD20 and a mouse anti-CD20 sequence in which a mouse IL-12 sequence is linked to the C-terminus of the heavy chain form a knob-into-hole structure.

T2.11m (seq22, seq23, seq32) is an antibody in which a mouse anti-CD20 sequence targeting protein CD20 and a mouse anti-CD20 sequence in which a mouse IL-12 sequence containing mutations (four) of amino acids involved in heparin binding is linked to the C-terminus of the heavy chain form a knob-into-hole structure.

T2.12m (seq23, seq33, seq35) is a bispecific antibody in which a mouse anti-CD20 sequence targeting protein CD20 and a mouse IL-12 sequence containing mutations (two) of amino acids involved in heparin binding form a knob-into-hole structure, and an effector function is removed.

T2.13m (seq34, seq40) is an antibody in which an effector function of a knob-into-hole structure having only a mouse IL-12 sequence is removed.

T2.14m (seq35, seq40) is an antibody in which an effector function of a knob-into-hole structure having only a mouse IL-12 sequence containing mutations (two) of amino acids involved in heparin binding is removed.

T2.15m (seq36) is an Fc-fusion protein having only a mouse IL-12 sequence containing mutations (four) of amino acids involved in heparin binding.

T2.16m (seq25, seq37, seq38) is a bispecific antibody in which a mouse anti-FAP sequence targeting protein FAP and a mouse IL-12 sequence containing mutations (two) of amino acids involved in heparin binding form a knob-into-hole structure.

T2.17m (seq38, seq39, seq41) is an antibody in which an effector function of a knob-into-hole structure having only a mouse anti-FAP sequence targeting protein FAP is removed. 18B12 (seq23, seq42) is a mouse anti-CD20 (18B12) antibody targeting protein CD20.

T2.18m (seq22, seq23, seq278) is a bispecific antibody in which a mouse anti-CD20 sequence and a mouse IL-12 sequence in which a single chain variable fragment (scFv) of a mouse anti-CD20 sequence is linked to the C-terminus form a knob-into-hole structure.

T2.19m (seq22, seq23, seq279) is a bispecific antibody in which a mouse anti-CD20 heavy chain sequence and a mouse IL-12 sequence containing mutations (four) of amino acids involved in heparin binding in which a single chain variable fragment (scFv) of a mouse anti-CD20 sequence is linked to the C-terminus form a knob-into-hole structure.

T2.20m (seq23, seq280) is an Fc-fusion protein dimer containing a mouse anti-CD20 sequence in which a mouse IL-12 sequence is linked to the C-terminus of the heavy chain.

T2.21m (seq23, seq281) is an Fc-fusion protein dimer containing a mouse anti-CD20 sequence in which a mouse IL-12 sequence containing mutations (four) of amino acids involved in heparin binding is linked to the C-terminus of the heavy chain.

T2.22m (seq282) is an Fc-fusion protein dimer containing a mouse IL-12 sequence in which a single chain variable fragment (scFv) of a mouse anti-CD20 sequence is linked to the C-terminus.

T2.23m (seq283) is an Fc-fusion protein dimer composed of a mouse IL-12 sequence containing mutations (four) of amino acids involved in heparin binding in which a single chain variable fragment (scFv) of a mouse anti-CD20 sequence is linked to the C-terminus.
[seq22] anti-mu CD20 HC mu IgG2a Fc knob 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
AYWGQGTLVTVSSAKTTAPSVYPLAPVCGDTTGSSVTLGCLVKGYFPEPVTLTWNSG
 
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTIS
KPKGSVRAPQVYVLPPPEEEMTKKQVTLWCMVTDFMPEDIYVEWTNNGKTELNYK
NTEPVLDSDGSYFMYSKLRVEKKNWVERNSYSCSVVHEGLHNHHTTKSFSRTPGK
[seq23] anti-mu CD20 LC 
QIVMSQSPAILSASPGEKVTMTCRARSSVSYIHWYQQKPGSSPKPWIYATSNL
ASGVPGRFSGSGSGTSYSLTITRVEAEDAATYYCQQWSSKPPTFGGGTKLEIKRTDAA
PTVSIFPPSSEQLTSGGASVVCFLNNFYPKDINVKWKIDGSERQNGVLNSWTDQDSKD
STYSMSSTLTLTKDEYERHNSYTCEATHKTSTSPIVKSFNRNEC
[seq24] mu scIL-12-mu IgG2a Fc hole 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQRKKEKMKETEEGCNQKGAFLVEKTSTEV
QCKGGNVCVQAQDRYYNSSCSKWACVPCRVRSGGGGSGGGGSGGGGSRVIPVSGPA
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVDVSEDDPDVQISWFVNNVEVHTAQTQTHREDYNSTLRVVSALPI
QHQDWMSGKEFKCKVNNKDLPAPIERTISKPKGSVRAPQVYVLPPPEEEMTKKQVTL
SCAVTDFMPEDIYVEWTNNGKTELNYKNTEPVLDSDGSYFMVSKLRVEKKNWVER
NSYSCSVVHEGLHNHHTTKSFSRTPGK
[seq25] mu scIL-12 mut1-mu IgG2a Fc hole 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQRAKEKMAETEEGCNQKGAFLVEKTSTEV
QCKGGNVCVQAQDRYYNSSCSKWACVPCRVRSGGGGSGGGGSGGGGSRVIPVSGPA
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVDVSEDDPDVQISWFVNNVEVHTAQTQTHREDYNSTLRVVSALPI
QHQDWMSGKEFKCKVNNKDLPAPIERTISKPKGSVRAPQVYVLPPPEEEMTKKQVTL
 
SCAVTDFMPEDIYVEWTNNGKTELNYKNTEPVLDSDGSYFMVSKLRVEKKNWVER
NSYSCSVVHEGLHNHHTTKSFSRTPGK
[seq26] mu scIL-12 mut2-mu IgG2a Fc hole 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQAAAEKMAETEEGCNQKGAFLVEKTSTEV
QCKGGNVCVQAQDRYYNSSCSKWACVPCRVRSGGGGSGGGGSGGGGSRVIPVSGPA
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVDVSEDDPDVQISWFVNNVEVHTAQTQTHREDYNSTLRVVSALPI
QHQDWMSGKEFKCKVNNKDLPAPIERTISKPKGSVRAPQVYVLPPPEEEMTKKQVTL
SCAVTDFMPEDIYVEWTNNGKTELNYKNTEPVLDSDGSYFMVSKLRVEKKNWVER
NSYSCSVVHEGLHNHHTTKSFSRTPGK
[seq27] (anti-mu CD20 VH)2-mu IgG2a Fc knob 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
AYWGQGTLVTVSSGGGGSGGGGSGGGGSQVQLQQPGAELVRPGTSVKLSCKASGY
TFTSYWMHWIKQRPGQGLEWIGVIDPSDNYTKYNQKFKGKATLTVDTSSSTAYMQL
SSLTSEDSAVYFCAREGYYGSSPWFAYWGQGTLVTVSSAKTTAPSVYPLAPVCGDTT
GSSVTLGCLVKGYFPEPVTLTWNSGSLSSGVHTFPAVLQSDLYTLSSSVTVTSSTWPSQ
SITCNVAHPASSTKVDKKIEPRGPTIKPCPPCKCPAPNLLGGPSVFIFPPKIKDVLMISLS
PIVTCVVVDVSEDDPDVQISWFVNNVEVHTAQTQTHREDYNSTLRVVSALPIQHQD
WMSGKEFKCKVNNKDLPAPIERTISKPKGSVRAPQVYVLPPPEEEMTKKQVTLWCM
VTDFMPEDIYVEWTNNGKTELNYKNTEPVLDSDGSYFMYSKLRVEKKNWVERNSY
SCSVVHEGLHNHHTTKSFSRTPGK
[seq28] (anti-mu CD20 VL)2 
QIVMSQSPAILSASPGEKVTMTCRARSSVSYIHWYQQKPGSSPKPWIYATSNL
ASGVPGRFSGSGSGTSYSLTITRVEAEDAATYYCQQWSSKPPTFGGGTKLEIKGGGGS
GGGGSGGGGSQIVMSQSPAILSASPGEKVTMTCRARSSVSYIHWYQQKPGSSPKPWI
YATSNLASGVPGRFSGSGSGTSYSLTITRVEAEDAATYYCQQWSSKPPTFGGGTKLEI
KRTDAAPTVSIFPPSSEQLTSGGASVVCFLNNFYPKDINVKWKIDGSERQNGVLNSWT
DQDSKDSTYSMSSTLTLTKDEYERHNSYTCEATHKTSTSPIVKSFNRNEC
[seq29] anti-mu CD20 HC mu IgG2a Fc knob-anti-mu CD20 scFv QVQLQQPGAELVRPGTSVKLSCKASGYTFTSYWMHWIKQRPGQGLEWIGVI 
DPSDNYTKYNQKFKGKATLTVDTSSSTAYMQLSSLTSEDSAVYFCAREGYYGSSPWF
AYWGQGTLVTVSSAKTTAPSVYPLAPVCGDTTGSSVTLGCLVKGYFPEPVTLTWNSG
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTIS
KPKGSVRAPQVYVLPPPEEEMTKKQVTLWCMVTDFMPEDIYVEWTNNGKTELNYK
NTEPVLDSDGSYFMYSKLRVEKKNWVERNSYSCSVVHEGLHNHHTTKSFSRTPGKG
GGGSGGGGSGGGGSQVQLQQPGAELVRPGTSVKLSCKASGYTFTSYWMHWIKQRP
GQGLEWIGVIDPSDNYTKYNQKFKGKATLTVDTSSSTAYMQLSSLTSEDSAVYFCARE
GYYGSSPWFAYWGQGTLVTVSSGGGGSGGGGSGGGGSGGGGSQIVMSQSPAILSASP
GEKVTMTCRARSSVSYIHWYQQKPGSSPKPWIYATSNLASGVPGRFSGSGSGTSYSLT
ITRVEAEDAATYYCQQWSSKPPTFGGGTKLEIK
[seq30] anti-mu CD20 LC-anti-mu CD20 scFv 
QIVMSQSPAILSASPGEKVTMTCRARSSVSYIHWYQQKPGSSPKPWIYATSNL
ASGVPGRFSGSGSGTSYSLTITRVEAEDAATYYCQQWSSKPPTFGGGTKLEIKRTDAA
PTVSIFPPSSEQLTSGGASVVCFLNNFYPKDINVKWKIDGSERQNGVLNSWTDQDSKD
STYSMSSTLTLTKDEYERHNSYTCEATHKTSTSPIVKSFNRNECGGGGSGGGGSGGGG
SQVQLQQPGAELVRPGTSVKLSCKASGYTFTSYWMHWIKQRPGQGLEWIGVIDPSD
NYTKYNQKFKGKATLTVDTSSSTAYMQLSSLTSEDSAVYFCAREGYYGSSPWFAYWG
QGTLVTVSSGGGGSGGGGSGGGGSGGGGSQIVMSQSPAILSASPGEKVTMTCRARSS
VSYIHWYQQKPGSSPKPWIYATSNLASGVPGRFSGSGSGTSYSLTITRVEAEDAATYY
CQQWSSKPPTFGGGTKLEIK
[seq31] anti-mu CD20 HC mu IgG2a Fc hole-mu scIL-12 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
AYWGQGTLVTVSSAKTTAPSVYPLAPVCGDTTGSSVTLGCLVKGYFPEPVTLTWNSG
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTIS
KPKGSVRAPQVYVLPPPEEEMTKKQVTLSCAVTDFMPEDIYVEWTNNGKTELNYKN
TEPVLDSDGSYFMVSKLRVEKKNWVERNSYSCSVVHEGLHNHHTTKSFSRTPGKGG
GGSGGGGSGGGGSMWELEKDVYVVEVDWTPDAPGETVNLTCDTPEEDDITWTSDQ
RHGVIGSGKTLTITVKEFLDAGQYTCHKGGETLSHSHLLLHKKENGIWSTEILKNFKN
KTFLKCEAPNYSGRFTCSWLVQRNMDLKFNIKSSSSSPDSRAVTCGMASLSAEKVTL
DQRDYEKYSVSCQEDVTCPTAEETLPIELALEARQQNKYENYSTSFFIRDIIKPDPPKN
LQMKPLKNSQVEVSWEYPDSWSTPHSYFSLKFFVRIQRKKEKMKETEEGCNQKGAF
LVEKTSTEVQCKGGNVCVQAQDRYYNSSCSKWACVPCRVRSGGGGSGGGGSGGGG
SRVIPVSGPARCLSQSRNLLKTTDDMVKTAREKLKHYSCTAEDIDHEDITRDQTSTLK
 
TCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCI
LLHAFSTRVVTINRVMGYLSSA
[seq32] anti-mu CD20 HC mu IgG2a Fc hole-mu scIL-12 mut2 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
AYWGQGTLVTVSSAKTTAPSVYPLAPVCGDTTGSSVTLGCLVKGYFPEPVTLTWNSG
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTIS
KPKGSVRAPQVYVLPPPEEEMTKKQVTLSCAVTDFMPEDIYVEWTNNGKTELNYKN
TEPVLDSDGSYFMVSKLRVEKKNWVERNSYSCSVVHEGLHNHHTTKSFSRTPGKGG
GGSGGGGSGGGGSMWELEKDVYVVEVDWTPDAPGETVNLTCDTPEEDDITWTSDQ
RHGVIGSGKTLTITVKEFLDAGQYTCHKGGETLSHSHLLLHKKENGIWSTEILKNFKN
KTFLKCEAPNYSGRFTCSWLVQRNMDLKFNIKSSSSSPDSRAVTCGMASLSAEKVTL
DQRDYEKYSVSCQEDVTCPTAEETLPIELALEARQQNKYENYSTSFFIRDIIKPDPPKN
LQMKPLKNSQVEVSWEYPDSWSTPHSYFSLKFFVRIQAAAEKMAETEEGCNQKGAF
LVEKTSTEVQCKGGNVCVQAQDRYYNSSCSKWACVPCRVRSGGGGSGGGGSGGGG
SRVIPVSGPARCLSQSRNLLKTTDDMVKTAREKLKHYSCTAEDIDHEDITRDQTSTLK
TCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCI
LLHAFSTRVVTINRVMGYLSSA
[seq33] anti-mu CD20 HC mu IgG2a Fc knob DANG 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
AYWGQGTLVTVSSAKTTAPSVYPLAPVCGDTTGSSVTLGCLVKGYFPEPVTLTWNSG
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVAVSEDDPDVQISWFVNN
VEVHTAQTQTHREDYGSTLRVVSALPIQHQDWMSGKEFKCKVNNKDLPAPIERTISK
PKGSVRAPQVYVLPPPEEEMTKKQVTLWCMVTDFMPEDIYVEWTNNGKTELNYKN
TEPVLDSDGSYFMYSKLRVEKKNWVERNSYSCSVVHEGLHNHHTTKSFSRTPGK
[seq34] mu scIL-12-mu IgG2a Fc hole DANG 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQRKKEKMKETEEGCNQKGAFLVEKTSTEV
QCKGGNVCVQAQDRYYNSSCSKWACVPCRVRSGGGGSGGGGSGGGGSRVIPVSGPA
 
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVAVSEDDPDVQISWFVNNVEVHTAQTQTHREDYGSTLRVVSALPI
QHQDWMSGKEFKCKVNNKDLPAPIERTISKPKGSVRAPQVYVLPPPEEEMTKKQVTL
SCAVTDFMPEDIYVEWTNNGKTELNYKNTEPVLDSDGSYFMVSKLRVEKKNWVER
NSYSCSVVHEGLHNHHTTKSFSRTPGK
[seq35] mu scIL-12 mut1-mu IgG2a Fc hole DANG 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQRAKEKMAETEEGCNQKGAFLVEKTSTEV
QCKGGNVCVQAQDRYYNSSCSKWACVPCRVRSGGGGSGGGGSGGGGSRVIPVSGPA
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVAVSEDDPDVQISWFVNNVEVHTAQTQTHREDYGSTLRVVSALPI
QHQDWMSGKEFKCKVNNKDLPAPIERTISKPKGSVRAPQVYVLPPPEEEMTKKQVTL
SCAVTDFMPEDIYVEWTNNGKTELNYKNTEPVLDSDGSYFMVSKLRVEKKNWVER
NSYSCSVVHEGLHNHHTTKSFSRTPGK
[seq36] mu scIL-12 mut2-mu IgG2a Fc 
MWELEKDVYVVEVDWTPDAPGETVNLTCDTPEEDDITWTSDQRHGVIGSG
KTLTITVKEFLDAGQYTCHKGGETLSHSHLLLHKKENGIWSTEILKNFKNKTFLKCEA
PNYSGRFTCSWLVQRNMDLKFNIKSSSSSPDSRAVTCGMASLSAEKVTLDQRDYEKY
SVSCQEDVTCPTAEETLPIELALEARQQNKYENYSTSFFIRDIIKPDPPKNLQMKPLKN
SQVEVSWEYPDSWSTPHSYFSLKFFVRIQAAAEKMAETEEGCNQKGAFLVEKTSTEV
QCKGGNVCVQAQDRYYNSSCSKWACVPCRVRSGGGGSGGGGSGGGGSRVIPVSGPA
RCLSQSRNLLKTTDDMVKTAREKLKHYSCTAEDIDHEDITRDQTSTLKTCLPLELHK
NESCLATRETSSTTRGSCLPPQKTSLMMTLCLGSIYEDLKMYQTEFQAINAALQNHN
HQQIILDKGMLVAIDELMQSLNHNGETLRQKPPVGEADPYRVKMKLCILLHAFSTRV
VTINRVMGYLSSAGGGGSGGGGSEPRGPTIKPCPPCKCPAPNLLGGPSVFIFPPKIKDV
LMISLSPIVTCVVVDVSEDDPDVQISWFVNNVEVHTAQTQTHREDYNSTLRVVSALPI
QHQDWMSGKEFKCKVNNKDLPAPIERTISKPKGSVRAPQVYVLPPPEEEMTKKQVTL
TCMVTDFMPEDIYVEWTNNGKTELNYKNTEPVLDSDGSYFMYSKLRVEKKNWVER
NSYSCSVVHEGLHNHHTTKSFSRTPGK
[seq37] anti-mu FAP HC mu IgG2a Fc knob 
QVQLQQSGAELARPGASVNLSCKASGYTFTNNGINWLKQRTGQGLEWIGEI
YPRSTNTLYNEKFKGKATLTADRSSNTAYMELRSLTSEDSAVYFCARTLTAPFAFWGQ
GTLVTVSAAKTTAPSVYPLAPVCGDTTGSSVTLGCLVKGYFPEPVTLTWNSGSLSSGV
HTFPAVLQSDLYTLSSSVTVTSSTWPSQSITCNVAHPASSTKVDKKIEPRGPTIKPCPPC
KCPAPNLLGGPSVFIFPPKIKDVLMISLSPIVTCVVVDVSEDDPDVQISWFVNNVEVHT
AQTQTHREDYNSTLRVVSALPIQHQDWMSGKEFKCKVNNKDLPAPIERTISKPKGSV
RAPQVYVLPPPEEEMTKKQVTLWCMVTDFMPEDIYVEWTNNGKTELNYKNTEPVL
DSDGSYFMYSKLRVEKKNWVERNSYSCSVVHEGLHNHHTTKSFSRTPGK
[seq38] anti-mu FAP LC 
QIVLTQSPAIMSASPGEKVTMTCSASSGVNFMHWYQQKSGTSPKRWIFDTSK
LASGVPARFSGSGSGTSYSLTISSMEAEDAATYYCQQWSFNPPTFGGGTKLEIKRADA
APTVSIFPPSSEQLTSGGASVVCFLNNFYPKDINVKWKIDGSERQNGVLNSWTDQDSK
DSTYSMSSTLTLTKDEYERHNSYTCEATHKTSTSPIVKSFNRNEC
[seq39] anti-mu FAP HC mu IgG2a Fc knob DANG 
QVQLQQSGAELARPGASVNLSCKASGYTFTNNGINWLKQRTGQGLEWIGEI
YPRSTNTLYNEKFKGKATLTADRSSNTAYMELRSLTSEDSAVYFCARTLTAPFAFWGQ
GTLVTVSAAKTTAPSVYPLAPVCGDTTGSSVTLGCLVKGYFPEPVTLTWNSGSLSSGV
HTFPAVLQSDLYTLSSSVTVTSSTWPSQSITCNVAHPASSTKVDKKIEPRGPTIKPCPPC
KCPAPNLLGGPSVFIFPPKIKDVLMISLSPIVTCVVVAVSEDDPDVQISWFVNNVEVHT
AQTQTHREDYGSTLRVVSALPIQHQDWMSGKEFKCKVNNKDLPAPIERTISKPKGSV
RAPQVYVLPPPEEEMTKKQVTLWCMVTDFMPEDIYVEWTNNGKTELNYKNTEPVL
DSDGSYFMYSKLRVEKKNWVERNSYSCSVVHEGLHNHHTTKSFSRTPGK
[seq40] mu IgG2a Fc knob DANG 
EPRGPTIKPCPPCKCPAPNLLGGPSVFIFPPKIKDVLMISLSPIVTCVVVAVSED
DPDVQISWFVNNVEVHTAQTQTHREDYGSTLRVVSALPIQHQDWMSGKEFKCKVN
NKDLPAPIERTISKPKGSVRAPQVYVLPPPEEEMTKKQVTLWCMVTDFMPEDIYVEW
TNNGKTELNYKNTEPVLDSDGSYFMYSKLRVEKKNWVERNSYSCSVVHEGLHNHH
TTKSFSRTPGK
[seq41] mu IgG2a Fc hole DANG 
EPRGPTIKPCPPCKCPAPNLLGGPSVFIFPPKIKDVLMISLSPIVTCVVVAVSED
DPDVQISWFVNNVEVHTAQTQTHREDYGSTLRVVSALPIQHQDWMSGKEFKCKVN
NKDLPAPIERTISKPKGSVRAPQVYVLPPPEEEMTKKQVTLSCAVTDFMPEDIYVEWT
NNGKTELNYKNTEPVLDSDGSYFMVSKLRVEKKNWVERNSYSCSVVHEGLHNHHT
TKSFSRTPGK
[seq42] anti-mu CD20 (18B12) HC 
QVQLQQPGAELVRPGTSVKLSCKASGYTFTSYWMHWIKQRPGQGLEWIGVI
DPSDNYTKYNQKFKGKATLTVDTSSSTAYMQLSSLTSEDSAVYFCAREGYYGSSPWF
 
AYWGQGTLVTVSSAKTTAPSVYPLAPVCGDTTGSSVTLGCLVKGYFPEPVTLTWNSG
SLSSGVHTFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQISWFVN
NVEVHTAQTQTHREDYNSTLRVVSALPIQHQDWMSGKEFKCKVNNKDLPAPIERTIS
KPKGSVRAPQVYVLPPPEEEMTKKQVTLTCMVTDFMPEDIYVEWTNNGKTELNYKN
TEPVLDSDGSYFMYSKLRVEKKNWVERNSYSCSVVHEGLHNHHTTKSFSRTPGK
[seq278] mu scIL-12-mu IgG2a Fc hole-anti-mu CD20 scFv 
MWELEKDVYVVEVDWTPDAPGETVNLTCDTPEEDDITWTSDQRHGVIGSGKTLTITVKEFLDAGQYTC
HKGGETLSHSHLLLHKKENGIWSTEILKNFKNKTFLKCEAPNYSGRFTCSWLVQRNMDLKFNIKSSSSSPDSRAV
TCGMASLSAEKVTLDQRDYEKYSVSCQEDVTCPTAEETLPIELALEARQQNKYENYSTSFFIRDIIKPDPPKNLQ
MKPLKNSQVEVSWEYPDSWSTPHSYFSLKFFVRIQRKKEKMKETEEGCNQKGAFLVEKTSTEVQCKGGNVCVQAQ
DRYYNSSCSKWACVPCRVRSGGGGSGGGGSGGGGSRVIPVSGPARCLSQSRNLLKTTDDMVKTAREKLKHYSCTA
EDIDHEDITRDQTSTLKTCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCILLHAFSTRVVTINRVMGYL
SSAGGGGSGGGGSEPRGPTIKPCPPCKCPAPNLLGGPSVFIFPPKIKDVLMISLSPIVTCVVVDVSEDDPDVQIS
WFVNNVEVHTAQTQTHREDYNSTLRVVSALPIQHQDWMSGKEFKCKVNNKDLPAPIERTISKPKGSVRAPQVYVL
PPPEEEMTKKQVTLSCAVTDFMPEDIYVEWTNNGKTELNYKNTEPVLDSDGSYFMVSKLRVEKKNWVERNSYSCS
VVHEGLHNHHTTKSFSRTPGGGGGSGGGGSGGGGSQVQLQQPGAELVRPGTSVKLSCKASGYTFTSYWMHWIKQR
PGQGLEWIGVIDPSDNYTKYNQKFKGKATLTVDTSSSTAYMQLSSLTSEDSAVYFCAREGYYGSSPWFAYWGQGT
LVTVSSGGGGSGGGGSGGGGSGGGGSQIVMSQSPAILSASPGEKVTMTCRARSSVSYIHWYQQKPGSSPKPWIYA
TSNLASGVPGRFSGSGSGTSYSLTITRVEAEDAATYYCQQWSSKPPTFGGGTKLEIK
[seq279] mu scIL-12 mut2-mu IgG2a Fc hole-anti-mu CD20 scFv 
MWELEKDVYVVEVDWTPDAPGETVNLTCDTPEEDDITWTSDQRHGVIGSGKTLTITVKEFLDAGQYTC
HKGGETLSHSHLLLHKKENGIWSTEILKNFKNKTFLKCEAPNYSGRFTCSWLVQRNMDLKFNIKSSSSSPDSRAV
TCGMASLSAEKVTLDQRDYEKYSVSCQEDVTCPTAEETLPIELALEARQQNKYENYSTSFFIRDIIKPDPPKNLQ
MKPLKNSQVEVSWEYPDSWSTPHSYFSLKFFVRIQAAAEKMAETEEGCNQKGAFLVEKTSTEVQCKGGNVCVQAQ
DRYYNSSCSKWACVPCRVRSGGGGSGGGGSGGGGSRVIPVSGPARCLSQSRNLLKTTDDMVKTAREKLKHYSCTA
EDIDHEDITRDQTSTLKTCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCILLHAFSTRVVTINRVMGYL
SSAGGGGSGGGGSEPRGPTIKPCPPCKCPAPNLLGGPSVFIFPPKIKDVLMISLSPIVTCVVVDVSEDDPDVQIS
WFVNNVEVHTAQTQTHREDYNSTLRVVSALPIQHQDWMSGKEFKCKVNNKDLPAPIERTISKPKGSVRAPQVYVL
PPPEEEMTKKQVTLSCAVTDFMPEDIYVEWTNNGKTELNYKNTEPVLDSDGSYFMVSKLRVEKKNWVERNSYSCS
VVHEGLHNHHTTKSFSRTPGGGGGSGGGGSGGGGSQVQLQQPGAELVRPGTSVKLSCKASGYTFTSYWMHWIKQR
PGQGLEWIGVIDPSDNYTKYNQKFKGKATLTVDTSSSTAYMQLSSLTSEDSAVYFCAREGYYGSSPWFAYWGQGT
LVTVSSGGGGSGGGGSGGGGSGGGGSQIVMSQSPAILSASPGEKVTMTCRARSSVSYIHWYQQKPGSSPKPWIYA
TSNLASGVPGRFSGSGSGTSYSLTITRVEAEDAATYYCQQWSSKPPTFGGGTKLEIK
[seq280] anti-mu CD20 HC mu IgG2a F_{c}-mu scIL-12 
QVQLQQPGAELVRPGTSVKLSCKASGYTFTSYWMHWIKQRPGQGLEWIGVIDPSDNYTKYNQKFKGKA
TLTVDTSSSTAYMQLSSLTSEDSAVYFCAREGYYGSSPWFAYWGQGTLVTVSSAKTTAPSVYPLAPVCGDTTGSS
VTLGCLVKGYFPEPVTLTWNSGSLSSGVHTFPAVLQSDLYTLSSSVTVTSSTWPSQSITCNVAHPASSTKVDKKI
EPRGPTIKPCPPCKCPAPNLLGGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPKGSVRAPQVYVLPPPEEEMTKKQVT
LTCMVTDFMPEDIYVEWTNNGKTELNYKNTEPVLDSDGSYFMYSKLRVEKKNWVERNSYSCSVVHEGLHNHHTTK
SFSRTPGGGGGSGGGGSGGGGSMWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSGRFTCSWLVQRNMDLK
FNIKSSSSSPDSRAVTCGMASLSAEKVTLDQRDYEKYSVSCQEDVTCPTAEETLPIELALEARQQNKYENYSTSF
FIRDIIKPDPPKNLQMKPLKNSQVEVSWEYPDSWSTPHSYFSLKFFVRIQRKKEKMKETEEGCNQKGAFLVEKTS
TEVQCKGGNVCVQAQDRYYNSSCSKWACVPCRVRSGGGGSGGGGSGGGGSRVIPVSGPARCLSQSRNLLKTTDDM
VKTAREKLKHYSCTAEDIDHEDITRDQTSTLKTCLPLELHKNESCLATRETSSTTRGSCLPPQKTSLMMTLCLGS
IYEDLKMYQTEFQAINAALQNHNHQQIILDKGMLVAIDELMQSLNHNGETLRQKPPVGEADPYRVKMKLCILLHA
FSTRVVTINRVMGYLSSA
[seq281] anti-mu CD20 HC mu IgG2a Fc-mu scIL-12 mut2 
QVQLQQPGAELVRPGTSVKLSCKASGYTFTSYWMHWIKQRPGQGLEWIGVIDPSDNYTKYNQKFKGKA
TLTVDTSSSTAYMQLSSLTSEDSAVYFCAREGYYGSSPWFAYWGQGTLVTVSSAKTTAPSVYPLAPVCGDTTGSS
VTLGCLVKGYFPEPVTLTWNSGSLSSGVHTFPAVLQSDLYTLSSSVTVTSSTWPSQSITCNVAHPASSTKVDKKI
EPRGPTIKPCPPCKCPAPNLLGGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPKGSVRAPQVYVLPPPEEEMTKKQVT
LTCMVTDFMPEDIYVEWTNNGKTELNYKNTEPVLDSDGSYFMYSKLRVEKKNWVERNSYSCSVVHEGLHNHHTTK
SFSRTPGGGGGSGGGGSGGGGSMWELEKDVYVVEVDWTPDAPGETVNLTCDTPEEDDITWTSDQRHGVIGSGKTL
TITVKEFLDAGQYTCHKGGETLSHSHLLLHKKENGIWSTEILKNFKNKTFLKCEAPNYSGRFTCSWLVQRNMDLK
FNIKSSSSSPDSRAVTCGMASLSAEKVTLDQRDYEKYSVSCQEDVTCPTAEETLPIELALEARQQNKYENYSTSF
FIRDIIKPDPPKNLQMKPLKNSQVEVSWEYPDSWSTPHSYFSLKFFVRIQAAAEKMAETEEGCNQKGAFLVEKTS
TEVQCKGGNVCVQAQDRYYNSSCSKWACVPCRVRSGGGGSGGGGSGGGGSRVIPVSGPARCLSQSRNLLKTTDDM
VKTAREKLKHYSCTAEDIDHEDITRDQTSTLKTCLPLELHKNESCLATRETSSTTRGSCLPPQKTSLMMTLCLGS
IYEDLKMYQTEFQAINAALQNHNHQQIILDKGMLVAIDELMQSLNHNGETLRQKPPVGEADPYRVKMKLCILLHA
FSTRVVTINRVMGYLSSA
[seq282] mu scIL-12-mu IgG2a Fc-anti-mu CD20 scFv 
MWELEKDVYVVEVDWTPDAPGETVNLTCDTPEEDDITWTSDQRHGVIGSGKTLTITVKEFLDAGQYTC
HKGGETLSHSHLLLHKKENGIWSTEILKNFKNKTFLKCEAPNYSGRFTCSWLVQRNMDLKFNIKSSSSSPDSRAV
TCGMASLSAEKVTLDQRDYEKYSVSCQEDVTCPTAEETLPIELALEARQQNKYENYSTSFFIRDIIKPDPPKNLQ
MKPLKNSQVEVSWEYPDSWSTPHSYFSLKFFVRIQRKKEKMKETEEGCNQKGAFLVEKTSTEVQCKGGNVCVQAQ
DRYYNSSCSKWACVPCRVRSGGGGSGGGGSGGGGSRVIPVSGPARCLSQSRNLLKTTDDMVKTAREKLKHYSCTA
EDIDHEDITRDQTSTLKTCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCILLHAFSTRVVTINRVMGYL
SSAGGGGSGGGGSEPRGPTIKPCPPCKCPAPNLLGGPSVFIFPPKIKDVLMISLSPIVTCVVVDVSEDDPDVQIS
WFVNNVEVHTAQTQTHREDYNSTLRVVSALPIQHQDWMSGKEFKCKVNNKDLPAPIERTISKPKGSVRAPQVYVL
PPPEEEMTKKQVTLTCMVTDFMPEDIYVEWTNNGKTELNYKNTEPVLDSDGSYFMYSKLRVEKKNWVERNSYSCS
VVHEGLHNHHTTKSFSRTPGGGGGSGGGGSGGGGSQVQLQQPGAELVRPGTSVKLSCKASGYTFTSYWMHWIKQR
PGQGLEWIGVIDPSDNYTKYNQKFKGKATLTVDTSSSTAYMQLSSLTSEDSAVYFCAREGYYGSSPWFAYWGQGT
LVTVSSGGGGSGGGGSGGGGSGGGGSQIVMSQSPAILSASPGEKVTMTCRARSSVSYIHWYQQKPGSSPKPWIYA
TSNLASGVPGRFSGSGSGTSYSLTITRVEAEDAATYYCQQWSSKPPTFGGGTKLEIK
[seq283] mu seIL-12 mut 2-mu IgG2a Fc-anti-mu CD20 seFv

MWELEKDVYVVEVDWTPDAPGETVNLTCDTPEEDDITWTSDQRHGVIGSGKTLTITVKEFLDAGQYTC
HKGGETLSHSHLLLHKKENGIWSTEILKNFKNKTFLKCEAPNYSGRFTCSWLVQRNMDLKFNIKSSSSSPDSRAV
TCGMASLSAEKVTLDQRDYEKYSVSCQEDVTCPTAEETLPIELALEARQQNKYENYSTSFFIRDIIKPDPPKNLQ
MKPLKNSQVEVSWEYPDSWSTPHSYFSLKFFVRIQAAAEKMAETEEGCNQKGAFLVEKTSTEVQCKGGNVCVQAQ
DRYYNSSCSKWACVPCRVRSGGGGSGGGGSGGGGSRVIPVSGPARCLSQSRNLLKTTDDMVKTAREKLKHYSCTA
EDIDHEDITRDQTSTLKTCLPLELHKNESCLATRETSSTTRGSCLPPQKTSLMMTLCLGSIYEDLKMYQTEFQAI
NAALQNHNHQQIILDKGMLVAIDELMQSLNHNGETLRQKPPVGEADPYRVKMKLCILLHAFSTRVVTINRVMGYL
SSAGGGGSGGGGSEPRGPTIKPCPPCKCPAPNLLGGPSVFIFPPKIKDVLMISLSPIVTCVVVDVSEDDPDVQIS
WFVNNVEVHTAQTQTHREDYNSTLRVVSALPIQHQDWMSGKEFKCKVNNKDLPAPIERTISKPKGSVRAPQVYVL
PPPEEEMTKKQVTLTCMVTDFMPEDIYVEWTNNGKTELNYKNTEPVLDSDGSYFMYSKLRVEKKNWVERNSYSCS
VVHEGLHNHHTTKSFSRTPGGGGGSGGGGSGGGGSQVQLQQPGAELVRPGTSVKLSCKASGYTFTSYWMHWIKQR
PGQGLEWIGVIDPSDNYTKYNQKFKGKATLTVDTSSSTAYMQLSSLTSEDSAVYFCAREGYYGSSPWFAYWGQGT
LVTVSSGGGGSGGGGSGGGGSGGGGSQIVMSQSPAILSASPGEKVTMTCRARSSVSYIHWYQQKPGSSPKPWIYA
TSNLASGVPGRFSGSGSGTSYSLTITRVEAEDAATYYCQQWSSKPPTFGGGTKLEIK

### Manufacturing Example 1. Manufacturing of fusion proteins

Reagents and equipment are described in Tables 3 and 4 below.

**[Table 3]**

| Reagent | Manufacturer | Catalog # |
|---|---|---|
| pTT5 | chempartner | |
| In-Fusion HD cloning kit | Clontech | 639648 |
| Accuprime pfx DNA polymerase | Invitrogen | 12344-04 |
| Gel DNA fragment purification Kit | TaKaRa | D823A |
| FastDigest^{®}BamHI | Fermentas | FD0055 |
| FastDigest^{®}EcoRI | Fermentas | FD0275 |

**[Table 4]**

| Equipment and tool | Manufacturer | Model name |
|---|---|---|
| Biosafety cabinet | NUAIRE | LabGard class ± |
| Centrifuge | Eppendorf | 5424 |
| Gel Imaging System | Tanon | 2500R |

The synthesized DNA fragment was amplified through PCR, and the PCR product was purified by gel. The pTT5 vector was cleaved by the restriction enzymes EcoRI and BamHI, and then the gel was purified. Each PCR product and the linear vector were ligated using the In-Fusion kit. The produced vector was transformed into ECOS101 DH5α competent cells and the cells were cultured on 2xYT agar plates containing 100 µg/mL ampicillin. All manipulation processes were performed according to standard transformation protocols. Positive recombinants were identified by colony PCR, and sequence-verification sequencing was performed on the recombinant plasmid. A single colony was selected and the seed culture was inoculated into 5 mL of 2xYT medium containing 100 µg/mL ampicillin. It was cultured with shaking at 37°C for 8 hours.

Thereafter, the seed culture was diluted in 200 mL of selective 2xYT medium at a ratio of 1: 1,000. It was cultured with shaking at 37°C for 16 hours. Bacterial cells were harvested by centrifugation at 4°C, 4,700 rpm for 10 minutes. The bacterial pellet was resuspended in 12 mL of RES-EF buffer. Thereafter, 12 mL of LYS-EF buffer was added, and the sealed tube was inverted vigorously to mix thoroughly and then incubated for 5 minutes at room temperature. 12 mL of NEU-EF buffer was added to the lysate and inverted vigorously to mix thoroughly and rapidly.

Before injecting the lysate into the NucleoBond^{®} Xtra column filter, a homogeneous suspension of the precipitate was prepared by inverting the lysate tube 3 times to prevent clogging of the filter. Thereafter, the NucleoBond^{®} Xtra column filter and the NucleoBond^{®} Xtra column were washed with 10 mL of filter wash buffer FIL-EF. The NucleoBond^{®} Xtra column filter was removed by pulling out or inverting the column. The NucleoBond^{®} Xtra column was washed with 90 mL of wash buffer ENDO.

The NucleoBond^{®} Xtra column was washed with 45 mL of wash buffer WASH-EF. Plasmid DNA was eluted with 15 mL of elution buffer ELU. The eluate was collected in a 50 mL centrifugation tube. 10.5 mL of isopropanol at room temperature was added to precipitate the eluted plasmid DNA. After vortex, the mixture was allowed to stand for 2 minutes.

Thereafter, 5 mL of 70% ethanol was added to the pellet. Ethanol was carefully and completely removed from the tube using a pipette tip. The pellet was dried at room temperature (20°C). Thereafter, the DNA pellet was dissolved with 1,000 µl of H₂O.

### Manufacturing Example 2. Cell transfection and protein expression

### Manufacturing Example 2.1. Cell transfection

Materials and reagents used are described in Table 5 below.

**[Table 5]**

| Material and reagent | Manufacturer (Product #) |
|---|---|
| 293F cells | Invitrogen (R790-07) |
| OPM 293 | OPM (81075-001) |
| Pluronic^{®}F-68, 10% (100X) | Gibco (24040-032) |
| 1 mg/mL PEI | Polyscience (23966) |
| OPTI MEM I | Gibco (31985088) |
| Peptone (20x) | FLUKA (P0521-1KG) |
| Shaker flask | |
| ISF1-X incubator shaker | Kuhner shaker |

The 293F seed strain containing the complete medium was maintained in an incubator shaker at 130 rpm, 37°C, and 8% CO₂. It was cultured at a density of 0.3 to 0.4 × 10⁶ cell/mL, and the medium was changed every 2 to 3 days. Twenty-four hours before transfection, a new passage number of 293F cells was prepared at 2.6 × 10⁶ cell/mL. The prepared cells were cultured in an incubator shaker at 130 rpm, 37°C, and 8% CO₂. On the day of transfection, a density of cells was adjusted to a density of 5.0 × 10⁶ cells/mL using a fresh medium. It was performed at a total volume of 1 L in a 3 L shaker flask. 0.4 mg of HC and 0.6 mg of LC plasmid were diluted with 50 mL of OPTI MEM I and filtered through a 0.22 µm filter. Thereafter, 2 mg of PEI was diluted with 50 mL of OPTI MEM I to prepare a transfection reagent.

The diluted PEI was added to the DNA mixture and then mixed immediately. Thereafter, it was cultured for 15 minutes at room temperature. The DNA-PEI mixture was added to 293F cells prepared at 2.6 × 10⁶ cell/mL. Thereafter, the cells were continuously cultured for 24 hours in an incubator shaker at 130 rpm, 37°C, and 8% CO₂. Twenty-four hours after transfection, 10% peptone was added to 1/20 of the culture solution so that the final concentration was 0.5%. Thereafter, the cells were continuously cultured in an incubator shaker at 130 rpm, 37°C, and 8% CO₂. The cell density/viability was measured and recorded daily during the 2 to 5 days period after transfection. The cells were harvested for purification 7 days after transfection or when the cell viability was less than 70%.

### Manufacturing Example 2.2. Protein purification

Reagents, composition of the buffers, and equipment used for protein purification are described in Tables 6 to 8 below.

**[Table 6]**

| Reagent | Manufacturer | Catalog # |
|---|---|---|
| Mabselect SuRe | GE Healthcare | 11003493 |
| Tris | SIGMA | 77-86-1 |
| NaCl | ACROS ORGANIVS | 7647-14-5 |
| Sodium citrate | Adamas-beta | 76198B |
| Citric acid | GENERAL-Reagent | G83162B |
| Arginine | VETEC | V900343-500G |
| Succinic acid | Sigma-Aldrich | S9512-500G |
| Triton X-100 | ABCONE | X10010-1L |
| Triton X-114 | Sigma-Aldrich | X114 |
| Millex-GP Filter Unit 0.22 µm Sterile | MILLIPORE | SLGP033RS |
| NaOH | Merck | B146369740 |

**[Table 7]**

| | |
|---|---|
| Buffer A | 25 mM Tris, 150 mM NaCl, pH 8.0 |
| Buffer B | 25 mM Tris, 150 mM NaCl, 0.1% Triton X-100, 0.1% Triton X-114 pH 8.0 |
| Buffer C | 100 mM Sodium Citrate, 150 mM NaCl, pH 3.0 |
| Buffer D | 1 M Arginine, 400 mM Succinic acid, pH 9.0 |
| Buffer E | 20 mM PB pH 6.5, 1 M (NH₄)₂SO₄ |
| Buffer F | 20 mM PB pH 6.5, 25% isopropyl alcohol |
| Final buffer | 20 mM HEPES, pH 7.5, 240 mM sucrose or 20 mM his acetate pH 5.5, 240 mM sucrose |

**[Table 8]**

| Equipment | Manufacturer | Model name |
|---|---|---|
| AKTA Pure | GE Healthcare | 29-0182-24 |
| centrifuge | Beckman | J-26xp |
| Gel Imaging System | Tanon | 2500R |
| Sartopore 2 filter | Sartorius | 5445307H9-OO-A |

The proteins were purified using a Mabselect sure column. Specifically, the supernatant was harvested by centrifugation at 2,000×g, 4°C for 20 minutes. Thereafter, the supernatant was filtered with a Sartopore 2 filter. A 5 mL of MabSelect Sure column equilibrated with Buffer A was loaded with the clarified supernatant. Thereafter, the column was washed with Buffer A until the A280 absorbance reached the baseline. The column was washed with 10 CV Buffer B. The column was washed with 10 CV Buffer A. The bound proteins were eluted with 6 CV Buffer C, and 1/6 volume of Buffer D was added to neutralize the eluted substance. SDS-PAGE and SEC-HPLC analyses were performed.

Thereafter, the proteins were purified using a HIC column. The proteins were then dialyzed against Buffer E at 4°C overnight. A HIC column equilibrated with Buffer E was loaded with the supernatant. Thereafter, the column was washed with Buffer E until the A280 absorbance reached the baseline. The bound proteins were eluted by gradient elution (10 CV Buffer F 0%-40%). The bound proteins were eluted with 2 CV 100% Buffer F. SDS-PAGE analysis was performed.

The purified proteins were pooled, and then the proteins were dialyzed against the final buffer at 4°C overnight. Thereafter, SDS-PAGE and SEC-HPLC analyses were performed.

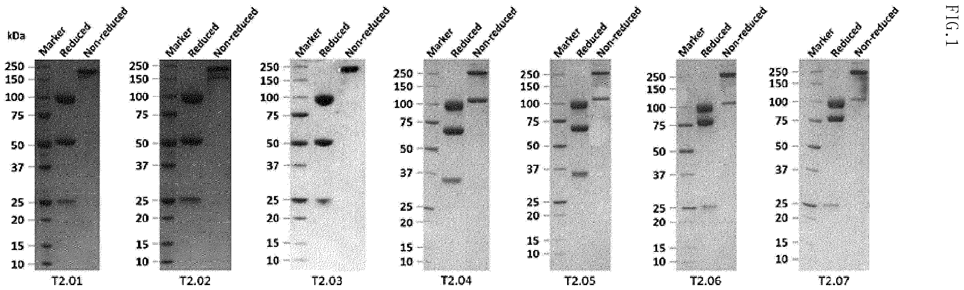

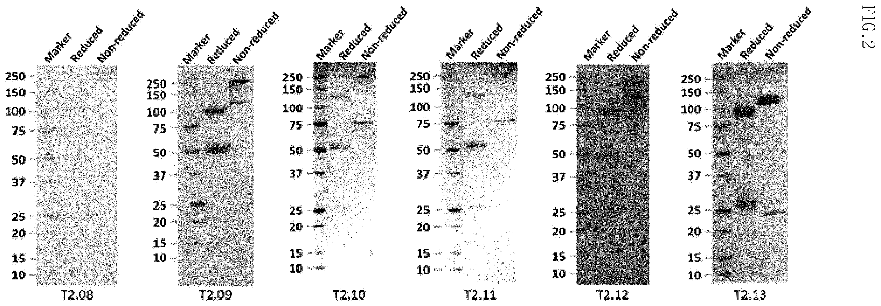

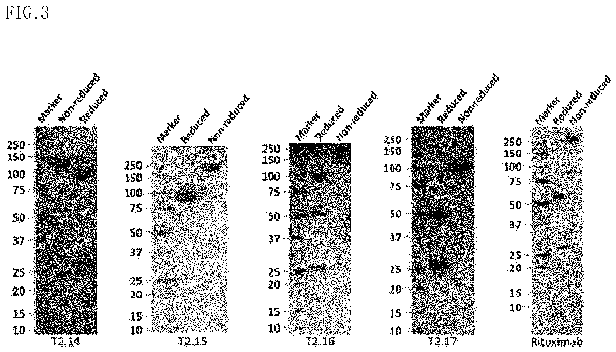

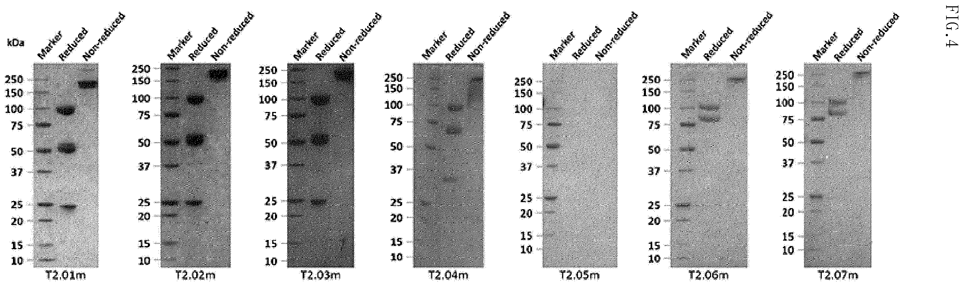

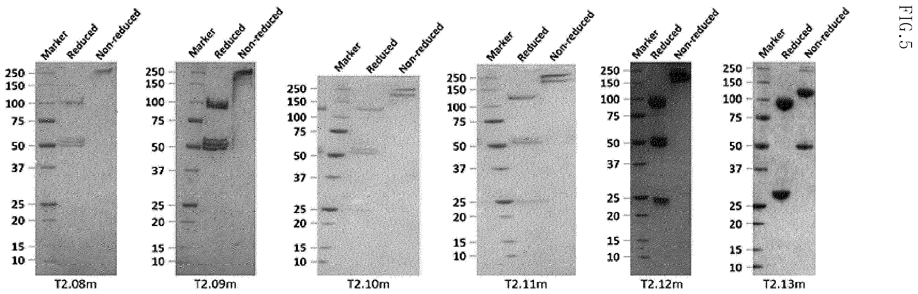

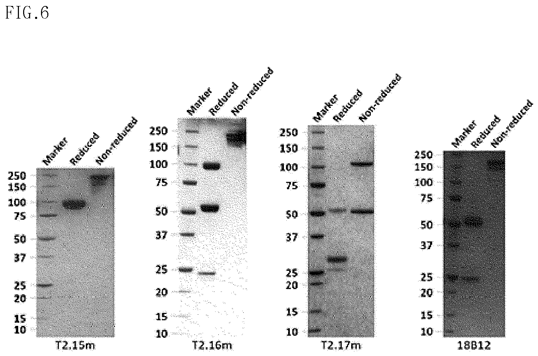

As a result, as shown in FIGs. 1 to 6, it was found that the human proteins and the mouse proteins of one embodiment were purified.

### Manufacturing Example 3. Improvement in production of bispecific antibody

### Manufacturing Example 3.1. Identification of improvement in production of fusion protein comprising human IL-12

Table 9 below shows changes in the production of a human anti-CD20/IL-12 bispecific antibody according to human IL-12 mutation. The parentheses indicate the production scale of the protein, and the unit is L. The number in front of parentheses is the production per liter obtained by dividing the amount of the purified proteins by the production scale.

**[Table 9]**

| PROTEIN | Description | Amount of protein after passing through Protein A column (mg) (production scale, L) |
|---|---|---|
| T2.01 | anti-hu CD20/hu IL-12, hu IgG1 | 208 (1) |
| T2.02 | anti-hu CD20/hu IL-12 mut1, hu IgG1 | 234 (1) |
| T2.03 | anti-hu CD20/hu IL-12 mut2, hu IgG1 | 315 (1) |

As shown in Table 9 above, as the number of mutations in amino acids involved in heparin binding in the human IL-12 p40 (beta) region increased, the amount of proteins purified through a Protein A column (MabSelect Sure column) was enhanced. It was found that the bispecific antibody T2.02 in which lysine (K) at the 280^{th} and 285^{th} amino acids was mutated to alanine (A) had improved production compared to T2.01. It was found that the bispecific antibody T2.03 in which lysine (K) at the 280^{th}, 282^{nd}, 285^{th} and 286^{th} amino acids was mutated to alanine (A) had improved production by about 1.5 times compared to T2.01.

### Manufacturing Example 3.2. Identification of improvement in production of fusion protein comprising mouse IL-12

Table 10 below shows changes in the production of a mouse anti-CD20/IL-12 bispecific antibody according to mouse IL-12 mutation.

**[Table 10]**

| PROTEIN | Description | Amount of protein after passing through Protein A column (mg) (production scale, L) |
|---|---|---|
| T2.01m | anti-mu CD20/mu IL-12, mu IgG2a | 17.1 (1), 36.9 (3), 21.7 (7.5) |
| T2.02m | anti-mu CD20/mu IL-12 mut1, mu IgG2a | 18.9 (1), 40.067 (3) |
| T2.03m | anti-mu CD20/mu IL-12 mut2, mu IgG2a | 32.8 (1), 114.4 (1), 96.7 (8) |

As shown in Table 10 above, as the number of mutations in amino acids involved in heparin binding in the mouse IL-12 p40 (beta) region increased, the amount of proteins purified through a Protein A column (MabSelect Sure column) was increased.

In each table, the amount of protein after passing through Protein A column was compared by dividing the total protein production by the production scale. It was found that the bispecific antibody T2.02m in which lysine (K) at the 277^{th} and 282^{nd} amino acids was mutated to alanine (A) had improved production compared to T2.01m. In addition, it was found that the bispecific antibody T2.03m in which arginine (R) at the 276^{th} amino acid was mutated to alanine (A), and lysine (K) at the 277^{th}, 278^{th} and 282^{nd} amino acids was mutated to alanine (A) had improved production in proteins compared to T2.01m and T2.02m. In addition, it was found that the production of T2.03m using the CHO cell line was improved by about 3 times compared to the production using the HEK293 cell line.

### Manufacturing Example 4. Cell line and culture of cell line

The human B-cell lymphoma cell line Raji, the human B-cell lymphoma cell line Ramos, the human T-lineage cell line Jurkat, and the mouse B-cell lymphoma cell line A20 were obtained from ATCC (American Type Culture Collection, Manassas, VA, USA). The Raji cells, the Ramos cells and the Jurkat cells were maintained in RPMI-1640 (GIBCO, Grand Island, NY, USA) containing 10% FBS (GIBCO). The A20 cells were maintained in RPMI-1640 (GIBCO) containing 10% FBS (GIBCO) and 0.05 mM 2-mercaptoethanol.

The HEK-Blue IL-12 cell line transformed with the IL-12 receptor gene and the STAT4 inducible SEAP reporter gene into HEK293 cells, which are human embryonic kidney fibroblasts, was obtained from Invivogen (San Diego, USA).

The HEK-Blue IL-12 cells were maintained in DMEM (GIBCO) containing 10% FBS (GIBCO), 100 µg/mL Normocin and 1xHEK-Blue selection.

### Manufacturing Example 5. Isolation and activation of human immune cells

A blood pack was obtained from the Korean Red Cross, Korea, with the approval of the Institutional Review Board (IRB), and peripheral blood mononuclear cells (PBMCs) were isolated and frozen. The thawed PBMCs were subjected to the positive selection method, and human mononuclear cells were isolated with an Easy sep (Stem cell, Vancouver, BC, CA) kit. The isolated mononuclear cells were differentiated into immature dendritic cells for 5 days by a dendritic cell culture kit (Stem cell) method. The immature dendritic cells were matured for 5 days by a dendritic cell culture kit (Stem cell) method.

The thawed PBMCs were subjected to the negative selection method, and human T cells were isolated with an Easy sep (Stem cell) kit. The human T cells were cultured on a plate coated with 1 µg/mL anti-CD3 (OKT3, Invitrogen) and activated for 72 hours. The human T cells were maintained in RPMI-1640 (GIBCO) containing 10% FBS (GIBCO).

### Example 1. Identification of binding affinity of fusion protein

### Example 1.1. Identification of binding of fusion protein to recombinant human CD20

Binding of T2.01 and T2.02 to recombinant human CD20 was identified by surface plasmon resonance (SPR).

Specifically, the surface of the CMS chip was activated with a 1:1 mixture of 50 nM NHS (N-hydroxysuccinimide) and 200 nM EDC (1-ethyl-3-(3-dimethylaminopropyl)carbodiimide), and 25 µg/mL of anti-human IgG (Fc) antibody was immobilized for 400 seconds at a rate of 10 µl/min. The remaining active ester groups were blocked with 1 M ethanolamine. T2.01 and T2.02 were diluted to 2 µg/mL and reacted on the CMS chip immobilized with the anti-human IgG (Fc) antibody. The recombinant human CD20 was diluted to 200 nM in 1xHBS-EP + buffer solution and diluted by serial dilution. The diluted recombinant human CD20 was reacted at a rate of 30 µl/min. The association and dissociation times were 180 seconds and 400 seconds, respectively. After the dissociation, stabilization was performed for 60 seconds, and then a regeneration was performed for 30 seconds with a 10 mM glycine pH 1.5 solution at a rate of 30 µl/min.

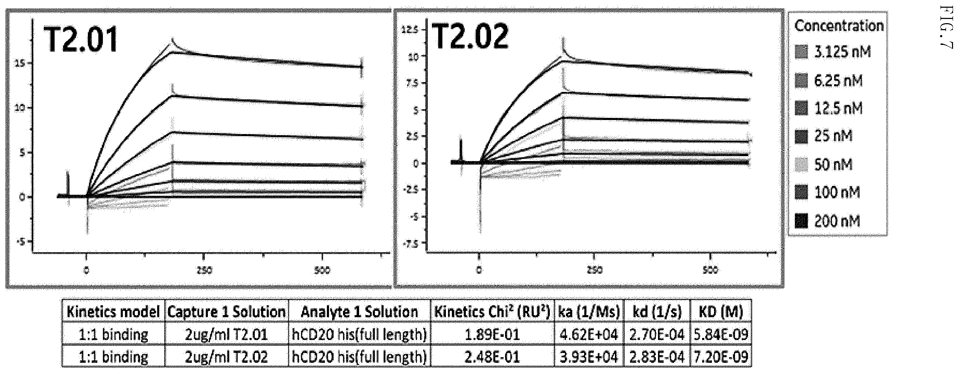

As a result, as shown in FIG. 7, it was found that T2.01 and T2.02 can specifically bind to recombinant human CD20.

In addition, binding of T2.04, T2.05, T2.06, T2.07, T2.08, T2.09, T2.10 and T2.11 to recombinant human CD20 was identified by surface plasmon resonance. T2.04, T2.05, T2.06, T2.07, T2.08, T2.09, T2.10 and T2.11 were diluted to 15 µg/mL, 2 µg/mL, 2 µg/mL, 2 µg/mL, 10 µg/mL, 2 µg/mL, 20 µg/mL, and 20 µg/mL, respectively, and reacted on the chip immobilized with the protein A. The recombinant human CD20 was diluted to 100 nM or 200 nM in 1xHBS-EP+, 0.05% DDM (detergent dodecylmatoside), 0.01% CHS (cholesteryl hemisuccinate) buffer solution and diluted by serial dilution. The diluted recombinant human CD20 was reacted at a rate of 30 µl/min. The association and dissociation times were 180 seconds and 400 seconds, respectively. After the dissociation, stabilization was performed for 60 seconds, and then a regeneration was performed for 30 seconds with a 10 mM glycine pH 1.5 solution at a rate of 30 µl/min.

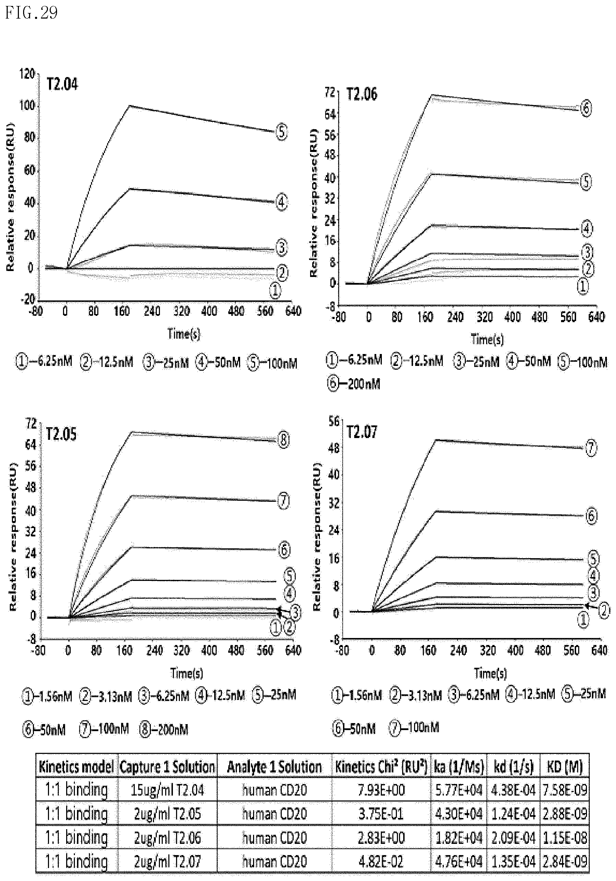

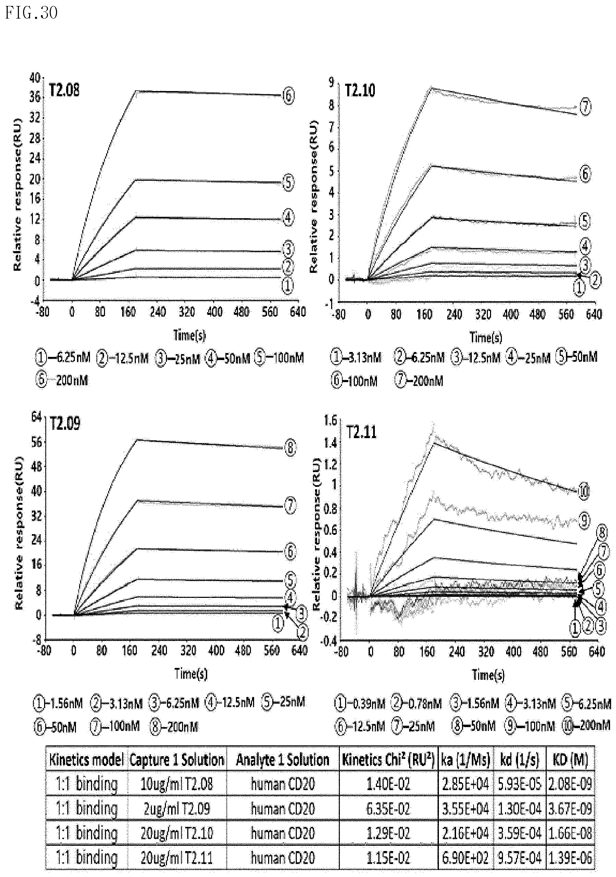

As a result, as shown in FIGs. 29 to 30, it was found that T2.04, T2.05, T2.06, T2.07, T2.08, T2.09, T2.10 and T2.11 can bind to recombinant human CD20 to specifically target CD20.

### Example 1.2. Identification of binding to CD20-expressing cells

It was identified whether T2.01 and T2.02 bind to the CD20-expressing cell lines, Raji and Ramos.

Specifically, the degrees of binding of T2.01 and T2.02 to the Raji, Ramos and Jurkat cells were identified. The Jurkat T cells that do not express CD20 were used as a control.

The anti-CD20 monoclonal antibody (Rituximab, Roche) is a drug approved by the Food and Drug Administration (FDA), which is known to be able to bind specifically to CD20.

The cell line was prepared by suspending in a FACS buffer solution at 1 × 10⁵ cells/100 µl, and treated with 1 µg each of Rituximab, T2.01 and T2.02. The cells were washed twice with a FACS buffer solution. The cells were stained with an anti-human IgG antibody (Biolegend). The negative control sample was stained only with an anti-human IgG Fc antibody (Biolegend). The expression rates of the stained cells were measured using BD LSR and analyzed using a FlowJo software.

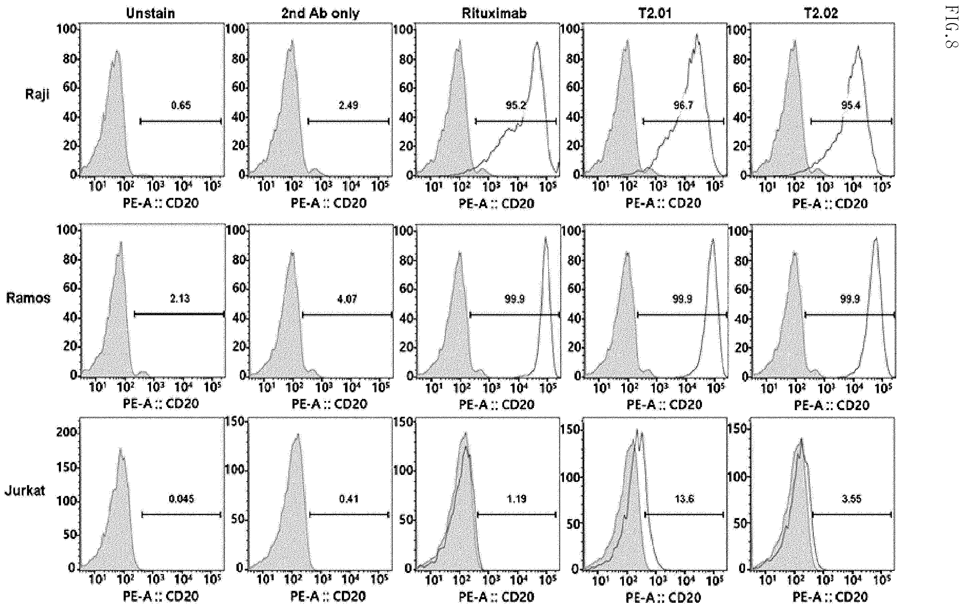

As a result, as shown in FIG. 8, it was found that T2.01 and T2.02 bind to the CD20-expressing cell line by 95% or more.

Next, the degrees of binding of the mouse surrogate antibody protein, 18B12, T2.01m and T2.02m to A20 cells were identified.

The cell line was prepared by suspending in a FACS buffer solution at 1 × 10⁵ cells/100 µl, and treated with 1 µg each of 18B12, T2.01m and T2.02m. The cells were washed twice with a FACS buffer solution. The cells were stained with an anti-mouse IgG2a antibody (Biolegend). The negative control sample was stained only with an anti-mouse IgG2a antibody (Biolegend). The expression rates of the stained cells were measured using BD LSR and analyzed using a FlowJo software.

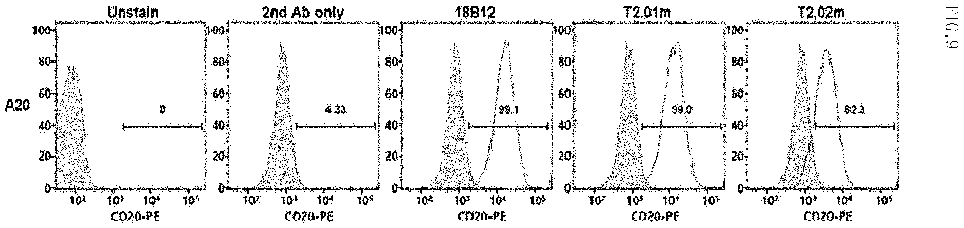

As a result, as shown in FIG. 9, it was found that T2.01m and T2.02m bind to the CD20-expressing cell line by 85% or more.

### Example 1.3. Identification of binding of fusion protein to recombinant human IL-12 receptor

Considering that IL-12 of the fusion protein must bind to the IL-12 receptor for the action, it was identified that T2.01 and T2.02 bind to the recombinant human IL-12 receptor.

Rituximab, T2.01 and T2.02 were immobilized on a plate, and a recombinant human IL-12 receptor protein conjugated with horseradish peroxidase (HRP) was bound. Then, Rituximab was used as a control.

Specifically, it was found that the fusion protein according to the present invention binds to the recombinant human IL-12 receptor, as follows:
(i) Rituximab, T2.01 and T2.02 were suspended at 100 nM and diluted by serial dilution. The diluted Rituximab, T2.01 and T2.02 were aliquoted in a 96-well immune plate and treated for 24 hours.
(ii) The wells were washed with a wash solution, and then blocking was performed for 1 hour with 1% BSA (bovine serum albumin, Sigma) solution.
(iii) The wells were washed with a wash solution, and then treated with 1 nM IL-12 receptor beta 1-biotin (IL-12Rβ1-Biotin; Acrobiosystems, Newark, USA) for 2 hours.
(iv) The wells were washed with a wash solution, and then treated with streptavidin-HRP for 20 minutes.
(v) The wells were washed with a wash solution, and then treated with a substrate solution for 20 minutes.
(vi) The wells were treated with a stop solution, and the absorbance was measured at 450 nm.

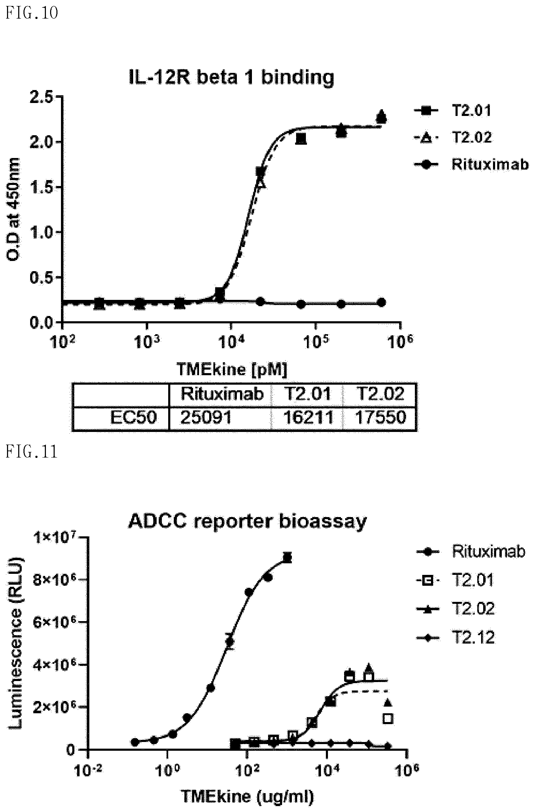

As a result, as shown in FIG. 10, it was found that T2.01 and T2.02 bind to the recombinant human IL-12 receptor protein in a concentration dependent manner.

### Example 1.4. Identification of antibody-dependent cellular cytotoxicity of fusion protein

The anti-CD20 monoclonal antibody binds to B-cell lymphoma and kills cells through antibody-dependent cellular cytotoxicity. Therefore, the antibody-dependent cellular cytotoxicity of the CD20-targeting monovalent fusion protein against B-cell lymphoma (Raji) were identified.

Specifically, the antibody-dependent cellular cytotoxicity (ADCC) of Rituximab, T2.01, T2.02 and T2.12 was identified using an ADCC Reporter Bioassay (Promega, Wisconsin, USA) kit as follows.
(i) The target cells (Raji) were suspended in RPMI-1640 containing 4% low IgG serum at a concentration of 5 × 10⁵ cells/mL, and each 25 µl was aliquoted.
(ii) Rituximab, T2.01, T2.02 and T2.12 were suspended in RPMI-1640 containing 4% low IgG serum at 250 µg/mL and diluted by serial dilution. The diluted Rituximab, T2.01, T2.02 and T2.12 were aliquoted by 25 µl each.
(iii) The effector cells were suspended in RPMI-1640 containing 4% low IgG serum at a concentration of 3 × 10⁶ cells/mL, and each 25 µl was aliquoted.
(iv) After culturing the cells for 6 hours, the cells were treated with BioGio^{™} luciferase assay reagent (Promega), and luminescence value (relative luminescence unit (RLU)) was measured.

As a result, as shown in FIG. 11, the sample treated with the anti-CD20 monoclonal antibody Rituximab showed a high antibody-dependent cellular cytotoxicity with a 50% effective concentration (EC50) of 31.1 µg/mL, while the sample treated with T2.01 showed a low antibody-dependent cellular cytotoxicity with an EC50 of 5.3 × 10³ µg/mL, and the sample treated with T2.02 showed a low antibody-dependent cellular cytotoxicity with an EC50 of 6.7 × 10³ µg/mL. The sample treated with the control T2.12 having no effector function due to the mutation of the Fc portion showed no antibody-dependent cellular cytotoxicity.

### Example 2. Identification of ability of fusion protein to induce signaling

### Example 2.1. Identification of signaling ability in IL-12 recognizing cells

Considering that IL-12 is known to initiate signaling by inducing phosphorylation of STAT4 (signal transducer and activator or transcription 4), a signaling mediator, when IL-12 binds to the IL-12 receptor, the signaling ability of the fusion protein of one embodiment in IL-12 recognizing cells that express the IL-12 receptor was evaluated.

Specifically, the HEK-Blue IL-12 cell line, which is an IL-12 recognizing cell, was diluted to 2.8 × 10⁵ cells/mL and dispensed at 180 µl/well. Recombinant human IL-12, T2.01m, T2.02m, T2.03m and 18B12 were suspended at 4 nM and diluted by serial dilution. The HEK-Blue IL-12 cells were treated with the diluted recombinant human IL-12, T2.01m, T2.02m, T2.03m and 18B12. After 24 hours of treatment, the cell supernatant was collected and mixed with QUANTI-Blue Solution (Invivogen) in a 96-well plate. The reporter expression level was measured at 620 nm using a spectrophotometer (Thermo Fisher).

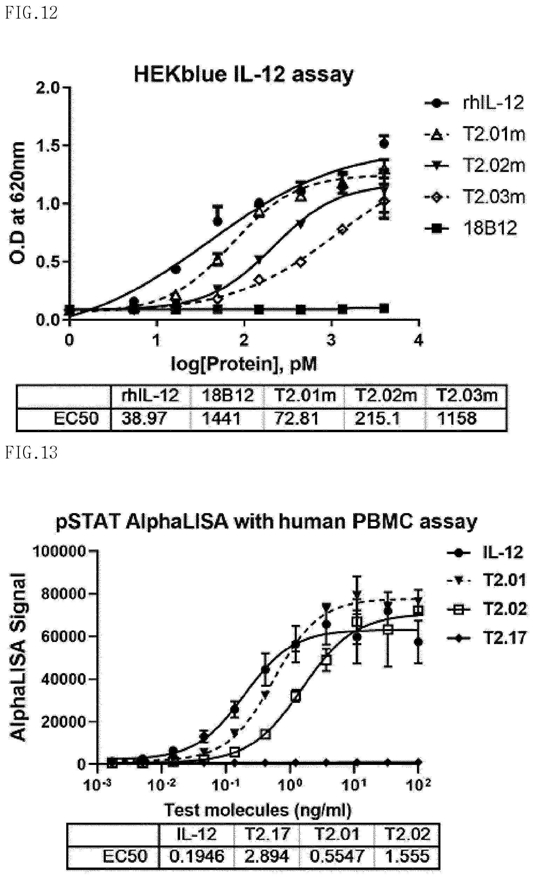

As a result, as shown in FIG. 12, it was identified by 620 nm absorbance that in the case of recombinant human IL-12, T2.01m, T2.02m and T2.03m, IL-12 was recognized, and STAT4 expressed a reporter gene by phosphorylation. In addition, it was identified that 18B12 did not recognize IL-12 when IL-12 recognizing cells were treated with 18B12. These results indicate that the fusion protein of one embodiment specifically binds to the IL-12 receptor to induce signaling.

### Example 2.2. Identification of signaling ability in human T cells

It was identified whether when IL-12 binds to the IL-12 receptor of human T cells, IL-12 initiates signaling by inducing phosphorylation of STAT4, a signaling mediator.

Specifically, the signaling ability in the human T cells was identified by analyzing the human T cells activated with anti-CD3 (OKT3, Invitrogen) using the AlphaLISA SureFire Ultra p-STAT4 (Tyr693) Assay Kit (PerkinElmer, Massachusetts, USA). The recombinant human IL-12, T2.01, T2.02 and T2.17 were suspended at 100 ng/mL and diluted by serial dilution. The activated human T cells were treated with the diluted recombinant human IL-12, T2.01, T2.02 and T2.17. After 2 hours of treatment, the cells were lysed and treated with a reagent (PerkinElmer) that detects p-STAT4 (phosphorylated STAT4). p-STAT4 was detected at an excitation wavelength of 680 nm and an emission wavelength of 615 nm with a spectrophotometer.

As a result, as shown in FIG. 13, the sample treated with the recombinant human IL-12 showed a 50% effective concentration (EC50) of 0.1946 ng/mL, and the sample treated with T2.01 showed an EC50 of 0.5547 ng/mL, and the sample treated with T2.02 showed an EC50 of 1.555 ng/mL. Through EC50 comparison, it was found that the T cell signaling ability was attenuated due to the IL-12 mutation. The phosphorylated STAT4 was not identified in the sample treated with T2.17. These results indicate that the IL-12 mutation attenuated the T cell signaling ability.

### Example 3. Identification of cytokine secretion ability of fusion protein

### Example 3.1. Enzyme-linked immunosorbent assay for cytokine measurement

Enzyme-linked immunosorbent assay (ELISA, R&D systems) for cytokine measurement was performed on the supernatant stored at -20°C in the following steps:
(i) The capture antibody was diluted according to the Certificate of Analysis (CoA) and coated on a 96-well plate for 24 hours.
(ii) The wells were washed with a wash solution, and then blocking was performed for 1 hour with 1% BSA (Bovine serum albumin, Sigma) solution.
(iii) The wells were washed with a wash solution, and then the samples were treated for 2 hours.
(iv) The wells were washed with a wash solution, and then the detection antibody was diluted according to the Certificate of Analysis and aliquoted into the wells, and then the wells were treated therewith for 2 hours.
(v) The wells were washed with a wash solution, and then treated with streptavidin-HRP for 20 minutes.
(vi) The wells were washed with a wash solution, and then treated with a substrate solution for 20 minutes.
(vii) The wells were treated with a stop solution, and the absorbance was measured at 450 nm.

### Example 3.2. Identification of cytokine secretion ability in human T cells

Considering that IFN-γ (interferon-gamma) secretion, which is a major immune response, occurs when human T cells are treated with IL-12, IFN-γ secretion ability in the cell supernatant when human T cells were treated with recombinant human IL-12, Rituximab, T2.01, T2.02 and T2.03 was evaluated through enzyme-linked immunosorbent assay.

Specifically, the human T cells activated with anti-CD3 (OKT3, Invitrogen) were diluted to 5 × 10⁵ cells/mL and dispensed in a 96-well plate at 200 µl/well. Recombinant human IL-12, Rituximab, T2.01, T2.02 and T2.03 were diluted to 10 nM and dispensed at 20 µl/well. It was treated with 250 pM each of recombinant human IL-12, Rituximab, T2.01, T2.02 and T2.03. After 48 hours of treatment, the cell supernatant was collected, and the samples were stored at -80°C.

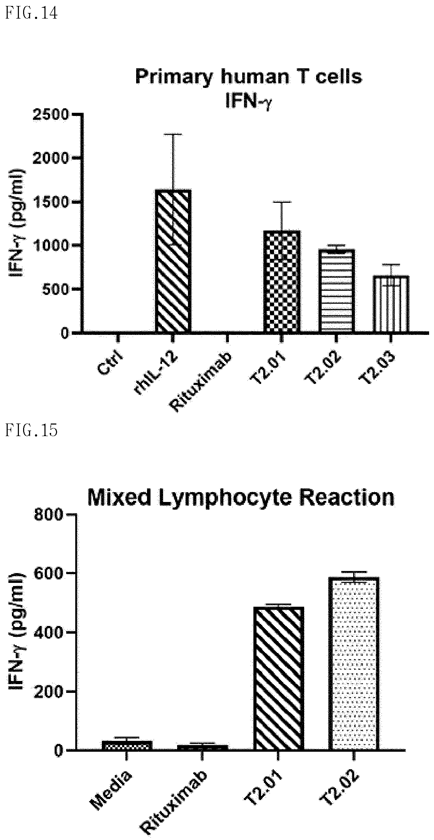

As a result, as shown in FIG. 14, it was found that the sample treated with the recombinant human IL-12 had an average of 1,640 pg/mL of IFN-γ, the sample treated with T2.01 had an average of 1,170 pg/mL of IFN-γ, the sample treated with T2.02 had an average of 956 ng/mL of IFN-γ, the sample treated with T2.03 had an average of 662 pg/mL of IFN-γ. It was found that the sample treated with Rituximab had no IFN-γ. These results indicate that T2.01, T2.02 and T2.03 act on human T cells to induce IFN-γ secretion.

### Example 3.3. Identification of cytokine secretion ability through mixed lymphocyte reaction test

It was identified whether the fusion protein induces cytokine secretion when an artificial syngeneic immune response is induced by mixing human T cells and syngeneic dendritic cells. IFN-γ concentration in the cell supernatant was evaluated through enzyme-linked immunosorbent assay.

Specifically, the mature human dendritic cells were diluted to 4 × 10⁵ cells/mL and dispensed in a 96-well plate at 50 µl/well. The human T cells from different blood donors activated with anti-CD3 antibody were diluted to 2 × 10⁶ cells/mL, dispensed at 100 µl/well, and mixed with the dispensed mature human dendritic cells (Mixed Lymphocyte Reaction, MLR). Rituximab, T2.01 and T2.02 were diluted to 1 µM and dispensed at 50 µl/well. After 5 days of mixed culture, the cell supernatant was collected, and the samples were stored at -80°C.

As a result, as shown in FIG. 15, it was found that the sample treated with T2.01 had an average of 487 pg/mL of IFN-γ, and the sample treated with T2.02 had an average of 587 pg/mL of IFN-γ. In contrast, it was found that the sample treated with Rituximab had no IFN-γ. These results indicate that T2.01 and T2.02 induce IFN-γ secretion during mixed lymphocyte immune response.

### Example 4. Evaluation of anticancer efficacy in syngeneic mouse model

### Example 4.1. Establishment of syngeneic mouse model and evaluation of anticancer efficacy

It was performed *in vivo* with the approval of the Institutional Animal Care and Use Committee (IACUC, Crownbio, U.S). 6-week-old female BALB/c mice were obtained from Shanghai Lingchang Biotechnology Co., Ltd (Shanghai, China). The A20 cells were suspended in PBS (phosphate-buffered saline, GIBCO) at a concentration of 5 × 10⁵ cells/100 µl, and then 5 × 10⁵ cells were subcutaneously injected into the right flank of the mouse.

When the size of the tumor reached 100 mm³, the mice were randomly divided into groups. 100 µg of T2.01m, 50 µg of T2.02m, 100 µg of T2.02m and 300 µg of T2.02m were intraperitoneally injected three times at an interval of 3 days. In the chemotherapy group, CHOP1 (100 mg/kg cyclophosphamide, 6 mg/kg doxolubicin, 0.1 mg/kg vincristine, 0.2 mg/kg dexamethasone) was intraperitoneally injected once, and CHOP2 (50 mg/kg cyclophosphamide, 3 mg/kg doxolubicin, 0.1 mg/kg vincristine, 0.2 mg/kg dexamethasone) was intraperitoneally injected twice at an interval of 1 week. 10 mice per group were used. The tumor size and body weight were measured twice a week. The anticancer activity was evaluated for two months.

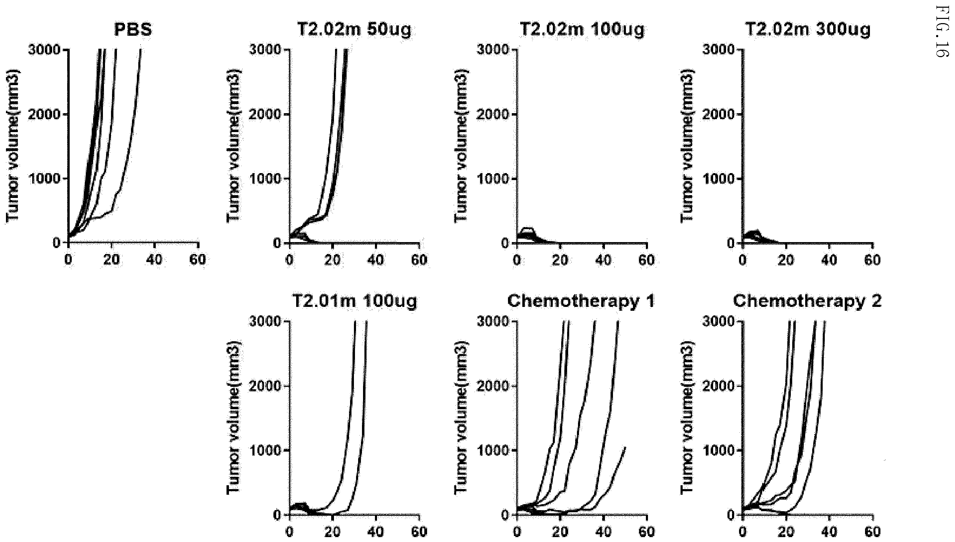

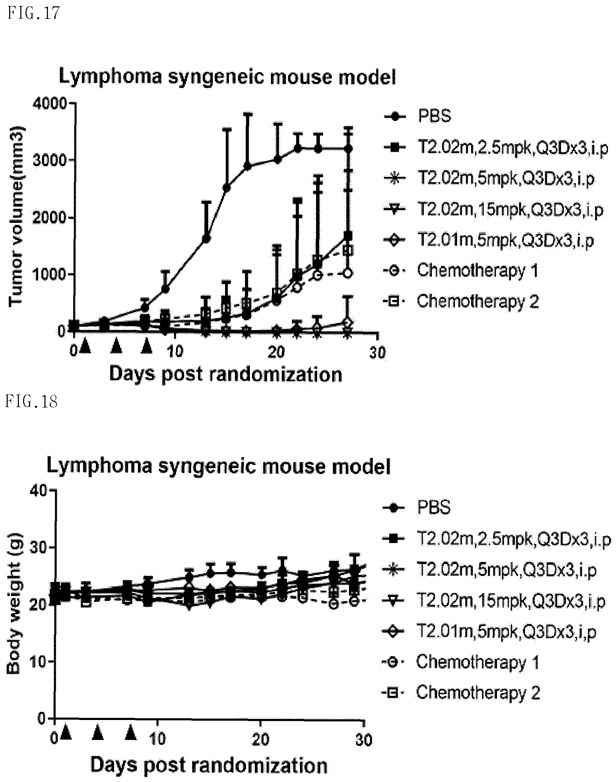

As a result, as shown in FIGs. 16 and 17, on day 20 after the first intraperitoneal injection, it was found that the group treated with T2.02m at 50 µg/mL showed a complete response (CR) of 43% (3/7), the group treated with T2.02m at 100 µg/mL showed a CR of 100% (7/7), and the group treated with T2.02m at 300 µg/mL showed a CR of 100% (7/7), indicating that T2.02m had an excellent anticancer effect. It was found that the group treated with T2.01m at 100 µg/mL showed a CR of 86% (6/7). On the other hand, it was found that the group treated with the chemotherapy CHOP1 showed a CR of 28.5% (2/7), and the group treated with the chemotherapy CHOP2 showed no CR.

In addition, T2.01m, T2.02m, CHOP1 and CHOP2 were intraperitoneally injected, and the resulting side effects were expressed as a change in body weight.

As a result, as shown in FIG. 18, no change in body weight due to intraperitoneal injection of T2.01m, T2.02m, CHOP1 and CHOP2 was observed. These results indicate that there were no side effects after intraperitoneal injection of the fusion protein of one embodiment.

Next, in order to compare the anticancer effect of the chemotherapy group and the fusion protein of one embodiment, on day 9 after intraperitoneal injection of T2.01m, T2.02m, CHOP1 and CHOP2, tumor tissues were separated from 3 animals for each group and relative tumor tissue weights for each group were compared.

Specifically, in the experimental group, tumor tissues were harvested from 3 animals for each group on day 9 after the first intraperitoneal injection through a treatment method according to IACUC regulations. The harvested tumor tissues were weighed on an electronic balance. The immune cells were isolated from the tumor tissues using a tumor dissociation (Miltenyi, Germany) kit. The cells were stained using anti-mouse CD45 antibody (Biolegend), anti-mouse CD3 antibody (BD), anti-mouse CD4 antibody (Biolegend), anti-mouse CD8 antibody (eBioscience), anti-mouse CD19 antibody (Biolegend), anti-mouse CD49/b (Biolegend), Live/Dead (eBioscience) and Counting beads (eBioscience). The expression rates of the stained cells were measured using BD LSR and analyzed using a FlowJo software.

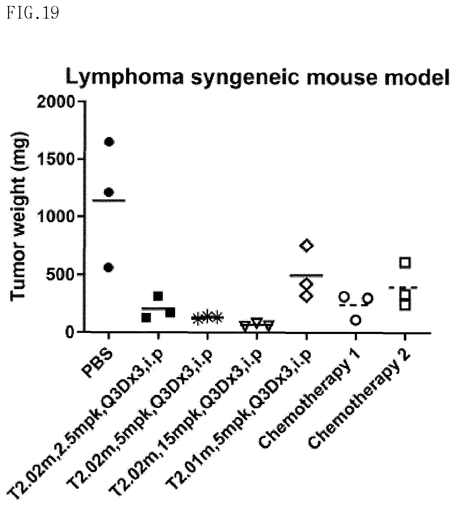

As a result, as shown in FIG. 19, it was found that the tumor tissue weight decreased as the dose of T2.02m injected intraperitoneally increased. These results indicate that the anticancer effect is excellent in proportion to the dose of T2.02m.

In addition, it was identified through analysis of the distribution of immune cells in the tumor tissue whether the above effect was an anticancer effect due to the activation of immune cells by the fusion protein.

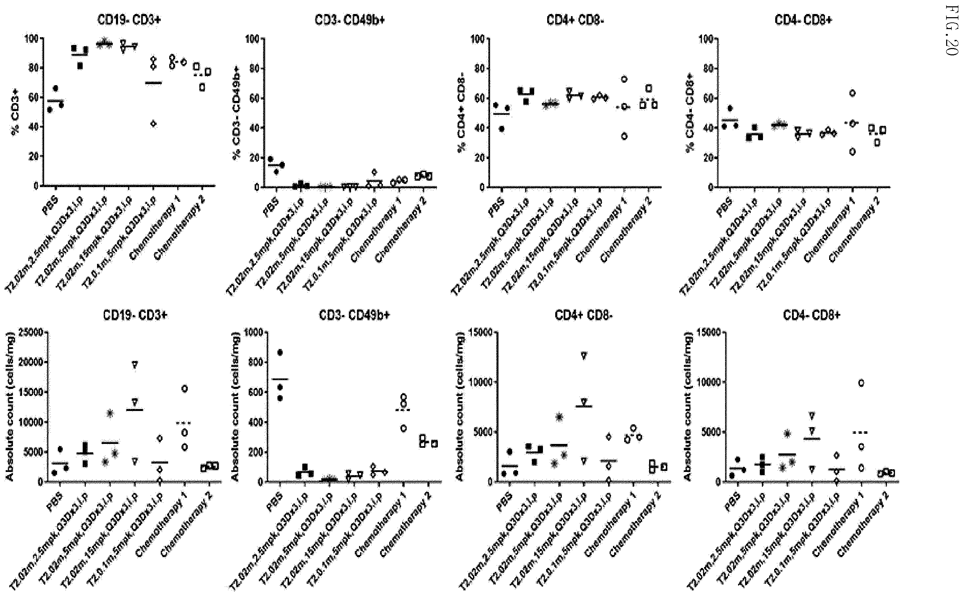

As a result, as shown in FIG. 20, when the group treated with T2.02m was compared with the control group, the chemotherapy CHOP1 group and the chemotherapy CHOP2 group, it was found that there was no difference in the distribution of CD3+ CD4+ T cells or CD3+ CD8+ T cells, but there was a difference in the absolute number of immune cells. These results indicate that the above results are anticancer effects due to the increase in penetration of effector cells by T2.02m.

### Example 4.2. Evaluation of inhibition of tumor rechallenge in tumor rechallenge model

Tumor rechallenge was performed using mice in which complete response was achieved.

A tumor rechallenge model was induced for mice in which complete response (CR) was achieved by treating the syngeneic mice transplanted with the A20 cells with T2.02m.

Specifically, the A20 cells were suspended in PBS (phosphate-buffered saline, GIBCO) at a concentration of 5 × 10⁵ cells/100 µl, and 5 × 10⁵ cells were subcutaneously injected into the right flank of the mouse. The 4T1 cells were suspended in PBS (phosphate-buffered saline, GIBCO) at a concentration of 5 × 10⁵ cells/100 µl, and 5 × 10⁵ cells were subcutaneously injected into the left flank of the mouse. As a control group, untreated mice of the same age as those of the experimental group were used. As a control group, mice of the same age as those of the experimental group were used. 5 mice per group were used. The tumor size and body weight were measured twice a week.

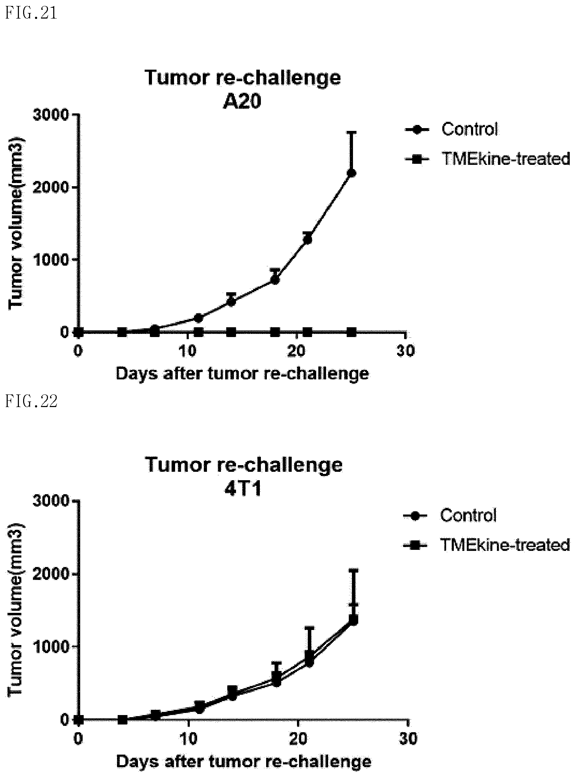

As a result, as shown in FIGs. 21 and 22, it was found that the 4T1 tumor grew but the A20 tumor did not grow in the group in which complete response was achieved by T2.02m treatment. On the other hand, it was found that both the 4T1 tumor and the A20 tumor grew in the control group. These results indicate that tumor rechallenge can be inhibited by the fusion protein.

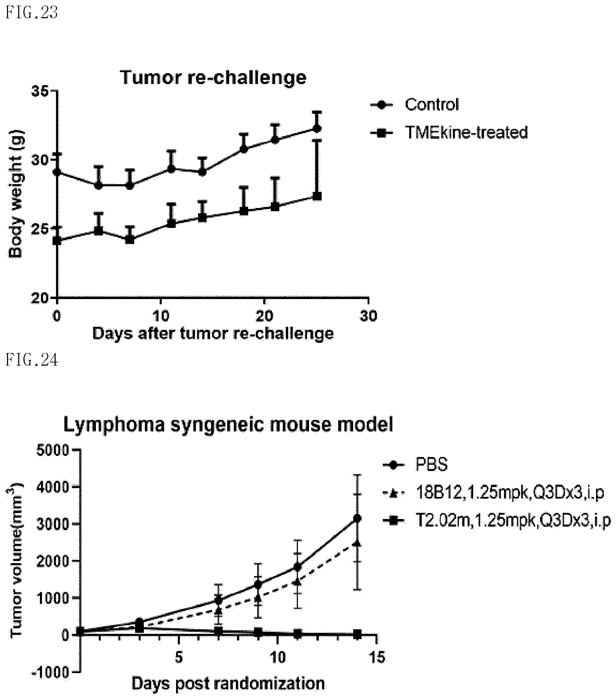

In addition, the body weight of the complete response model was measured to identify whether there were any side effects. As a result, as shown in FIG. 23, it was found that the tumor rechallenge by the fusion protein appeared without side effects.

### Example 4.3. Comparison of evaluation of antitumor activity of CD20-specific fusion protein and anti-CD20 antibody in syngeneic mouse model

The antitumor effect of the CD20-specific fusion protein according to one embodiment was compared with that of the non-specific fusion protein.

Specifically, it was performed *in vivo* with the approval of the Institutional Animal Care and Use Committee (IACUC, Crownbio, U.S). 6-week-old female BALB/c mice were obtained from Shanghai Lingchang Biotechnology Co., Ltd (Shanghai, China). The A20 cells were suspended in PBS (phosphate-buffered saline, GIBCO) at a concentration of 5 × 10⁵ cells/100 µl, and 5 × 10⁵ cells were subcutaneously injected into the right flank of the mouse.

When the size of the tumor reached 100 mm³, the mice were randomly divided into groups. 25 µg of the prepared T2.02m and 25 µg of 18B 12 were intraperitoneally injected three times at an interval of 3 days. 8 mice per group were used. The tumor size and body weight were measured twice a week, and the anticancer activity was evaluated for one month.

As a result, as shown in FIG. 24, it was found that on day 11 after the first intraperitoneal injection, the group treated with T2.02m showed a complete response (CR), and on day 18 after the first intraperitoneal injection, the group showed a CR of 100% (8/8), indicating that T2.02m had an excellent anticancer effect. On the other hand, in the case of the group treated with the non-fusion protein 18B 12, the anticancer effect could not be identified. These results indicate that the anticancer effect of the fusion protein of one embodiment is superior to that of the non-fusion protein.

### Example 4.4. Evaluation of target-specific anticancer efficacy of CD20-specific fusion protein in syngeneic mouse model

The antitumor effect of the target-specific fusion protein according to one embodiment was compared with that of the non-specific non-fusion protein.

It was performed *in vivo* with the approval of the Institutional Animal Care and Use Committee (IACUC, Crownbio, U.S). 6-week-old female BALB/c mice were obtained from Shanghai Lingchang Biotechnology Co., Ltd (Shanghai, China). The A20 cells were suspended in PBS (phosphate-buffered saline, GIBCO) at a concentration of 5 × 10⁵ cells/100 µl, and 5 × 10⁵ cells were subcutaneously injected into the right flank of the mouse.

When the size of the tumor reached 100 mm³, the mice were randomly divided into groups. 0.2 µg of the prepared T2.02m and 0.2 µg of T2.13m were intravenously injected twice at an interval of one week. 7 mice per group were used. The tumor size and body weight were measured twice a week.

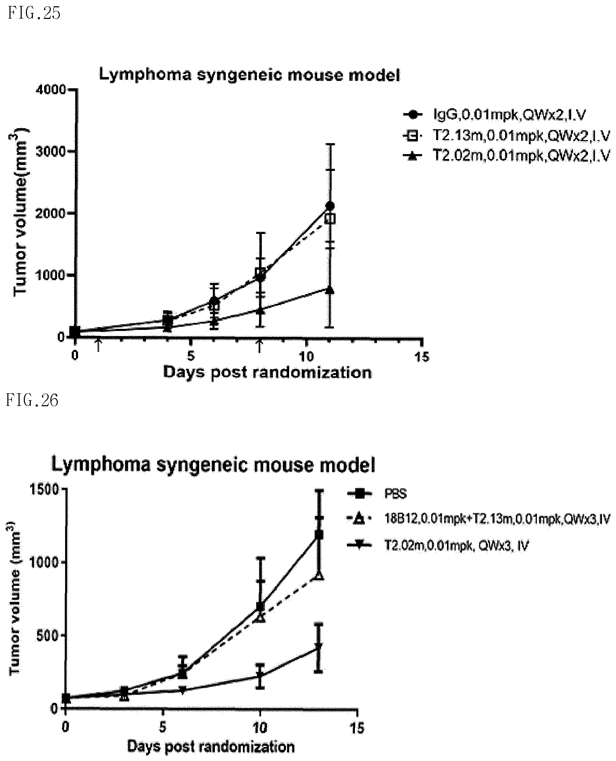

As a result, as shown in FIG. 25, it was found that on day 11 after the first intravenous injection, the group treated with T2.02m showed a tumor growth inhibition percent (TGI) of 63%, while the group treated with T2.13m showed a tumor growth inhibition percent of 20%. These results indicate that the anticancer effect of the target-specific fusion protein of one embodiment is superior to that of the non-target-specific non-fusion protein.

### Example 4.5. Comparison of evaluation of anticancer efficacy of coadministration of CD20-specific fusion protein and anti-CD20 antibody and cytokine in syngeneic mouse model

The effects of the combination therapy of the target-specific fusion protein of one embodiment and the non-specific fusion protein were compared.

Specifically, it was performed *in vivo* with the approval of the Institutional Animal Care and Use Committee (IACUC, Crownbio, U.S). 6-week-old female BALB/c mice were obtained from Shanghai Lingchang Biotechnology Co., Ltd (Shanghai, China). The A20 cells were suspended in PBS (phosphate-buffered saline, GIBCO) at a concentration of 5 × 10⁵ cells/100 µl, and 5 × 10⁵ cells were subcutaneously injected into the right flank of the mouse.

When the size of the tumor reached 100 mm³, the mice were randomly divided into groups. The mice were divided into the group treated with 0.2 µg of the prepared T2.02m; and the group treated with 0.2 µg of T2.13m and 0.2 µg of 18B12. Each dose was intravenously injected three times at an interval of one week and evaluated for two weeks. 7 mice per group were used. The tumor size and body weight were measured twice a week.

As a result, as shown in FIG. 26, it was found that on day 10 after the first intravenous injection, the group treated with T2.02m showed a tumor growth inhibition percent (TGI) of 80%, and the group treated with 18B12 and T2.13m showed a tumor growth inhibition percent of 65%. These results indicate that the anticancer effect of the target-specific fusion protein is superior to that of the combination therapy of the non-target-specific non-fusion proteins, and the synergistic effect of the fusion protein structure of one embodiment is identified.

### Example 4.6. Comparison of evaluation of anticancer efficacy of attenuated cytokine of CD20-specific fusion protein in syngeneic mouse model

It was identified whether the effect of IL-12 in T2.02m and T2.03m comprising heparin binding site mutations was attenuated compared to T2.01m.

It was performed *in vivo* with the approval of the Institutional Animal Care and Use Committee (IACUC, Crownbio, U.S). 6-week-old female BALB/c mice were obtained from Shanghai Lingchang Biotechnology Co., Ltd (Shanghai, China). The A20 cells were suspended in PBS (phosphate-buffered saline, GIBCO) at a concentration of 5 × 10⁵ cells/100 µl, and 5 × 10⁵ cells were subcutaneously injected into the right flank of the mouse.

When the size of the tumor reached 100 mm³, the mice were randomly divided into groups. 2 µg of the prepared T2.01m, 2 µg of T2.02m and 2 µg of T2.03m were intravenously injected three times at an interval of one week, and the anticancer activity in a mouse model was evaluated for one month. 7 mice per group were used. The tumor size and body weight were measured twice a week.

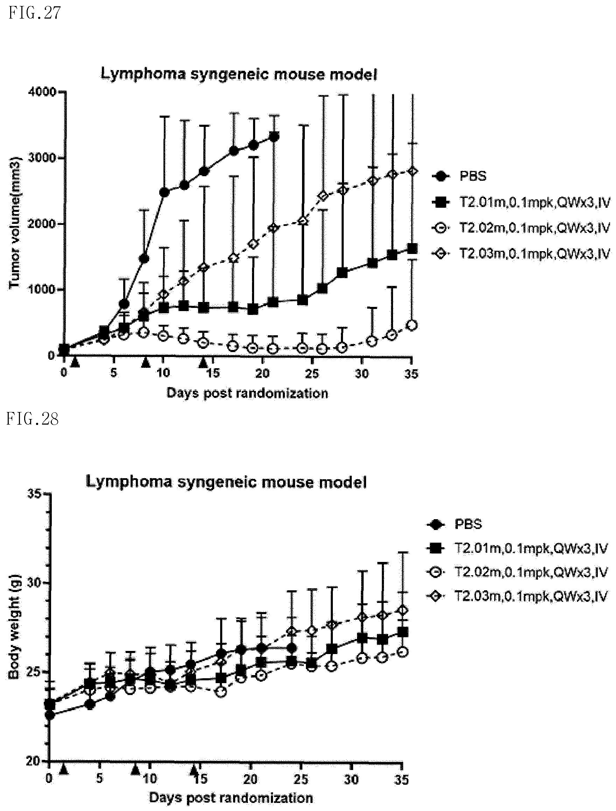

As a result, as shown in FIG. 27, it was found that on day 20 after the first intraperitoneal injection, the group treated with T2.01m showed a tumor growth inhibition percent (TGI) of 87%, the group treated with T2.02m showed a TGI of 98%, and the group treated with T2.03m showed a TGI of 40%.

In addition, T2.01m, T2.02m and T2.03m were intravenously injected, and the resulting side effects were expressed as a change in body weight.

As a result, as shown in FIG. 28, it was found that there were no side effects after intravenous injection of T2.01m, T2.02m and T2.03m.

## Claims

1. A fusion protein comprising:
a first monomer comprising IL-12 or a variant thereof; and
a second monomer comprising an antigen binding site that specifically binds to CD20.

2. The fusion protein according to claim 1, wherein the IL-12 or the variant thereof comprises IL-12A (p35) or a variant thereof; and IL-12B (p40) or a variant thereof.

3. The fusion protein according to claim 2, wherein the IL-12 or the variant thereof comprises the following structural formula (I) or (II):
N'-Y-[linker (1)]o-Z-C' (I)
N'-Z-[linker (1)]o-Y-C' (II)
in the structural formulas (I) and (II),
N' is the N-terminus of the fusion protein,
C' is the C-terminus of the fusion protein,
Y is the IL-12A or the variant thereof,
Z is the IL-12B or the variant thereof,
the linker (1) is a peptide linker, and
o is 0 or 1.

4. The fusion protein according to claim 2, wherein the variant of the IL-12B (p40) comprises an amino acid sequence obtained by at least one substitution selected from the group consisting of K258A, K260A, K263A, and K264A in the amino acid sequence of SEQ ID NO: 124.

5. The fusion protein according to claim 2, wherein the IL-12B (p40) comprises the amino acid sequence of SEQ ID NO: 124 or SEQ ID NO: 131.

6. The fusion protein according to claim 4, wherein the variant of the IL-12B (p40) comprises the amino acid sequence of SEQ ID NO: 127, SEQ ID NO: 129, SEQ ID NO: 134 or SEQ ID NO: 136.

7. The fusion protein according to claim 2, wherein the IL-12A (p35) comprises the amino acid sequence of SEQ ID NO: 125 or SEQ ID NO: 132.

8. The fusion protein according to claim 1, wherein the IL-12 comprises the amino acid sequence of SEQ ID NO: 123, SEQ ID NO: 126, SEQ ID NO: 128, SEQ ID NO: 130, SEQ ID NO: 133 or SEQ ID NO: 135.

9. The fusion protein according to claim 1, wherein the antigen binding site that specifically binds to CD20 comprises:
a heavy chain variable region comprising HCDR1 of SEQ ID NO: 107, HCDR2 of SEQ ID NO: 108 and HCDR3 of SEQ ID NO: 109; and a light chain variable region comprising LCDR1 of SEQ ID NO: 110, LCDR2 of SEQ ID NO: 111 and LCDR3 of SEQ ID NO: 112;
a heavy chain variable region comprising HCDR1 of SEQ ID NO: 115, HCDR2 of SEQ ID NO: 116 and HCDR3 of SEQ ID NO: 117; and a light chain variable region comprising LCDR1 of SEQ ID NO: 118, LCDR2 of SEQ ID NO: 119 and LCDR3 of SEQ ID NO: 120;
a heavy chain variable region comprising HCDR1 of SEQ ID NO: 143, HCDR2 of SEQ ID NO: 144 and HCDR3 of SEQ ID NO: 145; and a light chain variable region comprising LCDR1 of SEQ ID NO: 146, LCDR2 of SEQ ID NO: 147 and LCDR3 of SEQ ID NO: 148;
a heavy chain variable region comprising HCDR1 of SEQ ID NO: 149, HCDR2 of SEQ ID NO: 150 and HCDR3 of SEQ ID NO: 151; and a light chain variable region comprising LCDR1 of SEQ ID NO: 152, LCDR2 of SEQ ID NO: 153 and LCDR3 of SEQ ID NO: 154;
a heavy chain variable region comprising HCDR1 of SEQ ID NO: 155, HCDR2 of SEQ ID NO: 156 and HCDR3 of SEQ ID NO: 157; and a light chain variable region comprising LCDR1 of SEQ ID NO: 158, LCDR2 of SEQ ID NO: 159 and LCDR3 of SEQ ID NO: 160;
a heavy chain variable region comprising HCDR1 of SEQ ID NO: 161, HCDR2 of SEQ ID NO: 162 and HCDR3 of SEQ ID NO: 163; and a light chain variable region comprising LCDR1 of SEQ ID NO: 164, LCDR2 of SEQ ID NO: 165 and LCDR3 of SEQ ID NO: 166;
a heavy chain variable region comprising HCDR1 of SEQ ID NO: 167, HCDR2 of SEQ ID NO: 168 and HCDR3 of SEQ ID NO: 169; and a light chain variable region comprising LCDR1 of SEQ ID NO: 170, LCDR2 of SEQ ID NO: 171 and LCDR3 of SEQ ID NO: 172;
a heavy chain variable region comprising HCDR1 of SEQ ID NO: 173, HCDR2 of SEQ ID NO: 174 and HCDR3 of SEQ ID NO: 175; and a light chain variable region comprising LCDR1 of SEQ ID NO: 176, LCDR2 of SEQ ID NO: 177 and LCDR3 of SEQ ID NO: 178;
a heavy chain variable region comprising HCDR1 of SEQ ID NO: 179, HCDR2 of SEQ ID NO: 180 and HCDR3 of SEQ ID NO: 181; and a light chain variable region comprising LCDR1 of SEQ ID NO: 182, LCDR2 of SEQ ID NO: 183 and LCDR3 of SEQ ID NO: 184;
a heavy chain variable region comprising HCDR1 of SEQ ID NO: 185, HCDR2 of SEQ ID NO: 186 and HCDR3 of SEQ ID NO: 187; and a light chain variable region comprising LCDR1 of SEQ ID NO: 188, LCDR2 of SEQ ID NO: 189 and LCDR3 of SEQ ID NO: 190;
a heavy chain variable region comprising HCDR1 of SEQ ID NO: 191, HCDR2 of SEQ ID NO: 192 and HCDR3 of SEQ ID NO: 193; and a light chain variable region comprising LCDR1 of SEQ ID NO: 194, LCDR2 of SEQ ID NO: 195 and LCDR3 of SEQ ID NO: 196;
a heavy chain variable region comprising HCDR1 of SEQ ID NO: 197, HCDR2 of SEQ ID NO: 198 and HCDR3 of SEQ ID NO: 199 or SEQ ID NO: 200; and a light chain variable region comprising LCDR1 of SEQ ID NO: 201, LCDR2 of SEQ ID NO: 202 and LCDR3 of SEQ ID NO: 203;
a heavy chain variable region comprising HCDR1 of SEQ ID NO: 204, HCDR2 of SEQ ID NO: 205 and HCDR3 of SEQ ID NO: 206; and a light chain variable region comprising LCDR1 of SEQ ID NO: 207, LCDR2 of SEQ ID NO: 208 and LCDR3 of SEQ ID NO: 209;
a heavy chain variable region comprising HCDR1 of SEQ ID NO: 210, HCDR2 of SEQ ID NO: 211 and HCDR3 of SEQ ID NO: 212; and a light chain variable region comprising LCDR1 of SEQ ID NO: 213, LCDR2 of SEQ ID NO: 214 and LCDR3 of SEQ ID NO: 215;
a heavy chain variable region comprising HCDR1 of SEQ ID NO: 216, HCDR2 of SEQ ID NO: 217 and HCDR3 of SEQ ID NO: 218; and a light chain variable region comprising LCDR1 of SEQ ID NO: 219, LCDR2 of SEQ ID NO: 220 and LCDR3 of SEQ ID NO: 221;
a heavy chain variable region comprising HCDR1 of SEQ ID NO: 222, HCDR2 of SEQ ID NO: 223 and HCDR3 of SEQ ID NO: 224; and a light chain variable region comprising LCDR1 of SEQ ID NO: 225, LCDR2 of SEQ ID NO: 226 and LCDR3 of SEQ ID NO: 227;
a heavy chain variable region comprising HCDR1 of SEQ ID NO: 228, HCDR2 of SEQ ID NO: 229 and HCDR3 of SEQ ID NO: 230; and a light chain variable region comprising LCDR1 of SEQ ID NO: 231, LCDR2 of SEQ ID NO: 232 and LCDR3 of SEQ ID NO: 233; or
a heavy chain variable region comprising HCDR1 of SEQ ID NO: 234, HCDR2 of SEQ ID NO: 235 and HCDR3 of SEQ ID NO: 236; and a light chain variable region comprising LCDR1 of SEQ ID NO: 237, LCDR2 of SEQ ID NO: 238 and LCDR3 of SEQ ID NO: 239.

10. The fusion protein according to claim 1, wherein the antigen binding site that specifically binds to CD20 comprises:
a heavy chain variable region of SEQ ID NO: 113 and a light chain variable region of SEQ ID NO: 114;
a heavy chain variable region of SEQ ID NO: 121 and a light chain variable region of SEQ ID NO: 122;
a heavy chain variable region of SEQ ID NO: 240 and a light chain variable region of SEQ ID NO: 241;
a heavy chain variable region of SEQ ID NO: 242 and a light chain variable region of SEQ ID NO: 243;
a heavy chain variable region of SEQ ID NO: 244 and a light chain variable region of SEQ ID NO: 245;
a heavy chain variable region of SEQ ID NO: 246 and a light chain variable region of SEQ ID NO: 247;
a heavy chain variable region of SEQ ID NO: 248 and a light chain variable region of SEQ ID NO: 249;
a heavy chain variable region of SEQ ID NO: 250 and a light chain variable region of SEQ ID NO: 251;
a heavy chain variable region of SEQ ID NO: 252 and a light chain variable region of SEQ ID NO: 253;
a heavy chain variable region of SEQ ID NO: 254 and a light chain variable region of SEQ ID NO: 255;
a heavy chain variable region of SEQ ID NO: 256 and a light chain variable region of SEQ ID NO: 257;
a heavy chain variable region of SEQ ID NO: 258 and a light chain variable region of SEQ ID NO: 259;
a heavy chain variable region of SEQ ID NO: 260 and a light chain variable region of SEQ ID NO: 261;
a heavy chain variable region of SEQ ID NO: 262 and a light chain variable region of SEQ ID NO: 263;
a heavy chain variable region of SEQ ID NO: 264 and a light chain variable region of SEQ ID NO: 265;
a heavy chain variable region of SEQ ID NO: 266 and a light chain variable region of SEQ ID NO: 267;
a heavy chain variable region of SEQ ID NO: 268 and a light chain variable region of SEQ ID NO: 269;
a heavy chain variable region of SEQ ID NO: 270 and a light chain variable region of SEQ ID NO: 271;
scFv of SEQ ID NO: 88; or
scFv of SEQ ID NO: 89.

11. The fusion protein according to claim 1, wherein the first monomer further comprises an antigen binding site that specifically binds to CD20.

12. The fusion protein according to claim 1, wherein the first monomer comprises the following structural formula (III) or (IV):
N'-X-[linker (2)]p-Fc region fragment or variant thereof-[linker (3)]q-(T)r-C' (III)
N'-(T)r-[linker (2)]q-Fc region fragment or variant thereof-[linker (3)]p-X-C' (IV)
in the structural formulas (III) and (IV),
N' is the N-terminus of the fusion protein,
C' is the C-terminus of the fusion protein,
X is the structural formula (I) or (II),
T is the antigen binding site that specifically binds to CD20,
the linkers (2) and (3) are peptide linkers, and
p, q and r are each independently 0 or 1.

13. The fusion protein according to claim 3 or 12, wherein the linker comprises a (G₄S)ₙ linker, wherein n is any one of integers from 1 to 10.

14. The fusion protein according to claim 12, wherein the Fc region of the first monomer is derived from human IgG1 or mouse IgG2a.

15. The fusion protein according to claim 12, wherein the Fc of the first monomer comprises a knob structure or a hole structure.

16. The fusion protein according to claim 1, wherein the second monomer further comprises an antigen binding site that specifically binds to CD20.

17. The fusion protein according to claim 16, wherein the antigen binding site that specifically binds to CD20 is Fab, scFv, Fv, or a fragment thereof.

18. The fusion protein according to claim 16, wherein the additionally bound antigen binding site that specifically binds to CD20 is bound to the N-terminus or C-terminus of the second monomer.

19. The fusion protein according to claim 3, wherein the second monomer comprises the following structural formula (V):
N'-(R)s-[linker (4)]t-Q-[linker (5)]u-Fc region fragment or variant thereof-[linker (6)]v-(W)a-C' (V)
in the structural formula (V),
N' is the N-terminus of the fusion protein,
C' is the C-terminus of the fusion protein,
R is the antigen binding site that specifically binds to CD20,
Q is the antigen binding site that specifically binds to CD20,
W is scFv that specifically binds to CD20; or the IL-12 of structural formula (I) or (II) or the variant thereof,
the linkers (4) to (6) are peptide linkers, and
s, t, u and v are each independently 0 or 1.

20. The fusion protein according to claim 19, wherein the structural formula (V) comprises the following structural formulas (V') and (V"):
N'-(R')s-[linker (4)]t-Q'-[linker (5)]u-Fc region fragment or variant thereof-[linker (6)]p-(W)a-C' (V)
N'-(R")s-[linker (4)]t-Q"-[linker (7)]x-(W)b-C' (V")
in the structural formulas (V') and (V"),
R' is a heavy chain region of an antibody that specifically binds to CD20, comprising a variable region and a CH1 region, or a light chain region of the antibody;
R" is a light chain region of an antibody that specifically binds to CD20, or a heavy chain region of the antibody, comprising a variable region and a CH1 region;
wherein R' and R" bind to each other to form a variable region of the antibody, wherein the variable region specifically binds to CD20;
Q' is a heavy chain region of an antibody that specifically binds to CD20, comprising a variable region and a CH1 region, or a light chain region of the antibody;
Q" is a light chain region of an antibody that specifically binds to CD20, or a heavy chain region of the antibody, comprising a variable region and a CH1 region;
wherein Q' and Q" bind to each other to form a variable region of the antibody, wherein the variable region specifically binds to CD20,
R' and Q' are each a heavy chain region of the antibody, comprising a variable region and a CH1 region, or a light chain region of the antibody;
R" and Q" are each a light chain region of the antibody, or a heavy chain region of the antibody, comprising a variable region and a CH1 region;
wherein R' and R"; and Q' and Q" bind to each other to form a variable region of the antibody, wherein the variable region specifically binds to CD20,
W is scFv that specifically binds to CD20,
the linkers (4), (5) and (7) are peptide linkers, and
s, t, u, x, a and b are each independently 0 or 1.

21. The fusion protein according to claim 1,
wherein the fusion protein comprises structural formula (III) and structural formula (V); structural formula (IV) and structural formula (V); structural formula (III) and structural formula (III); structural formula (IV) and structural formula (IV); or structural formula (V) and structural formula (V).

22. The fusion protein according to claim 1, wherein the second monomer comprises a heavy chain consisting of the amino acid sequence of SEQ ID NO: 113 and a light chain consisting of the amino acid sequence of SEQ ID NO: 114; or
a heavy chain consisting of the amino acid sequence of SEQ ID NO: 121 and a light chain consisting of the amino acid sequence of SEQ ID NO: 122.

23. The fusion protein according to claim 2, wherein the Fc of the second monomer comprises a knob structure or a hole structure.

24. A pharmaceutical composition for preventing or treating cancer, comprising the fusion protein of any one of claims 1 to 23 as an active ingredient.

25. The pharmaceutical composition according to claim 24, wherein the cancer is any one selected from the group consisting of gastric cancer, liver cancer, lung cancer, colorectal cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer and lymphoma.

26. A polynucleotide encoding the first monomer of claim 1.

27. A vector comprising the polynucleotide of claim 26.

28. A polynucleotide encoding the second monomer of claim 1.

29. A vector comprising the polynucleotide of claim 28.

30. A transformed cell into which the vector of claim 27 or 29 has been introduced.

31. A method of producing a fusion protein, comprising:
i) culturing the transformed cell of claim 30; and
ii) recovering a fusion protein comprising the first monomer and the second monomer.

32. A method for treating or preventing cancer, comprising:
administering, to a subject, a fusion protein comprising a first monomer comprising IL-12 or a variant thereof; and a second monomer comprising an antigen binding site that specifically binds to CD20.

33. A use of a fusion protein comprising a first monomer comprising IL-12 or a variant thereof; and a second monomer comprising an antigen binding site that specifically binds to CD20 for treatment of cancer.

34. A use of a fusion protein comprising a first monomer comprising IL-12 or a variant thereof; and a second monomer comprising an antigen binding site that specifically binds to CD20 for manufacture of a medicament for treating cancer.
